# EUROPÄISCHE PATENTANMELDUNG

(11) **EP 3 284 739 A1**
(43) Veröffentlichungstag der Anmeldung: **21.02.2018**
(21) Anmeldenummer: 17182134.1
(22) Anmeldetag: 19.07.2017
(51) Int. Cl.: C07D 213/75, C07C 237/24, A01N 43/14, A01N 37/22

(54) **SUBSTITUIERTE (HET)ARYLVERBINDUNGEN ALS SCHÄDLINGSBEKÄMPFUNGSMITTEL**

(71) Anmelder: Bayer CropScience Aktiengesellschaft, 40789 Monheim am Rhein (DE)
(72) Erfinder: Die Erfindernennung liegt noch nicht vor
(74) Vertreter: BIP Patents

(57) **Zusammenfassung**

Die vorliegende Erfindung betrifft neue substituierte (Het)arylverbindungen der Formel (I), 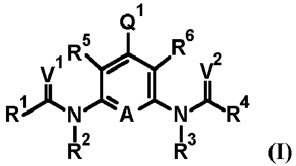 in welcher A, R¹, R², R³, R⁴, R⁵, R⁶, V¹, V² und Q¹ die in der Beschreibung angegebenen Bedeutungen haben sowie deren Anwendung als Akarizide und Insektizide zur Bekämpfung tierischer Schädlinge, vor allem von Arthropoden und insbesondere von Insekten und Spinnentieren und Verfahren und Zwischenprodukte zu ihrer Herstellung.

## Beschreibung

Die vorliegende Erfindung betrifft neue substituierte (Het)arylverbindungen der Formel (I), deren Anwendung als Akarizide und/oder Insektizide zur Bekämpfung tierischer Schädlinge, vor allem von Arthropoden und insbesondere von Insekten und Spinnentieren und Verfahren und Zwischenprodukte zu ihrer Herstellung.

In JP 05105649 A, JP 60092268 A, WO 2015/025018 A1, WO 2001/068151 A2, CN 101919862 A, European Journal of Pharmacology (2007), 565(1-3), 54-59, Chemical Communications (2008), (29), 3441-3443, Journal für Praktische Chemie (1924), 108, 75-87 sind bereits substituierte (Het)arylverbindungen beschrieben, die sich jedoch im Substitutionsmuster von den erfindungsgemäßen Verbindungen beispielsweise im Substituenten Q¹ unterscheiden.

Moderne Pflanzenschutzmittel müssen vielen Anforderungen genügen, beispielsweise in Bezug auf Höhe, Dauer und Breite ihrer Wirkung und möglichen Verwendung. Es spielen Fragen der Toxizität, der Kombinierbarkeit mit anderen Wirkstoffen oder Formulierhilfsmitteln eine Rolle sowie die Frage des Aufwands, der für die Synthese eines Wirkstoffs betrieben werden muss. Ferner können Resistenzen auftreten. Schon aus all diesen Gründen kann die Suche nach neuen Pflanzenschutzmitteln nicht als abgeschlossen betrachtet werden und es besteht ständig Bedarf an neuen Verbindungen mit gegenüber den bekannten Verbindungen zumindest in Bezug auf einzelne Aspekte verbesserten Eigenschaften.

Aufgabe der vorliegenden Erfindung war es, Verbindungen bereitzustellen, durch die das Spektrum der Schädlingsbekämpfungsmittel unter verschiedenen Aspekten verbreitert und/oder ihre Aktivität verbessert wird.

Es wurde nun überraschend gefunden, dass bestimmte neue substituierte (Het)arylverbindungen der Formel (I) sowie deren Salze starke insektizide und akarizide Eigenschaften bei gleichzeitig guter Pflanzenverträglichkeit, günstiger Warmblütertoxizität und guter Umweltverträglichkeit besitzen.

Als Schädlingsbekämpfungsmittel grundsätzlich geeignete substituierte (Het)arylverbindungen (**Ausgestaltung 1**) sind daher Verbindungen der allgemeinen Formel (I) 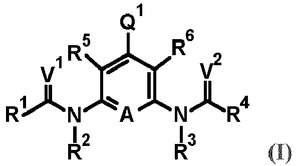
- Q¹: für jeweils gegebenenfalls einfach oder mehrfach, gleich oder verschieden substituiertes 6-gliedriges Aryl oder für 5-oder 6-gliedriges Hetaryl oder Oxo-Hetaryl steht,
wobei die Substituenten ausgewählt sind aus: Cyano, Carboxyl, Halogen, Nitro, Hydroxy, Amino, SCN, SF₅, Tri-(C₁-C₆)alkylsilyl, (C₁-C₆)Alkyl, (C₁-C₆)Halogenalkyl, (C₁-C₆)Cyanoalkyl, (C₁-C₆)Hydroxyalkyl, Hydroxycarbonyl-(C₁-C₆)-alkoxy, (C₁-C₆)Alkoxycarbonyl-(C₁-C₆)alkyl, (C₁-C₆)Alkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₁-C₆)Alkoxy, (C₁-C₆)Halogenalkoxy, (C₁-C₆)Cyanoalkoxy, (C₁-C₆)Alkoxycarbonyl-(C₁-C₆)alkoxy, (C₁-C₆)Alkoxy-(C₁-C₆)alkoxy, (C₃-C₈)Cycloalkyl, (C₃-C₈)Halogencycloalkyl, (C₁-C₆)Alkyl-(C₃-C₈)cycloalkyl, (C₁-C₆)Halogenalkyl-(C₃-C₈)cycloalkyl, (C₁-C₄)Halogenalkenoxy, (C₁-C₆)Alkylhydroxyimino, (C₁-C₆)Alkoxyimino, (C₁-C₆)Alkyl-(C₁-C₆)alkoxyimino, (C₁-C₆)Halogenalkyl-(C₁-C₆)alkoxyimino, (C₁-C₆)Alkylsulfanyl, (C₁-C₆)Alkoxy-(C₁-C₆)alkylsulfanyl, (C₁-C₆)Alkylsulfanyl-(C₁-C₆)alkyl, (C₁-C₆)Alkylsulfinyl, (C₁-C₆)Halogenalkylsulfinyl, (C₁-C₆)Alkoxy-(C₁-C₆)alkylsulfinyl, (C₁-C₆)Alkylsulfinyl-(C₁-C₆)alkyl, (C₁-C₆)Alkylsulfonyl, (C₁-C₆)Halogenalkylsulfonyl, (C₁-C₆)Alkoxy-(C₁-C₆)alkylsulfonyl, (C₁-C₆)Alkylsulfonyl-(C₁-C₆)alkyl, (C₁-C₆)Alkylsulfonyloxy, (C₁-C₆)Halogenalkylsulfanyl, (C₃-C₆)Cycloalkylsulfanyl, (C₃-C₆)Halogencycloalkylsulfanyl, (C₃-C₆)Cycloalkylsulfonyl, (C₃-C₆)Halogencycloalkylsulfonyl, (C₃-C₆)Cycloalkylsulfinyl, (C₃-C₆)Halogencycloalkylsulfinyl, (C₁-C₆)Alkylcarbonyl, (C₁-C₆)Halogenalkylcarbonyl, (C₁-C₆)Alkylcarbonyloxy, (C₁-C₆)Alkoxycarbonyl, (C₁-C₆)Halogenalkoxycarbonyl, Aminocarbonyl, (C₁-C₆)Alkylaminocarbonyl, Di-(C₁-C₆)alkyl-aminocarbonyl, (C₂-C₆)Alkenylaminocarbonyl, Di-(C₂-C₆)-alkenylaminocarbonyl, (C₃-C₈)Cycloalkylaminocarbonyl, (C₁-C₆)Alkylsulfonylamino, (C₁-C₆)Alkylamino, Bis-(C₁-C₆)Alkylamino, (C₁-C₆)Halogenalkylamino, Bis-(C₁-C₆)Halogenalkylamino, Aminosulfonyl, (C₁-C₆)Alkylaminosulfonyl, Di-(C₁-C₆)alkylaminosulfonyl, (C₁-C₆)Alkylsulfoximino, Aminothiocarbonyl, (C₁-C₆)Alkylaminothiocarbonyl, Di-(C₁-C₆)alkylaminothiocarbonyl, (C₃-C₈)Cycloalkylamino,
- R¹: für (C₁-C₆)Alkyl, (C₁-C₆)Halogenalkyl, (C₁-C₆)Cyanoalkyl, (C₁-C₆)Hydroxyalkyl, (C₁-C₆)Alkoxy-(C₁-C₆)alkyl, (C₁-C₆)Halogenalkoxy-(C₁-C₆)alkyl, (C₂-C₆)Alkenyl, (C₂-C₆)Alkenyloxy-(C₁-C₆)alkyl, (C₂-C₆)Halogenalkenyloxy-(C₁-C₆)alkyl, (C₂-C₆)Halogenalkenyl, (C₂-C₆)Cyanoalkenyl, (C₂-C₆)Alkinyl, (C₂-C₆)Alkinyloxy-(C₁-C₆)alkyl, (C₂-C₆)Halogenalkinyloxy-(C₁-C₆)alkyl, (C₂-C₆)Halogenalkinyl, (C₂-C₆)Cyanoalkinyl, (C₃-C₈)Cycloalkyl, (C₃-C₈)Cycloalkyl-(C₁-C₆)alkyl, (C₃-C₈)Cycloalkyl-(C₃-C₈)Cycloalkyl, (C₁-C₆)Alkyl-(C₃-C₈)Cycloalkyl, (C₁-C₆)Halogenalkyl-(C₃-C₈)cycloalkyl, Halogen(C₃-C₈)cycloalkyl, Cyano(C₃-C₈)cycloalkyl, (C₂-C₆)Alkinyl-(C₃-C₈)cycloalkyl, (C₁-C₆)Alkoxy-(C₃-C₈)cycloalkyl, (C₁-C₆)Alkoxycarbonyl-(C₃-C₈)cycloalkyl, (C₁-C₆)Alkylamino, Di-(C₁-C₆)alkyl-amino, (C₃-C₈)Cycloalkylamino, (C₁-C₆)Alkylcarbonyl-amino, (C₁-C₆)Alkylthio-(C₁-C₆)alkyl, (C₁-C₆)Halogenalkylthio-(C₁-C₆)alkyl, (C₁-C₆)Alkylsulfinyl-(C₁-C₆)alkyl, (C₁-C₆)Halogenalkylsulfinyl-(C₁-C₆)alkyl, (C₁-C₆)Alkylsulfonyl-(C₁-C₆)alkyl, (C₁-C₆)Halogenalkylsulfonyl-(C₁-C₆)alkyl, (C₁-C₆)Alkoxy-(C₁-C₆)alkylthio-(C₁-C₆)alkyl, (C₁-C₆)Alkoxy-(C₁-C₆)alkylsulfinyl-(C₁-C₆)alkyl, (C₁-C₆)Alkoxy-(C₁-C₆)alkylsulfonyl-(C₁-C₆)alkyl, (C₁-C₆)Alkylcarbonyl-(C₁-C₆)alkyl, (C₁-C₆)Halogenalkylcarbonyl-(C₁-C₆)alkyl, (C₁-C₆)Alkoxycarbonyl-(C₁-C₆)alkyl, (C₁-C₆)Halogenalkoxycarbonyl-(C₁-C₆)alkyl, (C₁-C₆)Alkylsulfonylamino, Aminosulfonyl-(C₁-C₆)alkyl, (C₁-C₆)Alkylaminosulfonyl-(C₁-C₆)alkyl, Di-(C₁-C₆)alkyl-aminosulfonyl-(C₁-C₆)alkyl, (C₁-C₄)Alkoxy, (C₃-C₆)Cycloalkoxy, (C₃-C₆)Cycloalkylcarbonyl oder (C₁-C₄)Alkylcarbonyl steht,
oder für jeweils gegebenenfalls einfach oder mehrfach, gleich oder verschieden substituiertes Aryl, Hetaryl, Oxo-Hetaryl, Heterocyclyl oder Oxo-Heterocyclyl steht, wobei die Substituenten ausgewählt sind aus Halogen, Cyano, Nitro, Hydroxy, Amino, Carboxy, Carbamoyl, (C₁-C₆)Alkyl, (C₃-C₈)Cycloalkyl, (C₁-C₆)-Alkoxy, (C₁-C₆)Halogenalkyl, (C₁-C₆)Halogenalkoxy, (C₁-C₆)Alkylthio, (C₁-C₆)Halogenalkylthio, (C₁-C₆)Alkylsulfinyl, (C₁-C₆)Alkylsulfonyl, (C₁-C₆)Alkylsulfimino, (C₁-C₆)Alkylsulfimino-(C₁-C₆)alkyl, (C₁-C₆)Alkylsulfimino-(C₂-C₆)alkylcarbonyl, (C₁-C₆)Alkylsulfoximino, (C₁-C₆)Alkylsulfoximino-(C₁-C₆)alkyl, (C₁-C₆)Alkylsulfoximino-(C₂-C₆)alkylcarbonyl, (C₁-C₆)Alkoxycarbonyl, (C₁-C₆)Alkylcarbonyl, (C₃-C₆)Trialkylsilyl,
- R², R³: unabhängig voneinander für Wasserstoff, (C₁-C₆)Alkyl, (C₃-C₈)Cycloalkyl, (C₁-C₆)Alkoxy, (C₁-C₆)Alkylcarbonyl, (C₃-C₈)Cycloalkylcarbonyl, (C₁-C₆)Alkoxycarbonyl, (C₁-C₆)Halogenalkyl, (C₁-C₆)Cyanoalkyl, (C₁-C₆)Hydroxyalkyl, (C₁-C₆)Alkoxy-(C₁-C₆)alkyl, (C₁-C₆)Halogenalkoxy-(C₁-C₆)alkyl, (C₂-C₆)Alkenyl, (C₂-C₆)Alkenyloxy-(C₁-C₆)alkyl, (C₂-C₆)Halogenalkenyloxy-(C₁-C₆)alkyl, (C₂-C₆)Halogenalkenyl, (C₂-C₆)Cyanoalkenyl, (C₂-C₆)Alkinyl, (C₂-C₆)Alkinyloxy-(C₁-C₆)alkyl, (C₂-C₆)Halogenalkinyloxy-(C₁-C₆)alkyl, (C₂-C₆)Halogenalkinyl, (C₂-C₆)Cyanoalkinyl, (C₃-C₈)Cycloalkyl-(C₃-C₈)Cycloalkyl, (C₁-C₆)Alkyl-(C₃-C₈)Cycloalkyl, Halogen(C₃-C₈)cycloalkyl, (C₁-C₆)Alkylthio-(C₁-C₆)alkyl, (C₁-C₆)Halogenalkylthio-(C₁-C₆)alkyl, (C₁-C₆)Alkylsulfinyl-(C₁-C₆)alkyl, (C₁-C₆)Halogenalkylsulfinyl-(C₁-C₆)alkyl, (C₁-C₆)Alkylsulfonyl-(C₁-C₆)alkyl, (C₁-C₆)Halogenalkylsulfonyl-(C₁-C₆)alkyl, (C₁-C₆)Alkoxy-(C₁-C₆)alkylthio-(C₁-C₆)alkyl, (C₁-C₆)Alkoxy-(C₁-C₆)alkylsulfinyl-(C₁-C₆)alkyl, (C₁-C₆)Alkoxy-(C₁-C₆)alkylsulfonyl-(C₁-C₆)alkyl, (C₁-C₆)Alkylcarbonyl-(C₁-C₆)alkyl, (C₁-C₆)Halogenalkylcarbonyl-(C₁-C₆)alkyl, (C₁-C₆)Alkoxycarbonyl-(C₁-C₆)alkyl, (C₁-C₆)Halogenalkoxycarbonyl-(C₁-C₆)alkyl, Aminocarbonyl-(C₁-C₆)alkyl, (C₁-C₆)Alkylamino-(C₁-C₆)alkyl, Di-(C₁-C₆)alkylamino-(C₁-C₆)alkyl oder (C₃-C₈)Cycloalkylamino-(C₁-C₆)alkyl steht,
- R⁴: für (C₁-C₆)Alkyl, (C₁-C₆)Halogenalkyl, (C₁-C₆)Cyanoalkyl, (C₁-C₆)Hydroxyalkyl, (C₁-C₆)Alkoxy-(C₁-C₆)alkyl, (C₁-C₆)Halogenalkoxy-(C₁-C₆)alkyl, (C₂-C₆)Alkenyl, (C₂-C₆)Alkenyloxy-(C₁-C₆)alkyl, (C₂-C₆)Halogenalkenyloxy-(C₁-C₆)alkyl, (C₂-C₆)Halogenalkenyl, (C₂-C₆)Cyanoalkenyl, (C₂-C₆)Alkinyl, (C₂-C₆)Alkinyloxy-(C₁-C₆)alkyl, (C₂-C₆)Halogenalkinyloxy-(C₁-C₆)alkyl, (C₂-C₆)Halogenalkinyl, (C₂-C₆)Cyanoalkinyl, (C₃-C₈)Cycloalkyl, (C₃-C₈)Cycloalkyl-(C₁-C₆)alkyl, Cyano(C₃-C₈)cycloalkyl-(C₁-C₆)alkyl, Halogen(C₃-C₈)cycloalkyl-(C₁-C₆)alkyl, (C₁-C₄)Halogenalkyl-(C₃-C₈)cycloalkyl-(C₁-C₆)alkyl, (C₃-C₈)Cycloalkyl-(C₃-C₈)Cycloalkyl, (C₁-C₆)Alkyl-(C₃-C₈)Cycloalkyl, (C₁-C₆)Halogenalkyl-(C₃-C₈)cycloalkyl, Halogen(C₃-C₈)cycloalkyl, Cyano(C₃-C₈)cycloalkyl, (C₂-C₆)Alkinyl-(C₃-C₈)cycloalkyl, (C₁-C₆)Alkoxy-(C₃-C₈)cycloalkyl, (C₁-C₆)Alkoxycarbonyl-(C₃-C₈)cycloalkyl, Carbamoyl-(C₃-C₈)cycloalkyl, Thiocarbamoyl-(C₃-C₈)cycloalkyl, (C₁-C₆)Alkylthio-(C₁-C₆)alkyl, (C₁-C₆)Halogenalkylthio-(C₁-C₆)alkyl, (C₁-C₆)Alkylsulfinyl-(C₁-C₆)alkyl, (C₁-C₆)Halogenalkylsulfinyl-(C₁-C₆)alkyl, (C₁-C₆)Alkylsulfonyl-(C₁-C₆)alkyl, (C₁-C₆)Halogenalkylsulfonyl-(C₁-C₆)alkyl, (C₁-C₆)Alkoxy-(C₁-C₆)alkylthio-(C₁-C₆)alkyl, (C₁-C₆)Alkoxy-(C₁-C₆)alkylsulfinyl-(C₁-C₆)alkyl, (C₁-C₆)Alkoxy-(C₁-C₆)alkylsulfonyl-(C₁-C₆)alkyl, (C₁-C₆)Alkylcarbonyl-(C₁-C₆)alkyl, (C₁-C₆)Halogenalkylcarbonyl-(C₁-C₆)alkyl, (C₁-C₆)Alkoxycarbonyl-(C₁-C₆)alkyl, (C₁-C₆)Halogenalkoxycarbonyl-(C₁-C₆)alkyl, (C₃-C₆)Cycloalkenyl, Halogen(C₃-C₆)Cycloalkenyl, (C₁-C₄)Alkyl-(C₃-C₆)Cycloalkenyl oder (C₁-C₄)Halogenalkyl-(C₃-C₆)Cycloalkenyl steht,
oder für durch Aryl, Hetaryl, Oxo-Hetaryl, Heterocyclyl oder Oxo-Heterocyclyl substituiertes (C₁-C₆)Alkyl, (C₁-C₆)Halogenalkyl, (C₂-C₆)Alkenyl, (C₂-C₆)Alkinyl oder (C₃-C₈)Cycloalkyl steht, wobei Aryl, Hetaryl, Oxo-Hetaryl, Heterocyclyl oder Oxo-Heterocyclyl jeweils gegebenenfalls einfach oder mehrfach, gleich oder verschieden durch Halogen, Cyano, Nitro, Hydroxy, Amino, Carboxy, Carbamoyl, Thiocarbamoyl, Aminosulfonyl, (C₁-C₆)Alkyl, (C₃-C₆)Cycloalkyl, (C₁-C₆)Alkoxy, (C₁-C₆)Halogenalkyl, (C₁-C₆)Halogenalkoxy, (C₁-C₆)Alkylthio, (C₁-C₆)Halogenalkylthio, (C₁-C₆)Alkylsulfinyl, (C₁-C₆)Alkylsulfonyl, (C₁-C₆)Halogenalkyl-sulfinyl, (C₁-C₆)Halogenalkylsulfonyl, (C₁-C₆)Alkylsulfimino, (C₁-C₆)Alkylsulfimino-(C₁-C₆)alkyl, (C₁-C₆)Alkylsulfimino-(C₂-C₆)alkylcarbonyl, (C₁-C₆)Alkylsulfoximino, (C₁-C₆)Alkylsulfoximino-(C₁-C₆)alkyl, (C₁-C₆)Alkylsulfoximino-(C₂-C₆)alkylcarbonyl, (C₁-C₆)Alkoxycarbonyl, (C₁-C₆)Alkylcarbonyl oder (C₃-C₆)Trialkylsilyl substituiert sein können,
oder für jeweils gegebenenfalls einfach oder mehrfach, gleich oder verschieden substituiertes Aryl, Hetaryl, Oxo-Hetaryl, Heterocyclyl oder Oxo-Heterocyclyl steht, wobei die Substituenten ausgewählt sind aus Halogen, Cyano, Nitro, Hydroxy, Amino, Carboxy, Carbamoyl, Thiocarbamoyl, (C₁-C₆)Alkyl, (C₃-C₈)Cycloalkyl, (C₁-C₆)-Alkoxy, (C₁-C₆)Halogenalkyl, (C₁-C₆)Halogenalkoxy, (C₁-C₆)Alkylthio, (C₁-C₆)Halogenalkylthio, (C₁-C₆)Alkylsulfinyl, (C₁-C₆)Alkylsulfonyl, (C₁-C₆)Halogenalkylsulfinyl, (C₁-C₆)Halogenalkylsulfonyl, (C₁-C₆)Alkylsulfimino, (C₁-C₆)Alkylsulfimino-(C₁-C₆)alkyl, (C₁-C₆)Alkylsulfimino-(C₂-C₆)alkylcarbonyl, (C₁-C₆)Alkylsulfoximino, (C₁-C₆)Alkylsulfoximino-(C₁-C₆)alkyl, (C₁-C₆)Alkylsulfoximino-(C₂-C₆)alkylcarbonyl, (C₁-C₆)Alkoxycarbonyl, (C₁-C₆)Alkylcarbonyl oder (C₃-C₆)Trialkylsilyl,
- R⁵, R⁶: unabhängig voneinander für Wasserstoff, Cyano, Halogen, Nitro, Acetyl, Hydroxy, Amino, (C₁-C₆)Alkylamino, Di-(C₁-C₆)Alkylamino, (C₃-C₆)Cycloalkyl, Halogen(C₃-C₆)cycloalkyl, (C₁-C₆)Alkyl, (C₁-C₆)Halogenalkyl, (C₂-C₆)Alkenyl, (C₂-C₆)Halogenalkenyl, (C₂-C₆)Alkinyl, (C₂-C₆)Halogenalkinyl, (C₁-C₆)Alkoxy, (C₁-C₆)Halogenalkoxy, (C₁-C₆)Alkylthio, (C₁-C₆)Halogenalkylthio, (C₁-C₆)Alkylsulfinyl, (C₁-C₆)Halogenalkylsulfinyl, (C₁-C₆)Alkylsulfonyl, (C₁-C₆)Halogenalkylsulfonyl, stehen,
- V¹, V²: unabhängig voneinander für Sauerstoff oder Schwefel stehen,
- A: für Stickstoff, =N⁺(O⁻)- oder =C(H)- steht.

Weiterhin wurde gefunden, dass die Verbindungen der Formel (I) eine sehr gute Wirksamkeit als Schädlingsbekämpfungsmittel, vorzugsweise als Insektizide und/oder Akarizide aufweisen, darüber hinaus in der Regel insbesondere gegenüber Kulturpflanzen sehr gut pflanzenverträglich sind.

Die grundsätzlich geeigneten und die erfindungsgemäßen Verbindungen sind durch die Formel (I) allgemein definiert. Bevorzugte Substituenten bzw. Bereiche der in der oben und nachstehend erwähnten Formeln aufgeführten Reste werden im Folgenden erläutert:

### Bevorzugt geeigneter Bereich:

Bevorzugt und als Schädlingsbekämpfungsmittel grundsätzlich geeignet (Ausgestaltung 2) sind die Verbindungen der Formel (I), in denen
- Q¹: für jeweils gegebenenfalls einfach oder mehrfach, gleich oder verschieden substituiertes Phenyl, Thiophen, Pyridin, Pyrimidin, Pyrazol, Triazol oder Imidazol steht,
wobei die Substituenten ausgewählt sind aus Cyano, Halogen, Nitro, Amino, SF₅, (C₁-C₄)Alkyl, (C₁-C₄)Halogenalkyl, (C₁-C₄)Cyanoalkyl, (C₁-C₄)Hydroxyalkyl, (C₁-C₄)Alkoxy-(C₁-C₄)alkyl, (C₂-C₄)Alkenyl, (C₂-C₄)Halogenalkenyl, (C₂-C₄)Cyanoalkenyl, (C₂-C₄)Alkinyl, (C₂-C₄)Halogenalkinyl, (C₂-C₄)Cyanoalkinyl, (C₁-C₄)Alkoxy, (C₁-C₄)Halogenalkoxy, (C₁-C₄)Cyanoalkoxy, (C₁-C₄)Alkoxy-(C₁-C₄)alkoxy, (C₃-C₆)Cycloalkyl, (C₃-C₆)Halogencycloalkyl, (C₁-C₄)Alkyl-(C₃-C₆)cycloalkyl, (C₁-C₄)Halogenalkyl-(C₃-C₆)cycloalkyl, (C₁-C₄)Halogenalkenoxy, (C₁-C₄)Alkylhydroxyimino, (C₁-C₄)Alkoxyimino, (C₁-C₄)Alkyl-(C₁-C₄)alkoxyimino, (C₁-C₄)Halogenalkyl-(C₁-C₄)alkoxyimino, (C₁-C₄)Alkylsulfanyl, (C₁-C₄)Alkylsulfanyl-(C₁-C₄)alkyl, (C₁-C₄)Alkylsulfinyl, (C₁-C₄)Halogenalkylsulfinyl, (C₁-C₄)Alkylsulfinyl-(C₁-C₄)alkyl, (C₁-C₄)Alkylsulfonyl, (C₁-C₄)Halogenalkylsulfonyl, (C₁-C₄)Alkylsulfonyl-(C₁-C₄)alkyl, (C₁-C₄)Alkylsulfonyloxy, (C₁-C₄)Halogenalkylsulfanyl, (C₃-C₆)Cycloalkylsulfanyl, (C₃-C₆)Halogencycloalkylsulfanyl, (C₃-C₆)Cycloalkylsulfonyl, (C₃-C₆)Halogencycloalkylsulfonyl, (C₃-C₆)Cycloalkylsulfinyl, (C₃-C₆)Halogencycloalkylsulfinyl, (C₁-C₄)Alkylcarbonyl, (C₁-C₄)Halogenalkylcarbonyl, Aminocarbonyl, (C₁-C₄)Alkylaminocarbonyl, Di-(C₁-C₄)alkyl-aminocarbonyl, (C₁-C₄)Alkylsulfonylamino, (C₁-C₄)Alkylamino, Bis-(C₁-C₄)Alkylamino, (C₁-C₆)Halogenalkylamino, Bis-(C₁-C₆)Halogenalkylamino, Aminosulfonyl, (C₁-C₄)Alkylaminosulfonyl, Di-(C₁-C₄)alkylaminosulfonyl,
- R¹: für (C₁-C₆)Alkyl, (C₁-C₆)Halogenalkyl, (C₁-C₆)Cyanoalkyl, (C₁-C₆)Hydroxyalkyl, (C₁-C₄)Alkoxy-(C₁-C₄)alkyl, (C₁-C₄)Halogenalkoxy-(C₁-C₄)alkyl, (C₂-C₄)Alkenyl, (C₂-C₄)Alkenyloxy-(C₁-C₆)alkyl, (C₂-C₄)Halogenalkenyloxy-(C₁-C₆)alkyl, (C₂-C₄)Halogenalkenyl, (C₂-C₄)Cyanoalkenyl, (C₂-C₄)Alkinyl, (C₂-C₄)Alkinyloxy-(C₁-C₆)alkyl, (C₂-C₄)Halogenalkinyloxy-(C₁-C₄)alkyl, (C₂-C₄)Halogenalkinyl, (C₂-C₄)Cyanoalkinyl, (C₃-C₆)Cycloalkyl, (C₃-C₆)Cycloalkyl-(C₁-C₄)alkyl, (C₃-C₆)Cycloalkyl-(C₃-C₆)Cycloalkyl, (C₁-C₄)Alkyl-(C₃-C₆)Cycloalkyl, (C₁-C₄)Halogenalkyl-(C₃-C₆)cycloalkyl, Halogen(C₃-C₆)cycloalkyl, Cyano(C₃-C₆)cycloalkyl, (C₂-C₄)Alkinyl-(C₃-C₆)cycloalkyl, (C₁-C₄)Alkoxy-(C₃-C₆)cycloalkyl, (C₁-C₄)Alkoxycarbonyl-(C₃-C₆)cycloalkyl, (C₁-C₆)Alkylamino, Di-(C₁-C₆)alkyl-amino, (C₃-C₆)Cycloalkylamino, (C₁-C₄)Alkylthio-(C₁-C₆)alkyl, (C₁-C₄)Halogenalkylthio-(C₁-C₆)alkyl, (C₁-C₄)Alkylsulfinyl-(C₁-C₆)alkyl, (C₁-C₄)Halogenalkylsulfinyl-(C₁-C₆)alkyl, (C₁-C₄)Alkylsulfonyl-(C₁-C₆)alkyl, (C₁-C₄)Halogenalkylsulfonyl-(C₁-C₆)alkyl, (C₁-C₄)Alkylcarbonyl-(C₁-C₆)alkyl, (C₁-C₄)Alkoxycarbonyl-(C₁-C₆)alkyl, (C₁-C₄)Alkoxy, (C₃-C₆)Cycloalkoxy, (C₃-C₆)Cycloalkylcarbonyl oder (C₁-C₄)Alkylcarbonyl steht,
oder für jeweils gegebenenfalls einfach oder mehrfach, gleich oder verschieden substituiertes Aryl, Hetaryl, Oxo-Hetaryl, Heterocyclyl oder Oxo-Heterocyclyl steht, wobei die Substituenten ausgewählt sind aus Halogen, Cyano, Nitro, Hydroxy, Amino, Carboxy, Carbamoyl, (C₁-C₆)Alkyl, (C₃-C₆)Cycloalkyl, (C₁-C₄)-Alkoxy, (C₁-C₄)Halogenalkyl, (C₁-C₄)Halogenalkoxy, (C₁-C₄)Alkylthio, (C₁-C₄)Halogenalkylthio, (C₁-C₄)Alkylsulfinyl, (C₁-C₄)Alkylsulfonyl, (C₁-C₆)Alkoxycarbonyl, (C₁-C₆)Alkylcarbonyl, (C₃-C₆)Trialkylsilyl steht,
- R², R³: unabhängig voneinander für Wasserstoff, (C₁-C₆)Alkyl, (C₃-C₆)Cycloalkyl, (C₁-C₄)Alkoxy, (C₁-C₆)Alkoxy-(C₁-C₆)alkyl, (C₁-C₆)Alkylcarbonyl, (C₃-C₆)Cycloalkylcarbonyl, (C₁-C₆)Alkoxycarbonyl, (C₁-C₄)Halogenalkyl oder (C₁-C₄)Cyanoalkyl stehen,
- R⁴: für (C₁-C₆)Alkyl, (C₁-C₆)Halogenalkyl, (C₁-C₆)Cyanoalkyl, (C₁-C₆)Hydroxyalkyl, (C₁-C₆)Alkoxy-(C₁-C₆)alkyl, (C₁-C₆)Halogenalkoxy-(C₁-C₆)alkyl, (C₂-C₆)Alkenyl, (C₂-C₆)Alkenyloxy-(C₁-C₆)alkyl, (C₂-C₆)Halogenalkenyloxy-(C₁-C₆)alkyl, (C₂-C₆)Halogenalkenyl, (C₂-C₆)Cyanoalkenyl, (C₂-C₆)Alkinyl, (C₂-C₆)Alkinyloxy-(C₁-C₆)alkyl, (C₂-C₆)Halogenalkinyloxy-(C₁-C₆)alkyl, (C₂-C₆)Halogenalkinyl, (C₂-C₆)Cyanoalkinyl, (C₃-C₈)Cycloalkyl, (C₃-C₈)Cycloalkyl-(C₁-C₆)alkyl, Cyano(C₃-C₈)cycloalkyl-(C₁-C₆)alkyl, Halogen(C₃-C₈)cycloalkyl-(C₁-C₆)alkyl, (C₁-C₄)Halogenalkyl-(C₃-C₈)cycloalkyl-(C₁-C₆)alkyl, (C₃-C₈)Cycloalkyl-(C₃-C₈)Cycloalkyl, (C₁-C₆)Alkyl-(C₃-C₈)Cycloalkyl, (C₁-C₆)Halogenalkyl-(C₃-C₈)cycloalkyl, Halogen(C₃-C₈)cycloalkyl, Cyano(C₃-C₈)cycloalkyl, (C₂-C₆)Alkinyl-(C₃-C₈)cycloalkyl, (C₁-C₆)Alkoxy-(C₃-C₈)cycloalkyl, (C₁-C₆)Alkoxycarbonyl-(C₃-C₈)cycloalkyl, Carbamoyl-(C₃-C₈)cycloalkyl, Thiocarbamoyl-(C₃-C₈)cycloalkyl, (C₁-C₆)Alkylthio-(C₁-C₆)alkyl, (C₁-C₆)Halogenalkylthio-(C₁-C₆)alkyl, (C₁-C₆)Alkylsulfinyl-(C₁-C₆)alkyl, (C₁-C₆)Halogenalkylsulfinyl-(C₁-C₆)alkyl, (C₁-C₆)Alkylsulfonyl-(C₁-C₆)alkyl, (C₁-C₆)Halogenalkylsulfonyl-(C₁-C₆)alkyl, (C₁-C₆)Alkoxy-(C₁-C₆)alkylthio-(C₁-C₆)alkyl, (C₁-C₆)Alkoxy-(C₁-C₆)alkylsulfinyl-(C₁-C₆)alkyl, (C₁-C₆)Alkoxy-(C₁-C₆)alkylsulfonyl-(C₁-C₆)alkyl, (C₁-C₆)Alkylcarbonyl-(C₁-C₆)alkyl, (C₁-C₆)Halogenalkylcarbonyl-(C₁-C₆)alkyl, (C₁-C₆)Alkoxycarbonyl-(C₁-C₆)alkyl, (C₁-C₆)Halogenalkoxycarbonyl-(C₁-C₆)alkyl, (C₃-C₆)Cycloalkenyl, Halogen(C₃-C₆)Cycloalkenyl, (C₁-C₄)Alkyl-(C₃-C₆)Cycloalkenyl oder (C₁-C₄)Halogenalkyl-(C₃-C₆)Cycloalkenyl steht,
oder für durch Aryl, Hetaryl oder Oxo-Hetaryl substituiertes (C₁-C₆)Alkyl, (C₁-C₆)Halogenalkyl, (C₂-C₆)Alkenyl, (C₂-C₆)Alkinyl oder (C₃-C₈)Cycloalkyl steht, wobei Aryl, Hetaryl oder Oxo-Hetaryl jeweils gegebenenfalls einfach oder mehrfach, gleich oder verschieden durch Halogen, Cyano, Nitro, Hydroxy, Amino, Carboxy, Carbamoyl, Thiocarbamoyl, Aminosulfonyl, (C₁-C₆)Alkyl, (C₃-C₆)Cycloalkyl, (C₁-C₆)Alkoxy, (C₁-C₆)Halogenalkyl, (C₁-C₆)Halogenalkoxy, (C₁-C₆)Alkylthio, (C₁-C₆)Halogenalkylthio, (C₁-C₆)Alkylsulfinyl, (C₁-C₆)Alkylsulfonyl, (C₁-C₆)Halogenalkylsulfinyl, (C₁-C₆)Halogenalkylsulfonyl, (C₁-C₆)Alkylsulfimino, (C₁-C₆)Alkylsulfimino-(C₁-C₆)alkyl, (C₁-C₆)Alkylsulfimino-(C₂-C₆)alkylcarbonyl, (C₁-C₆)Alkylsulfoximino, (C₁-C₆)Alkylsulfoximino-(C₁-C₆)alkyl, (C₁-C₆)Alkoxycarbonyl oder (C₁-C₆)Alkylcarbonyl substituiert sein können,
oder für jeweils gegebenenfalls einfach oder mehrfach, gleich oder verschieden substituiertes Aryl, Hetaryl oder Oxo-Hetaryl steht, wobei die Substituenten ausgewählt sind aus Halogen, Cyano, Nitro, Hydroxy, Amino, Carboxy, Carbamoyl, Thiocarbamoyl, (C₁-C₆)Alkyl, (C₃-C₈)Cycloalkyl, (C₁-C₆)-Alkoxy, (C₁-C₆)Halogenalkyl, (C₁-C₆)Halogenalkoxy, (C₁-C₆)Alkylthio, (C₁-C₆)Halogenalkylthio, (C₁-C₆)Alkylsulfinyl, (C₁-C₆)Alkylsulfonyl, (C₁-C₆)Halogenalkylsulfinyl, (C₁-C₆)Halogenalkylsulfonyl, (C₁-C₆)Alkylsulfimino, (C₁-C₆)Alkylsulfimino-(C₁-C₆)alkyl, (C₁-C₆)Alkylsulfimino-(C₂-C₆)alkylcarbonyl, (C₁-C₆)Alkylsulfoximino, (C₁-C₆)Alkylsulfoximino-(C₁-C₆)alkyl, (C₁-C₆)Alkylsulfoximino-(C₂-C₆)alkylcarbonyl, (C₁-C₆)Alkoxycarbonyl oder (C₁-C₆)Alkylcarbonyl,
- R⁵, R⁶: unabhängig voneinander für Wasserstoff, Cyano, Halogen, Nitro, (C₃-C₆)Cycloalkyl, (C₁-C₄)Alkyl, (C₁-C₄)Halogenalkyl oder (C₁-C₄)Halogenalkoxy stehen,
- V¹, V²: unabhängig voneinander für Sauerstoff oder Schwefel stehen,
- A: für Stickstoff, =N⁺(O⁻)- oder =C(H)- steht.

### Besonders bevorzugt geeigneter Bereich:

Besonders bevorzugt und als Schädlingsbekämpfungsmittel grundsätzlich geeignet (Ausgestaltung 3) sind die Verbindungen der Formel (I), in denen
- Q¹: für jeweils gegebenenfalls einfach oder mehrfach, gleich oder verschieden substituiertes Phenyl oder Thiophen steht,
wobei die Substituenten ausgewählt sind aus Cyano, Halogen, Nitro, SF₅, (C₁-C₄)Alkyl, (C₁-C₄)Halogenalkyl, (C₂-C₄)Alkenyl, (C₂-C₄)Halogenalkenyl, (C₂-C₄)Alkinyl, (C₂-C₄)Halogenalkinyl, (C₁-C₄)Alkoxy, (C₁-C₄)Halogenalkoxy, (C₃-C₆)Cycloalkyl, (C₃-C₆)Halogencycloalkyl, (C₁-C₄)Alkyl-(C₃-C₆)cycloalkyl, (C₁-C₄)Halogenalkyl-(C₃-C₆)cycloalkyl, (C₁-C₄)Halogenalkenoxy, (C₁-C₄)Alkylsulfanyl, (C₃-C₆)Halogencycloalkylsulfanyl, (C₁-C₄)Alkylsulfinyl, (C₁-C₄)Halogenalkylsulfinyl, (C₃-C₆)Cycloalkylsulfinyl, (C₁-C₄)Alkylsulfonyl, (C₁-C₄)Halogenalkylsulfonyl, (C₃-C₆)Cycloalkylsulfonyl, (C₁-C₄)Halogenalkylsulfanyl, (C₃-C₆)Cycloalkylsulfanyl, (C₁-C₄)Alkylcarbonyl, (C₁-C₄)Halogenalkylcarbonyl, Bis-(C₁-C₄)Alkylamino, Bis-(C₁-C₄)Halogenalkylamino,
- R¹: für (C₁-C₄)Alkyl, (C₁-C₄)Halogenalkyl, (C₁-C₄)Hydroxyalkyl, (C₁-C₄)Alkoxy-(C₁-C₄)alkyl, (C₂-C₄)Alkenyl, (C₂-C₄)Halogenalkenyl, (C₂-C₄)Alkinyl, (C₂-C₄)Alkinyloxy-(C₁-C₄)alkyl, (C₃-C₆)Cycloalkyl, (C₃-C₆)Cycloalkyl-(C₁-C₄)alkyl, (C₃-C₆)Cycloalkyl-(C₃-C₆)Cycloalkyl, (C₁-C₄)Alkyl-(C₃-C₆)Cycloalkyl, (C₁-C₄)Halogenalkyl-(C₃-C₆)cycloalkyl, Halogen(C₃-C₆)cycloalkyl, Cyano(C₃-C₆)cycloalkyl, (C₂-C₄)Alkinyl-(C₃-C₆)cycloalkyl, (C₁-C₄)Alkoxy-(C₃-C₆)cycloalkyl, (C₁-C₄)Alkoxycarbonyl-(C₃-C₆)cycloalkyl, (C₁-C₄)Alkylthio-(C₁-C₆)alkyl, (C₁-C₄)Alkylcarbonyl-(C₁-C₆)alkyl, (C₁-C₄)Alkoxycarbonyl-(C₁-C₆)alkyl oder (C₁-C₄)Alkoxy steht,
oder für jeweils gegebenenfalls einfach oder mehrfach, gleich oder verschieden substituiertes Thienyl, Oxetanyl oder Thietanyl steht, wobei die Substituenten ausgewählt sind aus Halogen, Cyano, Nitro, (C₁-C₄)Alkyl, (C₃-C₆)Cycloalkyl, (C₁-C₄)-Alkoxy, (C₁-C₄)Halogenalkyl, (C₁-C₄)Halogenalkoxy, (C₁-C₄)Alkylthio, (C₁-C₄)Halogenalkylthio, (C₁-C₆)Alkoxycarbonyl,
- R², R³: unabhängig voneinander für Wasserstoff, (C₁-C₄)Alkyl, (C₃-C₆)Cycloalkyl, (C₁-C₄)Alkoxy, (C₁-C₆)Alkoxy-(C₁-C₆)alkyl, (C₁-C₄)Alkylcarbonyl, (C₃-C₆)Cycloalkylcarbonyl oder (C₁-C₄)Alkoxycarbonyl stehen,
- R⁴: für (C₁-C₆)Alkyl, (C₁-C₆)Halogenalkyl, (C₁-C₆)Cyanoalkyl, (C₁-C₆)Hydroxyalkyl, (C₁-C₆)Alkoxy-(C₁-C₆)alkyl, (C₁-C₆)Halogenalkoxy-(C₁-C₆)alkyl, (C₂-C₆)Alkenyl, (C₂-C₆)Halogenalkenyl, (C₂-C₆)Cyanoalkenyl, (C₂-C₆)Alkinyl, (C₂-C₆)Halogenalkinyl, (C₂-C₆)Cyanoalkinyl, (C₃-C₆)Cycloalkyl, (C₃-C₆)Cycloalkyl-(C₁-C₆)alkyl, Cyano(C₃-C₆)cycloalkyl-(C₁-C₆)alkyl, Halogen(C₃-C₈)cycloalkyl-(C₁-C₆)alkyl, (C₁-C₄)Halogenalkyl-(C₃-C₆)cycloalkyl-(C₁-C₆)alkyl, (C₃-C₆)Cycloalkyl-(C₃-C₆)Cycloalkyl, (C₁-C₆)Alkyl-(C₃-C₆)Cycloalkyl, (C₁-C₆)Halogenalkyl-(C₃-C₆)cycloalkyl, Halogen(C₃-C₆)cycloalkyl, Cyano(C₃-C₆)cycloalkyl, (C₂-C₆)Alkinyl-(C₃-C₆)cycloalkyl, (C₁-C₆)Alkoxy-(C₃-C₆)cycloalkyl, (C₁-C₆)Alkoxycarbonyl-(C₃-C₆)cycloalkyl, Carbamoyl-(C₃-C₆)cycloalkyl, Thiocarbamoyl-(C₃-C₆)cycloalkyl, (C₁-C₆)Alkylthio-(C₁-C₆)alkyl, (C₁-C₆)Halogenalkylthio-(C₁-C₆)alkyl, (C₁-C₆)Alkylsulfinyl-(C₁-C₆)alkyl, (C₁-C₆)Halogenalkylsulfinyl-(C₁-C₆)alkyl, (C₁-C₆)Alkylsulfonyl-(C₁-C₆)alkyl, (C₁-C₆)Halogenalkylsulfonyl-(C₁-C₆)alkyl, (C₁-C₆)Halogenalkylcarbonyl-(C₁-C₆)alkyl, (C₁-C₆)Alkoxycarbonyl-(C₁-C₆)alkyl, (C₁-C₆)Halogenalkoxycarbonyl-(C₁-C₆)alkyl, (C₃-C₆)Cycloalkenyl, Halogen(C₃-C₆)Cycloalkenyl, (C₁-C₄)Alkyl-(C₃-C₆)Cycloalkenyl oder (C₁-C₄)Halogenalkyl-(C₃-C₆)Cycloalkenyl steht,
oder für durch Phenyl oder Pyridyl substituiertes (C₁-C₆)Alkyl, (C₁-C₆)Halogenalkyl oder (C₃-C₈)Cycloalkyl steht, wobei Phenyl oder Pyridyl jeweils gegebenenfalls einfach oder mehrfach, gleich oder verschieden durch Halogen, Cyano, Nitro, Hydroxy, Amino, Carboxy, Carbamoyl, Thiocarbamoyl, Aminosulfonyl, (C₁-C₆)Alkyl, (C₃-C₆)Cycloalkyl, (C₁-C₆)Alkoxy, (C₁-C₆)Halogenalkyl, (C₁-C₆)Halogenalkoxy, (C₁-C₆)Alkylthio, (C₁-C₆)Halogenalkylthio, (C₁-C₆)Alkylsulfinyl, (C₁-C₆)Alkylsulfonyl, (C₁-C₆)Halogenalkylsulfinyl, (C₁-C₆)Halogenalkylsulfonyl, (C₁-C₆)Alkylsulfimino, (C₁-C₆)Alkylsulfoximino, (C₁-C₆)Alkoxycarbonyl oder (C₁-C₆)Alkylcarbonyl substituiert sein können,
oder für jeweils gegebenenfalls einfach oder mehrfach, gleich oder verschieden substituiertes Aryl oder Hetaryl steht, wobei die Substituenten ausgewählt sind aus Halogen, Cyano, Nitro, Hydroxy, Amino, Carboxy, Carbamoyl, Thiocarbamoyl, (C₁-C₆)Alkyl, (C₃-C₈)Cycloalkyl, (C₁-C₆)-Alkoxy, (C₁-C₆)Halogenalkyl, (C₁-C₆)Halogenalkoxy, (C₁-C₆)Alkylthio, (C₁-C₆)Halogenalkylthio, (C₁-C₆)Alkylsulfinyl, (C₁-C₆)Alkylsulfonyl, (C₁-C₆)Halogenalkylsulfinyl, (C₁-C₆)Halogenalkylsulfonyl, (C₁-C₆)Alkylsulfimino, (C₁-C₆)Alkylsulfoximino, (C₁-C₆)Alkoxycarbonyl oder (C₁-C₆)Alkylcarbonyl,
- R⁵, R⁶: unabhängig voneinander für Wasserstoff, Cyano, Halogen, Methyl, Ethyl, Difluormethyl, Trifluormethyl oder Trifluormethoxy stehen,
- V¹, V²: unabhängig voneinander für Sauerstoff oder Schwefel stehen,
- A: für Stickstoff, =N⁺(O⁻)- oder =C(H)- steht.

### Gegenstand der Erfindung:

Gegenstand der vorliegenden Erfindung (**Ausgestaltung 4**) sind die Verbindungen der Formel (I), in denen
- Q¹: für eine Substruktur der Formel (Q¹-A) steht: 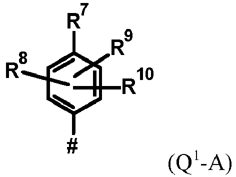
wobei Q¹-A an der mit # gekennzeichneten Stelle mit dem Sechsring gemäß Formel (I) verbunden ist,
- R⁷: für Fluormethyl, Difluormethyl, Trifluormethyl, Fluorethyl, Difluorethyl, Trifluorethyl, Tetrafluorethyl, Pentafluorethyl, Trifluormethoxy, Difluormethoxy, Difluorchlormethoxy, Dichlorfluormethoxy, Pentafluorethoxy, Tetrafluorethoxy, Trifluorethoxy, Difluorethoxy Chlortetrafluorethoxy, Dichlortrifluorethoxy, Trichlordifluorethoxy, Chlortrifluorethoxy, Chlordifluorethoxy, Trifluormethylsulfonyl, Trifluormethylsulfinyl, Trifluormethylsulfanyl, Difluormethylsulfonyl, Difluormethylsulfinyl, Difluormethylsulfanyl, Pentafluorethylsulfonyl, Pentafluorethylsulfinyl, Pentafluorethylsulfanyl, Trifluorethylsulfonyl, Trifluorethylsulfinyl, Trifluorethylsulfanyl, Difluorethylsulfonyl, Difluorethylsulfinyl, Difluorethylsulfanyl, Tetrafluorethylsulfonyl, Tetrafluorethylsulfinyl, Tetrafluorethylsulfanyl, Trifluormethylcyclopropyl oder SF₅ steht,
- R⁸, R⁹, R¹⁰: unabhängig voneinander für Wasserstoff, Cyano, Fluor, Chlor, Brom, Iod, Methyl, Ethyl, n-Propyl, i-Propyl, Cyclopropyl, Trifluormethyl, Methoxy oder Ethoxy stehen.
- R¹: für (C₁-C₄)Alkyl, (C₁-C₄)Halogenalkyl, (C₁-C₄)Alkoxy-(C₁-C₄)alkyl, (C₂-C₄)Alkenyl, (C₂-C₄)Alkinyl, (C₃-C₆)Cycloalkyl, (C₃-C₆)Cycloalkyl-(C₁-C₄)alkyl, (C₃-C₆)Cycloalkyl-(C₃-C₆)Cycloalkyl, (C₁-C₄)Alkyl-(C₃-C₆)Cycloalkyl, (C₁-C₄)Halogenalkyl-(C₃-C₆)cycloalkyl, Halogen(C₃-C₆)cycloalkyl, Cyano(C₃-C₆)cycloalkyl oder (C₁-C₄)Alkoxy-(C₃-C₆)cycloalkyl steht,
- R², R³: unabhängig voneinander für Wasserstoff, Methyl, Ethyl, n-Propyl, i-Propyl, cyclo-Propyl, n-Butyl, i-Butyl, tert.-Butyl, cyclo-Butyl, Methoxy, Ethoxy, Methoxymethyl, Ethoxymethyl, Methoxycarbonyl, Ethoxycarbonyl, n-Propoxycarbonyl oder i-Propoxycarbonyl stehen,
- R⁴: für (C₁-C₆)Alkyl, (C₁-C₆)Halogenalkyl, (C₁-C₆)Cyanoalkyl, (C₁-C₄)Alkoxy-(C₁-C₄)alkyl, (C₁-C₄)Halogenalkoxy-(C₁-C₄)alkyl, (C₂-C₄)Alkenyl, (C₂-C₄)Halogenalkenyl, (C₂-C₄)Cyanoalkenyl, (C₂-C₄)Alkinyl, (C₂-C₆)Halogenalkinyl, (C₂-C₆)Cyanoalkinyl, (C₃-C₆)Cycloalkyl, (C₃-C₆)Cycloalkyl-(C₁-C₄)alkyl, Cyano(C₃-C₆)cycloalkyl-(C₁-C₄)alkyl, Halogen(C₃-C₆)cycloalkyl-(C₁-C₄)alkyl, (C₁-C₄)Halogenalkyl-(C₃-C₆)cycloalkyl-(C₁-C₄)alkyl, (C₃-C₆)Cycloalkyl-(C₃-C₆)Cycloalkyl, (C₁-C₄)Alkyl-(C₃-C₆)Cycloalkyl, (C₁-C₄)Halogenalkyl-(C₃-C₆)cycloalkyl, Halogen(C₃-C₆)cycloalkyl, Cyano(C₃-C₆)cycloalkyl, (C₂-C₄)Alkinyl-(C₃-C₆)cycloalkyl, (C₁-C₄)Alkoxy-(C₃-C₆)cycloalkyl, Carbamoyl-(C₃-C₈)cycloalkyl, Thiocarbamoyl-(C₃-C₈)cycloalkyl, (C₃-C₆)Cycloalkenyl, Halogen(C₃-C₆)Cycloalkenyl, (C₁-C₄)Alkyl-(C₃-C₆)Cycloalkenyl oder (C₁-C₄)Halogenalkyl-(C₃-C₆)Cycloalkenyl steht,
oder für durch Phenyl oder Pyridyl substituiertes (C₁-C₄)Alkyl, (C₁-C₄)Halogenalkyl oder (C₃-C₆)Cycloalkyl steht, wobei Phenyl oder Pyridyl jeweils gegebenenfalls einfach oder mehrfach, gleich oder verschieden durch Halogen, Cyano, (C₁-C₄)Alkyl, (C₃-C₆)Cycloalkyl, (C₁-C₄)Alkoxy, (C₁-C₄)Halogenalkyl, (C₁-C₄)Halogenalkoxy, (C₁-C₄)Alkylthio, (C₁-C₄)Halogenalkylthio, (C₁-C₄)Alkylsulfinyl, (C₁-C₄)Alkylsulfonyl, (C₁-C₄)Halogenalkylsulfinyl oder (C₁-C₄)Halogenalkylsulfonyl substituiert sein können,
oder für jeweils gegebenenfalls einfach oder mehrfach, gleich oder verschieden substituiertes Phenyl oder Pyridyl steht, wobei die Substituenten ausgewählt sind aus Halogen, Cyano, (C₁-C₄)Alkyl, (C₃-C₆)Cycloalkyl, (C₁-C₄)-Alkoxy, (C₁-C₄)Halogenalkyl, (C₁-C₄)Halogenalkoxy, (C₁-C₄)Alkylthio, (C₁-C₄)Halogenalkylthio, (C₁-C₄)Alkylsulfinyl, (C₁-C₄)Alkylsulfonyl, (C₁-C₄)Halogenalkylsulfinyl, (C₁-C₄)Halogenalkylsulfonyl,
- R⁵, R⁶: unabhängig voneinander für Wasserstoff, Cyano, Fluor, Chlor, Brom, oder Methyl stehen,
- V¹, V²: unabhängig voneinander für Sauerstoff oder Schwefel stehen,
- A: für Stickstoff, =N⁺(O⁻)- oder =C(H)- steht.

### Bevorzugter Bereich der Erfindung:

Bevorzugter Bereich der Erfindung (**Ausgestaltung 5**) sind die Verbindungen der Formel (I), in denen
- Q¹: für eine Substruktur der Formel (Q¹-A) steht:

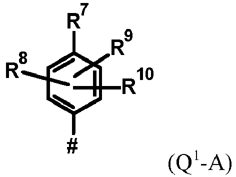

- wobei Q¹-A: an der mit # gekennzeichneten Stelle mit dem Sechsring gemäß Formel (I) verbunden ist,
- R⁷: für Trifluormethyl, Difluormethyl, Difluorethyl, Trifluorethyl, Pentafluorethyl, Trifluormethoxy, Difluormethoxy, Pentafluorethoxy, Trifluorethoxy, Trifluormethylsulfonyl, Trifluormethylsulfinyl, Trifluormethylsulfanyl, Difluormethylsulfonyl, Difluormethylsulfinyl, Difluormethylsulfanyl, Trifluormethylcyclopropyl oder SF₅ steht,
- R⁸, R⁹, R¹⁰: unabhängig voneinander für Wasserstoff, Fluor, Chlor oder Methyl stehen.
- R¹: für Methyl, Ethyl, Isopropyl oder Cyclopropyl steht,
- R²: für Wasserstoff, Ethyl oder Methoxycarbonyl steht,
- R³: für Wasserstoff, Methyl, Ethyl, Isopropyl oder Methoxycarbonyl steht,
- R⁴: für Methyl, Ethyl, Propyl, Butyl, Isopropyl, Isobutyl, tert-Butyl, Trifluormethyl, Cyanomethyl, Cyanoethyl, Cyanopropyl, Cyanobutyl, Cyanopentyl, (Trifluormethyl)propyl, Fluorpropyl, Chlorpropyl, Phenylpropyl, Cyclopropyl, Cyanocyclopropyl, Fluorcyclopropyl, Chlorcyclopropyl, Methylcyclopropyl, (Trifluormethyl)cyclopropyl, Cyclopropylcyclopropyl, Dicyclopropylcyclopropyl, Phenylcyclopropyl, Cyclobutyl, Cyclopentyl, Fluorcyclopentyl, Chlorcyclopentyl, Methylcyclopentyl, (Trifluormethyl)cyclopentyl, [(Trifluormethyl)phenyl]methyl, (Cyanophenyl)methyl, (Fluorphenyl)methyl, (Chlorphenyl)methyl, Phenyl, Cyanophenyl, Fluorphenyl, Chlorphenyl oder Methylcycloprop-2-en-1-yl steht,
- R⁵: für Wasserstoff oder Chlor steht,
- R⁶: für Wasserstoff steht,
- V¹, V²: unabhängig voneinander für Sauerstoff oder Schwefel stehen,
- A: für Stickstoff, oder =C(H)- steht.

### Besonders bevorzugter Bereich der Erfindung:

Besonders bevorzugter Bereich der Erfindung (**Ausgestaltung 6**) sind die Verbindungen der Formel (I), in denen
- Q¹: für 4-(Trifluormethoxy)phenyl 4-(Pentafluorethyl)phenyl, 3-Fluor-4-(pentafluorethyl)phenyl, 2-Chlor-3-fluor-4-(pentafluorethyl)phenyl, 3-Fluor-5-methyl-4-(pentafluorethyl)phenyl, 2,6-Difluor-4-(pentafluorethyl)phenyl, 2-Chlor-5-fluor-4-(pentafluorethyl)phenyl, 3-Methyl-4-(pentafluorethyl)phenyl, 3,5-Difluor-4-(pentafluorethyl)phenyl, 2,3-Difluor-4-(pentafluorethyl)phenyl, 3-Chlor-4-(pentafluorethyl)phenyl, 2,3,5-Trifluor-4-(pentafluorethyl)phenyl, 2,5-Difluor-4-(pentafluorethyl)phenyl, 2,6-Dichlor-3-fluor-4-(pentafluorethyl)phenyl, 3-Chlor-5-fluor-4-(pentafluorethyl)phenyl oder 2,3,6-Trifluor-4-(pentafluorethyl)phenyl steht,
- R¹: für Methyl, Ethyl, Isopropyl oder Cyclopropyl steht,
- R²: für Wasserstoff, Ethyl oder Methoxycarbonyl steht,
- R³: für Wasserstoff, Methyl, Ethyl, Isopropyl oder Methoxycarbonyl steht,
- R⁴: für Cyclopropyl, [4-(Trifluormethyl)phenyl]methyl, (3-Cyanophenyl)methyl, 1-methylcyclopropyl, 1-Cyanocyclopropyl, 1-(Trifluormethyl)cyclopropyl, (4-Cyanophenyl)methyl, 1-Phenylcyclopropyl, 2-Cyanopropan-2-yl, 2-Phenylpropan-2-yl, 1-Methylcyclopentyl, 1-Fluorcyclopropyl, 1-Chlorcyclopropyl, 1-Cyclopropylcyclopropyl, 2-Fluorpropan-2-yl, 1,1,1-Trifluor-2-methylpropan-2-yl, tert-Butyl, Isopropyl, Butyl, Ethyl, Cyclopentyl, Methyl, Propyl, Cyanomethyl, 1-Cyanoethan-2-yl, Phenyl, 4-Cyanophenyl, 2,2-Dicyclopropylcyclopropyl oder 2-Methylcycloprop-2-en-1-yl steht,
- R⁵: für Wasserstoff oder Chlor steht,
- R⁶: für Wasserstoff steht,
- V¹, V²: unabhängig voneinander für Sauerstoff oder Schwefel stehen,
- A: für Stickstoff, oder =C(H)- steht.

### Weitere bevorzugte, besonders bevorzugte und ganz besonders bevorzugte Bereiche:

In einer weiteren bevorzugten Ausführungsform betrifft die Erfindung die Verbindungen der allgemeinen Formeln (I-a) 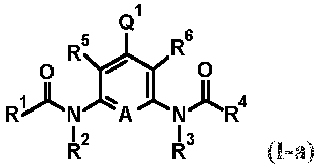 wobei R¹, R², R³, R⁴, R⁵, R⁶, Q¹ und A die oben beschriebene Bedeutung haben, insbesondere die in Ausgestaltung (4) oder die in Ausgestaltung (5) oder die in Ausgestaltung (6) beschriebene Bedeutung haben.

In einer weiteren bevorzugten Ausführungsform betrifft die Erfindung die Verbindungen der allgemeinen Formeln (I-b) 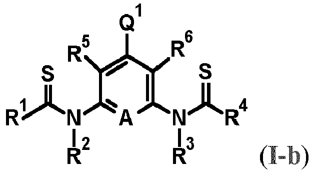 wobei R¹, R², R³, R⁴, R⁵, R⁶, Q¹ und A die oben beschriebene Bedeutung haben, insbesondere die in Ausgestaltung (4) oder die in Ausgestaltung (5) oder die in Ausgestaltung (6) beschriebene Bedeutung haben.

In einer weiteren bevorzugten Ausführungsform betrifft die Erfindung die Verbindungen der allgemeinen Formeln (I), wobei A für Stickstoff steht und wobei R¹, R², R³, R⁴, R⁵, R⁶, Q¹, V¹ und V² die oben beschriebene Bedeutung haben, insbesondere die in Ausgestaltung (4) oder die in Ausgestaltung (5) oder die in Ausgestaltung (6) beschriebene Bedeutung haben.

In einer weiteren bevorzugten Ausführungsform betrifft die Erfindung die Verbindungen der allgemeinen Formeln (I), wobei A für =C(H)- steht und wobei R¹, R², R³, R⁴, R⁵, R⁶, Q¹, V¹ und V² die oben beschriebene Bedeutung haben, insbesondere die in Ausgestaltung (4) oder die in Ausgestaltung (5) oder die in Ausgestaltung (6) beschriebene Bedeutung haben.

In einer weiteren bevorzugten Ausführungsform betrifft die Erfindung die Verbindungen der allgemeinen Formeln (I), wobei R¹ für Cyclopropyl steht und wobei R², R³, R⁴, R⁵, R⁶, Q¹, A, V¹ und V² die oben beschriebene Bedeutung haben, insbesondere die in Ausgestaltung (1) oder die in Ausgestaltung (2) oder die in Ausgestaltung (3) oder die in Ausgestaltung (4) oder die in Ausgestaltung (5) oder die in Ausgestaltung (6) beschriebene Bedeutung haben.

In einer weiteren bevorzugten Ausführungsform betrifft die Erfindung die Verbindungen der allgemeinen Formeln (I), wobei R⁴ für ggf. substituiertes Cyclopropyl steht und wobei R¹, R², R³, R⁵, R⁶, Q¹, A, V¹ und V² die oben beschriebene Bedeutung haben, insbesondere die in Ausgestaltung (1) oder die in Ausgestaltung (2) oder die in Ausgestaltung (3) oder die in Ausgestaltung (4) oder die in Ausgestaltung (5) oder die in Ausgestaltung (6) beschriebene Bedeutung haben.

In einer weiteren bevorzugten Ausführungsform betrifft die Erfindung die Verbindungen der allgemeinen Formeln (I), wobei R¹ und R⁴ für ggf. substituiertes Cyclopropyl stehen und wobei R², R³, R⁵, R⁶, Q¹, A, V¹ und V² die oben beschriebene Bedeutung haben, insbesondere die in Ausgestaltung (1) oder die in Ausgestaltung (2) oder die in Ausgestaltung (3) oder die in Ausgestaltung (4) oder die in Ausgestaltung (5) oder die in Ausgestaltung (6) beschriebene Bedeutung haben.

In einer weiteren bevorzugten Ausführungsform betrifft die Erfindung die Verbindungen der allgemeinen Formeln (I), wobei Q¹ für die Substruktur (Q¹-A) steht, wobei die Reste R⁷, R⁸, R⁹, R¹⁰ die in Ausgestaltung 4 oder 5 angegebene Bedeutung haben, oder wobei Q¹ besonders bevorzugt die in Ausgestaltung 6 beschriebene Bedeutung hat, und wobei R¹, R², R³, R⁴, R⁵, R⁶, A, V¹ und V² die oben beschriebene Bedeutung haben, insbesondere die in Ausgestaltung (1) oder die in Ausgestaltung (2) oder die in Ausgestaltung (3) oder die in Ausgestaltung (4) oder die in Ausgestaltung (5) oder die in Ausgestaltung (6) beschriebene Bedeutung haben.

Ebenfalls ganz besonders bevorzugte erfindungsgemäße Verbindungen sind die in Tabelle 1 gezeigten Verbindungen der allgemeinen Formel (I).

Gegebenenfalls substituierte Reste können einfach oder mehrfach substituiert sein, wobei bei einer Mehrfachsubstitution die Substituenten gleich oder verschieden sein können.

Die oben aufgeführten allgemeinen oder in Vorzugsbereichen aufgeführten Restedefinitionen bzw. Erläuterungen gelten für die Endprodukte und für die Ausgangsprodukte und Zwischenprodukte entsprechend. Diese Restedefinitionen können untereinander, also auch zwischen den jeweiligen Vorzugsbereichen, beliebig kombiniert werden.

Erfindungsgemäß bevorzugt werden Verbindungen der Formel (I), in welchen eine Kombination der vorstehend als bevorzugt aufgeführten Bedeutungen vorliegt.

Erfindungsgemäß besonders bevorzugt werden Verbindungen der Formel (I), in welchen eine Kombination der vorstehend als besonders bevorzugt aufgeführten Bedeutungen vorliegt.

Erfindungsgemäß ganz besonders bevorzugt werden Verbindungen der Formel (I), in welchen eine Kombination der vorstehend als ganz besonders bevorzugt aufgeführten Bedeutungen vorliegt.

Die Verbindungen der Formel (I) können gegebenenfalls auch in Abhängigkeit von der Art der Substituenten als Stereoisomere, d.h. als geometrische und/oder als optische Isomere oder Isomerengemische in unterschiedlichen Zusammensetzungen vorliegen. Diese Stereoisomere sind beispielsweise Enantiomere, Diastereomere, Atropisomere oder geometrische Isomere. Sowohl die reinen Stereoisomeren als auch beliebige Gemische dieser Isomeren sind Gegenstand dieser Erfindung, auch wenn hier im Allgemeinen nur von Verbindungen der Formel (I) die Rede ist.

Vorzugsweise werden jedoch die optisch aktiven, stereoisomeren Formen der Verbindungen der Formel (I) und deren Salze erfindungsgemäß verwendet.

Die Erfindung betrifft daher sowohl die reinen Enantiomeren und Diastereomeren, als auch deren Gemische zur Bekämpfung von tierischen Schädlingen, zu denen Arthropoden und insbesondere Insekten zählen.

Die Verbindungen der Formel (I) können gegebenenfalls in verschiedenen polymorphen Formen oder als Mischung verschiedener polymorpher Formen vorliegen. Sowohl die reinen Polymorphe als auch die Polymorphgemische sind Gegenstand der Erfindung und können erfindungsgemäß verwendet werden.

Die vorliegenden Verbindungen der allgemeinen Formel (I) können gegebenenfalls ein chirales Kohlenstoffatom aufweisen.

Gemäß den Regeln nach Cahn, Ingold und Prelog (CIP-Regeln) können diese Substituenten sowohl eine (R)- als auch eine (S)-Konfiguration aufweisen.

Von der vorliegenden Erfindung werden Verbindungen der allgemeinen Formel (I) sowohl mit (S)- als auch mit (R)-Konfiguration an den jeweiligen chiralen Kohlenstoffatomen umfasst, d.h., dass die vorliegende Erfindung die Verbindungen der allgemeinen Formel (I) erfasst, in welchen die betreffenden Kohlenstoffatome jeweils unabhängig voneinander
(1) eine (R)-Konfiguration; oder
(2) eine (S)-Konfiguration
aufweisen. Wenn mehrere Chiralitätszentren in den Verbindungen der allgemeinen Formel (I) vorliegen, sind beliebige Kombinationen der Konfigurationen der Chiralitätszentren möglich, d.h. dass
(1) ein Chiralitätszentrum die (R)-Konfiguration und das andere Chiralitätszentrum die (S)-Konfiguration;
(2) ein Chiralitätszentrum die (R)-Konfiguration und das andere Chiralitätszentrum die (R)-Konfiguration; und
(3) ein Chiralitätszentrum die (S)-Konfiguration und das andere Chiralitätszentrum die (S)-Konfiguration
aufweisen kann.

In den allgemeinen oder in Vorzugsbereichen aufgeführten Definitionen ist, sofern nichts anderes angegeben ist, Halogen ausgewählt aus der Reihe Fluor, Chlor, Brom und Iod, bevorzugt wiederum aus der Reihe Fluor, Chlor und Brom,

Sofern nicht an anderer Stelle anders definiert, wird unter dem Begriff "Alkyl", entweder in Alleinstellung oder aber in Kombination mit weiteren Begriffen, wie beispielsweise Halogenalkyl, im Rahmen der vorliegenden Erfindung ein Rest einer gesättigten, aliphatischen Kohlenwasserstoffgruppe mit 1 bis 12 Kohlenstoffatomen verstanden, die verzweigt oder unverzweigt sein kann. Beispiele für C₁-C₁₂-Alkylreste sind Methyl, Ethyl, n-Propyl, iso-Propyl, n-Butyl, iso-Butyl, sek.-Butyl, tert.-Butyl, n-Pentyl, iso-Pentyl, Neopentyl, tert.-Pentyl, 1-Methylbutyl, 2-Methylbutyl, 1-Ethylpropyl, 1,2-Dimethylpropyl, Hexyl n-Heptyl, n-Octyl, n-Nonyl, n-Decyl, n-Undecyl und n-Dodecyl. Von diesen Alkylresten sind C₁-C₆-Alkylreste besonders bevorzugt. Insbesondere bevorzugt sind C₁-C₄-Alkylreste.

Sofern nicht an anderer Stelle anders definiert, wird unter dem Begriff "Alkenyl", entweder in Alleinstellung oder aber in Kombination mit weiteren Begriffen, erfindungsgemäß ein linearer oder verzweigter C₂-C₁₂-Alkenylrest, welcher mindestens eine Doppelbindung aufweist, beispielsweise Vinyl, Allyl, 1-Propenyl, Isopropenyl, 1-Butenyl, 2-Butenyl, 3-Butenyl, 1,3-Butadienyl, 1-Pentenyl, 2-Pentenyl, 3-Pentenyl, 4-Pentenyl, 1,3-Pentadienyl, 1-Hexenyl, 2-Hexenyl, 3-Hexenyl, 4-Hexenyl, 5-Hexenyl und 1,4-Hexadienyl, verstanden. Bevorzugt hiervon sind C₂-C₆-Alkenylreste und besonders bevorzugt sind C₂-C₄-Alkenylreste.

Sofern nicht an anderer Stelle anders definiert, wird unter dem Begriff "Alkinyl", entweder in Alleinstellung oder aber in Kombination mit weiteren Begriffen, erfindungsgemäß ein linearer oder verzweigter C₂-C₁₂-Alkinylrest, welcher mindestens eine Dreifachbindung aufweist, beispielsweise Ethinyl, 1-Propinyl und Propargyl, verstanden. Bevorzugt hiervon sind C₃-C₆-Alkinylreste und besonders bevorzugt sind C₃-C₄-Alkinylreste. Der Alkinylrest kann dabei auch mindestens eine Doppelbindung aufweisen.

Sofern nicht an anderer Stelle anders definiert, wird unter dem Begriff "Cycloalkyl", entweder in Alleinstellung oder aber in Kombination mit weiteren Begriffen, erfindungsgemäß ein C₃-C₈-Cycloalkylrest verstanden, beispielsweise Cyclopropyl, Cyclobutyl, Cyclopentyl, Cyclohexyl, Cycloheptyl und Cyclooctyl, verstanden. Bevorzugt hiervon sind C₃-C₆-Cycloalkylreste.

Unter dem Begriff **"Alkoxy"** oder "**Alkyloxy**", entweder in Alleinstellung oder aber in Kombination mit weiteren Begriffen, wie beispielsweise Halogenalkoxy, wird vorliegend ein Rest O-Alkyl verstanden, wobei der Begriff "Alkyl" die oben stehende Bedeutung aufweist.

Sofern nicht an anderer Stelle anders definiert, bedeutet "**Oxy**" die Gruppe -O-.

Durch Halogen substituierte Reste, z.B. Halogenalkyl (=Haloalkyl), sind einfach oder mehrfach bis zur maximal möglichen Substituentenzahl halogeniert. Bei mehrfacher Halogenierung können die Halogenatome gleich oder verschieden sein. Halogen steht dabei für Fluor, Chlor, Brom oder Iod, insbesondere für Fluor, Chlor oder Brom.

Sofern nicht an anderer Stelle anders definiert, wird unter dem Begriff **"Aryl"** erfindungsgemäß ein aromatischer Rest mit 6 bis 14 Kohlenstoffatomen, vorzugsweise Phenyl, verstanden.

Sofern nicht an anderer Stelle anders definiert, wird unter dem Begriff **"Arylalkyl"** eine Kombination von erfindungsgemäß definierten Resten "Aryl" und "Alkyl" verstanden, wobei der Rest im Allgemeinen über die Alkylgrupe gebunden wird, Beispiele hierfür sind Benzyl, Phenylethyl oder α-Methylbenzyl, wobei Benzyl besonders bevorzugt ist.

Sofern nicht an anderer Stelle anders definiert, bedeutet **"Hetaryl"** eine mono-, bi- oder tricyclische heterocyclische Gruppe aus C-Atomen und mindestens einem Heteroatom, wobei mindestens ein Zyklus aromatisch ist. Bevorzugt enthält die Hetaryl-Gruppe 3, 4, 5 oder 6 C-Atome. Bevorzugt steht Hetaryl für Pyridinyl oder Pyrimidinyl.

Sofern nicht an anderer Stelle anders definiert, bedeutet **"Heterocyclyl"** einen monocyclischen, gesättigten oder teilgesättigten 4-, 5-, 6- oder 7-gliedrigen Ring aus C-Atomen und mindestens einem Heteroatom im Ring. Bevorzugt enthält die Heterocyclyl-Gruppe 3, 4, 5 oder 6 C-Atome und 1 oder 2 Heteroatome aus der Reihe Sauerstoff, Schwefel oder Stickstoff. Beispiele für Heterocyclyl sind Azetidinyl, Azolidinyl, Azinanyl, Oxetanyl, Oxolanyl, Oxanyl, Dioxanyl, Thiethanyl, Thiolanyl, Thianyl, Tetrahydrofuryl.

Sofern nicht an anderer Stelle anders definiert, bedeutet **"Oxo-Hetaryl"** ein Hetaryl, das an mindestens einer Position im Ring ein Ring-Kohlenstoffatom enthält, das mit einer (=O) -Gruppe substituiert ist, wobei sich dann die Gruppe -C(=O)- ergibt, wobei das C-Atom Bestandteil des Rings ist.

Sofern nicht an anderer Stelle anders definiert, bedeutet **"Oxo-Heterocyclyl"** ein Heterocyclyl, das an mindestens einer Position im Ring ein Ring-Kohlenstoffatom enthält, das mit einer (=O) -Gruppe substituiert ist, wobei sich dann die Gruppe -C(=O)- ergibt, wobei das C-Atom Bestandteil des Rings ist. Bevorzugt enthält Oxo-Heterocyclyl eine oder zwei -C(=O)- Gruppen. Als Beispiel sei Oxotetrahydrofuranyl oder Pyrrolidinonyl genannt.

### Isomere

Die Verbindungen der Formel (I) können in Abhängigkeit von der Art der Substituenten als geometrische und/oder als optisch aktive Isomere oder entsprechende Isomerengemische in unterschiedlicher Zusammensetzung vorliegen. Diese Stereoisomere sind beispielsweise Enantiomere, Diastereomere, Atropisomere oder geometrische Isomere. Die Erfindung umfasst somit sowohl reine Stereoisomere als auch beliebige Gemische dieser Isomere.

### Verfahren und Verwendungen

Die Erfindung betrifft auch Verfahren zur Bekämpfung von tierischen Schädlingen, bei dem man Verbindungen der Formel (I) auf tierische Schädlinge und/oder ihren Lebensraum einwirken lässt. Bevorzugt wird die Bekämpfung der tierischen Schädlinge in der Land- und Forstwirtschaft und im Materialschutz durchgeführt. Hierunter vorzugsweise ausgeschlossen sind Verfahren zur chirurgischen oder therapeutischen Behandlung des menschlichen oder tierischen Körpers und Diagnostizierverfahren, die am menschlichen oder tierischen Körper vorgenommen werden.

Die Erfindung betrifft ferner die Verwendung der Verbindungen der Formel (I) als Schädlingsbekämpfungsmittel, insbesondere Pflanzenschutzmittel.

Im Rahmen der vorliegenden Anmeldung umfasst der Begriff Schädlingsbekämpfungsmittel jeweils immer auch den Begriff Pflanzenschutzmittel.

Die Verbindungen der Formel (I) eignen sich bei guter Pflanzenverträglichkeit, günstiger Warmblütertoxizität und guter Umweltverträglichkeit zum Schutz von Pflanzen und Pflanzenorganen vor biotischen und abiotischen Stressfaktoren, zur Steigerung der Ernteerträge, Verbesserung der Qualität des Erntegutes und zur Bekämpfung von tierischen Schädlingen, insbesondere Insekten, Spinnentieren, Helminthen, insbesondere Nematoden, und Mollusken, die in der Landwirtschaft, im Gartenbau, bei der Tierzucht, in Aquakulturen, in Forsten, in Gärten und Freizeiteinrichtungen, im Vorrats- und Materialschutz sowie auf dem Hygienesektor vorkommen.

Im Rahmen der vorliegenden Patentanmeldung ist der Begriff "Hygiene" so zu verstehen, dass damit jegliche und alle Maßnahmen, Vorschriften und Verfahrensweisen gemeint sind, deren Ziel es ist, Krankheiten, insbesondere Infektionskrankheiten, zu verhindern, und die dazu dienen, die Gesundheit von Menschen und Tieren zu schützen und/oder die Umwelt zu schützen, und/oder die Sauberkeit aufrechterhalten. Erfindungsgemäß schließt dies insbesondere Maßnahmen zur Reinigung, Desinfektion und Sterilisation beispielsweise von Textilien oder harten Oberflächen, insbesondere Oberflächen aus Glas, Holz, Zement, Porzellan, Keramik, Kunststoff oder auch Metall(en) ein, um sicherzustellen, dass diese frei von Hygieneschädlingen und/oder ihren Ausscheidungen sind. Vorzugsweise ausgeschlossen vom Schutzbereich der Erfindung sind in dieser Hinsicht chirurgische oder therapeutische, auf den menschlichen Körper oder die Körper von Tieren anzuwendende Behandlungsvorschriften und diagnostische Vorschriften, die am menschlichen Körper oder den Körpern von Tieren durchgeführt werden.

Der Begriff "Hygienesektor" deckt alle Gebiete, technischen Felder und industriellen Anwendungen ab, bei denen diese Hygienemaßnahmen, -Vorschriften und -Verfahrensweisen wichtig sind, zum Beispiel im Hinblick auf Hygiene in Küchen, Bäckereien, Flughäfen, Badezimmern, Schwimmbecken, Kaufhäusern, Hotels, Krankenhäusern, Ställen, Tierhaltungen usw.

Der Begriff "Hygieneschädling" ist daher so zu verstehen, dass damit ein oder mehrere Tierschädlinge gemeint sind, deren Gegenwart im Hygienesektor problematisch ist, insbesondere aus Gesundheitsgründen. Es ist daher ein Hauptziel, das Vorhandensein von Hygieneschädlingen und/oder das Ausgesetztsein ihnen gegenüber im Hygienesektor zu vermeiden oder auf ein Mindestmaß zu begrenzen. Dies lässt sich insbesondere durch die Anwendung eines Pestizids erreichen, das sich sowohl zum Verhindern eines Befalls als auch zum Verhindern eines bereits vorhandenen Befalls einsetzen lässt. Man kann auch Zubereitungen verwenden, die eine Exposition gegenüber Schädlingen verhindern oder reduzieren. Hygieneschädlinge schließen zum Beispiel die unten erwähnten Organismen ein.

Der Begriff "Hygieneschutz" deckt somit alle Handlungen ab, mit denen diese Hygienemaßnahmen, -Vorschriften und -Verfahrensweisen aufrechterhalten und/oder verbessert werden.

Die Verbindungen der Formel (I) können vorzugsweise als Schädlingsbekämpfungsmittel eingesetzt werden. Sie sind gegen normal sensible und resistente Arten sowie gegen alle oder einzelne Entwicklungsstadien wirksam. Zu den oben erwähnten Schädlingen gehören:
Schädlinge aus dem Stamm der Arthropoda, insbesondere aus der Klasse der Arachnida z. B. Acarus spp., z. B. Acarus siro, Aceria kuko, Aceria sheldoni, Aculops spp., Aculus spp., z. B. Aculus fockeui, Aculus schlechtendali, Amblyomma spp., Amphitetranychus viennensis, Argas spp., Boophilus spp., Brevipalpus spp., z. B. Brevipalpus phoenicis, Bryobia graminum, Bryobia praetiosa, Centruroides spp., Chorioptes spp., Dermanyssus gallinae, Dermatophagoides pteronyssinus, Dermatophagoides farinae, Dermacentor spp., Eotetranychus spp., z. B. Eotetranychus hicoriae, Epitrimerus pyri, Eutetranychus spp., z. B. Eutetranychus banksi, Eriophyes spp., z. B. Eriophyes pyri, Glycyphagus domesticus, Halotydeus destructor, Hemitarsonemus spp., z. B. Hemitarsonemus latus (=Polyphagotarsonemus latus), Hyalomma spp., Ixodes spp., Latrodectus spp., Loxosceles spp., Neutrombicula autumnalis, Nuphersa spp., Oligonychus spp., z. B. Oligonychus coffeae, Oligonychus coniferarum, Oligonychus ilicis, Oligonychus indicus, Oligonychus mangiferus, Oligonychus pratensis, Oligonychus punicae, Oligonychus yothersi, Ornithodorus spp., Ornithonyssus spp., Panonychus spp., z. B. Panonychus citri (=Metatetranychus citri), Panonychus ulmi (=Metatetranychus ulmi), Phyllocoptruta oleivora, Platytetranychus multidigituli, Polyphagotarsonemus latus, Psoroptes spp., Rhipicephalus spp., Rhizoglyphus spp., Sarcoptes spp., Scorpio maurus, Steneotarsonemus spp., Steneotarsonemus spinki, Tarsonemus spp., z. B. Tarsonemus confusus, Tarsonemus pallidus, Tetranychus spp., z. B. Tetranychus canadensis, Tetranychus cinnabarinus, Tetranychus turkestani, Tetranychus urticae, Trombicula alfreddugesi, Vaejovis spp., Vasates lycopersici;
aus der Klasse der Chilopoda z. B. Geophilus spp., Scutigera spp.;
aus der Ordnung oder der Klasse der Collembola z. B. Onychiurus armatus; Sminthurus viridis;
aus der Klasse der Diplopoda z. B. Blaniulus guttulatus;
aus der Klasse der Insecta, z. B. aus der Ordnung der Blattodea z. B. Blatta orientalis, Blattella asahinai, Blattella germanica, Leucophaea maderae, Loboptera decipiens, Neostylopyga rhombifolia, Panchlora spp., Parcoblatta spp., Periplaneta spp., z. B. Periplaneta americana, Periplaneta australasiae, Pycnoscelus surinamensis, Supella longipalpa;
aus der Ordnung der Coleoptera z. B. Acalymma vittatum, Acanthoscelides obtectus, Adoretus spp., Aethina tumida, Agelastica alni, Agrilus spp., z. B. Agrilus planipennis, Agrilus coxalis, Agrilus bilineatus, Agrilus anxius, Agriotes spp., z. B. Agriotes linneatus, Agriotes mancus, Alphitobius diaperinus, Amphimallon solstitialis, Anobium punctatum, Anoplophora spp., z. B. Anoplophora glabripennis, Anthonomus spp., z. B. Anthonomus grandis, Anthrenus spp., Apion spp., Apogonia spp., Atomaria spp., z. B. Atomaria linearis, Attagenus spp., Baris caerulescens, Bruchidius obtectus, Bruchus spp., z. B. Bruchus pisorum, Bruchus rufimanus, Cassida spp., Cerotoma trifurcata, Ceutorrhynchus spp., z. B. Ceutorrhynchus assimilis, Ceutorrhynchus quadridens, Ceutorrhynchus rapae, Chaetocnema spp., z. B. Chaetocnema confinis, Chaetocnema denticulata, Chaetocnema ectypa, Cleonus mendicus, Conoderus spp., Cosmopolites spp., z. B. Cosmopolites sordidus, Costelytra zealandica, Ctenicera spp., Curculio spp., z. B. Curculio caryae, Curculio caryatrypes, Curculio obtusus, Curculio sayi, Cryptolestes ferrugineus, Cryptolestes pusillus, Cryptorhynchus lapathi, Cryptorhynchus mangiferae, Cylindrocopturus spp., Cylindrocopturus adspersus, Cylindrocopturus furnissi, Dendroctonus spp., z. B. Dendroctonus ponderosae, Dermestes spp., Diabrotica spp., z. B. Diabrotica balteata, Diabrotica barberi, Diabrotica undecimpunctata howardi, Diabrotica undecimpunctata undecimpunctata, Diabrotica virgifera virgifera, Diabrotica virgifera zeae, Dichocrocis spp., Dicladispa armigera, Diloboderus spp., Epicaerus spp., Epilachna spp., z. B. Epilachna borealis, Epilachna varivestis, Epitrix spp., z. B. Epitrix cucumeris, Epitrix fuscula, Epitrix hirtipennis, Epitrix subcrinita, Epitrix tuberis, Faustinus spp., Gibbium psylloides, Gnathocerus cornutus, Hellula undalis, Heteronychus arator, Heteronyx spp., Hylamorpha elegans, Hylotrupes bajulus, Hypera postica, Hypomeces squamosus, Hypothenemus spp., z. B. Hypothenemus hampei, Hypothenemus obscurus, Hypothenemus pubescens, Lachnosterna consanguinea, Lasioderma serricorne, Latheticus oryzae, Lathridius spp., Lema spp., Leptinotarsa decemlineata, Leucoptera spp., z. B. Leucoptera coffeella, Limonius ectypus, Lissorhoptrus oryzophilus, Listronotus (=Hyperodes) spp., Lixus spp., Luperodes spp., Luperomorpha xanthodera, Lyctus spp., Megacyllene spp., z. B. Megacyllene robiniae, Megascelis spp., Melanotus spp., z. B. Melanotus longulus oregonensis, Meligethes aeneus, Melolontha spp., z. B. Melolontha melolontha, Migdolus spp., Monochamus spp., Naupactus xanthographus, Necrobia spp., Neogalerucella spp., Niptus hololeucus, Oryctes rhinoceros, Oryzaephilus surinamensis, Oryzaphagus oryzae, Otiorhynchus spp., z. B. Otiorhynchus cribricollis, Otiorhynchus ligustici, Otiorhynchus ovatus, Otiorhynchus rugosostriarus, Otiorhynchus sulcatus, Oulema spp., z. B. Oulema melanopus, Oulema oryzae, Oxycetonia jucunda, Phaedon cochleariae, Phyllophaga spp., Phyllophaga helleri, Phyllotreta spp., z. B. Phyllotreta armoraciae, Phyllotreta pusilla, Phyllotreta ramosa, Phyllotreta striolata, Popillia japonica, Premnotrypes spp., Prostephanus truncatus, Psylliodes spp., z. B. Psylliodes affinis, Psylliodes chrysocephala, Psylliodes punctulata, Ptinus spp., Rhizobius ventralis, Rhizopertha dominica, Rhynchophorus spp., Rhynchophorus ferrugineus, Rhynchophorus palmarum, Scolytus spp., z. B. Scolytus multistriatus, Sinoxylon perforans, Sitophilus spp., z. B. Sitophilus granarius, Sitophilus linearis, Sitophilus oryzae, Sitophilus zeamais, Sphenophorus spp., Stegobium paniceum, Sternechus spp., z. B. Sternechus paludatus, Symphyletes spp., Tanymecus spp., z. B. Tanymecus dilaticollis, Tanymecus indicus, Tanymecus palliatus, Tenebrio molitor, Tenebrioides mauretanicus, Tribolium spp., z. B. Tribolium audax, Tribolium castaneum, Tribolium confusum, Trogoderma spp., Tychius spp., Xylotrechus spp., Zabrus spp., z. B. Zabrus tenebrioides;
aus der Ordnung der Dermaptera z. B. Anisolabis maritime, Forficula auricularia, Labidura riparia;
aus der Ordnung der Diptera z. B. Aedes spp., z. B. Aedes aegypti, Aedes albopictus, Aedes sticticus, Aedes vexans, Agromyza spp., z. B. Agromyza frontella, Agromyza parvicornis, Anastrepha spp., Anopheles spp., z. B. Anopheles quadrimaculatus, Anopheles gambiae, Asphondylia spp., Bactrocera spp., z. B. Bactrocera cucurbitae, Bactrocera dorsalis, Bactrocera oleae, Bibio hortulanus, Calliphora erythrocephala, Calliphora vicina, Ceratitis capitata, Chironomus spp., Chrysomya spp., Chrysops spp., Chrysozona pluvialis, Cochliomya spp., Contarinia spp., z. B. Contarinia johnsoni, Contarinia nasturtii, Contarinia pyrivora, Contarinia schulzi, Contarinia sorghicola, Contarinia tritici, Cordylobia anthropophaga, Cricotopus sylvestris, Culex spp., z. B. Culex pipiens, Culex quinquefasciatus, Culicoides spp., Culiseta spp., Cuterebra spp., Dacus oleae, Dasineura spp., z. B. Dasineura brassicae, Delia spp., z. B. Delia antiqua, Delia coarctata, Delia florilega, Delia platura, Delia radicum, Dermatobia hominis, Drosophila spp., z. B. Drosphila melanogaster, Drosophila suzukii, Echinocnemus spp., Euleia heraclei, Fannia spp., Gasterophilus spp., Glossina spp., Haematopota spp., Hydrellia spp., Hydrellia griseola, Hylemya spp., Hippobosca spp., Hypoderma spp., Liriomyza spp., z. B. Liriomyza brassicae, Liriomyza huidobrensis, Liriomyza sativae, Lucilia spp., z. B. Lucilia cuprina, Lutzomyia spp., Mansonia spp., Musca spp., z. B. Musca domestica, Musca domestica vicina, Oestrus spp., Oscinella frit, Paratanytarsus spp., Paralauterborniella subcincta, Pegomya oder Pegomyia spp., z. B. Pegomya betae, Pegomya hyoscyami, Pegomya rubivora, Phlebotomus spp., Phorbia spp., Phormia spp., Piophila casei, Platyparea poeciloptera, Prodiplosis spp., Psila rosae, Rhagoletis spp., z. B. Rhagoletis cingulata, Rhagoletis completa, Rhagoletis fausta, Rhagoletis indifferens, Rhagoletis mendax, Rhagoletis pomonella, Sarcophaga spp., Simulium spp., z. B. Simulium meridionale, Stomoxys spp., Tabanus spp., Tetanops spp., Tipula spp., z. B. Tipula paludosa, Tipula simplex, Toxotrypana curvicauda;
aus der Ordnung der Hemiptera z. B. Acizzia acaciaebaileyanae, Acizzia dodonaeae, Acizzia uncatoides, Acrida turrita, Acyrthosipon spp., z. B. Acyrthosiphon pisum, Acrogonia spp., Aeneolamia spp., Agonoscena spp., Aleurocanthus spp., Aleyrodes proletella, Aleurolobus barodensis, Aleurothrixus floccosus, Allocaridara malayensis, Amrasca spp., z. B. Amrasca bigutulla, Amrasca devastans, Anuraphis cardui, Aonidiella spp., z. B. Aonidiella aurantii, Aonidiella citrina, Aonidiella inornata, Aphanostigma piri, Aphis spp., z. B. Aphis citricola, Aphis craccivora, Aphis fabae, Aphis forbesi, Aphis glycines, Aphis gossypii, Aphis hederae, Aphis illinoisensis, Aphis middletoni, Aphis nasturtii, Aphis nerii, Aphis pomi, Aphis spiraecola, Aphis viburniphila, Arboridia apicalis, Arytainilla spp., Aspidiella spp., Aspidiotus spp., z. B. Aspidiotus nerii, Atanus spp., Aulacorthum solani, Bemisia tabaci, Blastopsylla occidentalis, Boreioglycaspis melaleucae, Brachycaudus helichrysi, Brachycolus spp., Brevicoryne brassicae, Cacopsylla spp., z. B. Cacopsylla pyricola, Calligypona marginata, Capulinia spp., Carneocephala fulgida, Ceratovacuna lanigera, Cercopidae, Ceroplastes spp., Chaetosiphon fragaefolii, Chionaspis tegalensis, Chlorita onukii, Chondracris rosea, Chromaphis juglandicola, Chrysomphalus aonidum, Chrysomphalus ficus, Cicadulina mbila, Coccomytilus halli, Coccus spp., z. B. Coccus hesperidum, Coccus longulus, Coccus pseudomagnoliarum, Coccus viridis, Cryptomyzus ribis, Cryptoneossa spp., Ctenarytaina spp., Dalbulus spp., Dialeurodes chittendeni, Dialeurodes citri, Diaphorina citri, Diaspis spp., Diuraphis spp., Doralis spp., Drosicha spp., Dysaphis spp., z. B. Dysaphis apiifolia, Dysaphis plantaginea, Dysaphis tulipae, Dysmicoccus spp., Empoasca spp., z. B. Empoasca abrupta, Empoasca fabae, Empoasca maligna, Empoasca solana, Empoasca stevensi, Eriosoma spp., z. B. Eriosoma americanum, Eriosoma lanigerum, Eriosoma pyricola, Erythroneura spp., Eucalyptolyma spp., Euphyllura spp., Euscelis bilobatus, Ferrisia spp., Fiorinia spp., Furcaspis oceanica, Geococcus coffeae, Glycaspis spp., Heteropsylla cubana, Heteropsylla spinulosa, Homalodisca coagulata, Hyalopterus arundinis, Hyalopterus pruni, Icerya spp., z. B. Icerya purchasi, Idiocerus spp., Idioscopus spp., Laodelphax striatellus, Lecanium spp., z. B. Lecanium corni (=Parthenolecanium corni), Lepidosaphes spp., z. B. Lepidosaphes ulmi, Lipaphis erysimi, Lopholeucaspis japonica, Lycorma delicatula, Macrosiphum spp., z. B. Macrosiphum euphorbiae, Macrosiphum lilii, Macrosiphum rosae, Macrosteles facifrons, Mahanarva spp., Melanaphis sacchari, Metcalfiella spp., Metcalfa pruinosa, Metopolophium dirhodum, Monellia costalis, Monelliopsis pecanis, Myzus spp., z. B. Myzus ascalonicus, Myzus cerasi, Myzus ligustri, Myzus ornatus, Myzus persicae, Myzus nicotianae, Nasonovia ribisnigri, Neomaskellia spp., Nephotettix spp., z. B. Nephotettix cincticeps, Nephotettix nigropictus, Nettigoniclla spectra, Nilaparvata lugens, Oncometopia spp., Orthezia praelonga, Oxya chinensis, Pachypsylla spp., Parabemisia myricae, Paratrioza spp., z. B. Paratrioza cockerelli, Parlatoria spp., Pemphigus spp., z. B. Pemphigus bursarius, Pemphigus populivenae, Peregrinus maidis, Perkinsiella spp., Phenacoccus spp., z. B. Phenacoccus madeirensis, Phloeomyzus passerinii, Phorodon humuli, Phylloxera spp., z. B. Phylloxera devastatrix, Phylloxera notabilis, Pinnaspis aspidistrae, Planococcus spp., z. B. Planococcus citri, Prosopidopsylla flava, Protopulvinaria pyriformis, Pseudaulacaspis pentagona, Pseudococcus spp., z. B. Pseudococcus calceolariae, Pseudococcus comstocki, Pseudococcus longispinus, Pseudococcus maritimus, Pseudococcus viburni, Psyllopsis spp., Psylla spp., z. B. Psylla buxi, Psylla mali, Psylla pyri, Pteromalus spp., Pulvinaria spp., Pyrilla spp., Quadraspidiotus spp., z. B. Quadraspidiotus juglansregiae, Quadraspidiotus ostreaeformis, Quadraspidiotus perniciosus, Quesada gigas, Rastrococcus spp., Rhopalosiphum spp., z. B. Rhopalosiphum maidis, Rhopalosiphum oxyacanthae, Rhopalosiphum padi, Rhopalosiphum rufiabdominale, Saissetia spp., z. B. Saissetia coffeae, Saissetia miranda, Saissetia neglecta, Saissetia oleae, Scaphoideus titanus, Schizaphis graminum, Selenaspidus articulatus, Sipha flava, Sitobion avenae, Sogata spp., Sogatella furcifera, Sogatodes spp., Stictocephala festina, Siphoninus phillyreae, Tenalaphara malayensis, Tetragonocephela spp., Tinocallis caryaefoliae, Tomaspis spp., Toxoptera spp., z. B. Toxoptera aurantii, Toxoptera citricidus, Trialeurodes vaporariorum, Trioza spp., z. B. Trioza diospyri, Typhlocyba spp., Unaspis spp., Viteus vitifolii, Zygina spp.;
aus der Unterordnung der Heteroptera z. B. Aelia spp., Anasa tristis, Antestiopsis spp., Boisea spp., Blissus spp., Calocoris spp., Campylomma livida, Cavelerius spp., Cimex spp., z. B. Cimex adjunctus, Cimex hemipterus, Cimex lectularius, Cimex pilosellus, Collaria spp., Creontiades dilutus, Dasynus piperis, Dichelops furcatus, Diconocoris hewetti, Dysdercus spp., Euschistus spp., z. B. Euschistus heros, Euschistus servus, Euschistus tristigmus, Euschistus variolarius, Eurydema spp., Eurygaster spp., Halyomorpha halys, Heliopeltis spp., Horcias nobilellus, Leptocorisa spp., Leptocorisa varicornis, Leptoglossus occidentalis, Leptoglossus phyllopus, Lygocoris spp., z. B. Lygocoris pabulinus, Lygus spp., z. B. Lygus elisus, Lygus hesperus, Lygus lineolaris, Macropes excavatus, Megacopta cribraria, Miridae, Monalonion atratum, Nezara spp., z. B. Nezara viridula, Nysius spp., Oebalus spp., Pentomidae, Piesma quadrata, Piezodorus spp., z. B. Piezodorus guildinii, Psallus spp., Pseudacysta persea, Rhodnius spp., Sahlbergella singularis, Scaptocoris castanea, Scotinophora spp., Stephanitis nashi, Tibraca spp., Triatoma spp.;
aus der Ordnung der Hymenoptera z. B. Acromyrmex spp., Athalia spp., z. B. Athalia rosae, Atta spp., Camponotus spp., Dolichovespula spp., Diprion spp., z. B. Diprion similis, Hoplocampa spp., z. B. Hoplocampa cookei, Hoplocampa testudinea, Lasius spp., Linepithema (Iridiomyrmex) humile, Monomorium pharaonis, Paratrechina spp., Paravespula spp., Plagiolepis spp., Sirex spp., z. B. Sirex noctilio, Solenopsis invicta, Tapinoma spp., Technomyrmex albipes, Urocerus spp., Vespa spp., z. B. Vespa crabro, Wasmannia auropunctata, Xeris spp.;
aus der Ordnung der Isopoda z. B. Armadillidium vulgare, Oniscus asellus, Porcellio scaber;
aus der Ordnung der Isoptera z. B. Coptotermes spp., z. B. Coptotermes formosanus, Cornitermes cumulans, Cryptotermes spp., Incisitermes spp., Kalotermes spp., Microtermes obesi, Nasutitermis spp., Odontotermes spp., Porotermes spp., Reticulitermes spp., z. B. Reticulitermes flavipes, Reticulitermes hesperus;
aus der Ordnung der Lepidoptera z. B. Achroia grisella, Acronicta major, Adoxophyes spp., z. B. Adoxophyes orana, Aedia leucomelas, Agrotis spp., z. B. Agrotis segetum, Agrotis ipsilon, Alabama spp., z. B. Alabama argillacea, Amyelois transitella, Anarsia spp., Anticarsia spp., z. B. Anticarsia gemmatalis, Argyroploce spp., Autographa spp., Barathra brassicae, Blastodacna atra, Borbo cinnara, Bucculatrix thurberiella, Bupalus piniarius, Busseola spp., Cacoecia spp., Caloptilia theivora, Capua reticulana, Carpocapsa pomonella, Carposina niponensis, Cheimatobia brumata, Chilo spp., z. B. Chilo plejadellus, Chilo suppressalis, Choreutis pariana, Choristoneura spp., Chrysodeixis chalcites, Clysia ambiguella, Cnaphalocerus spp., Cnaphalocrocis medinalis, Cnephasia spp., Conopomorpha spp., Conotrachelus spp., Copitarsia spp., Cydia spp., z. B. Cydia nigricana, Cydia pomonella, Dalaca noctuides, Diaphania spp., Diparopsis spp., Diatraea saccharalis, Dioryctria spp., z. B. Dioryctria zimmermani, Earias spp., Ecdytolopha aurantium, Elasmopalpus lignosellus, Eldana saccharina, Ephestia spp., z. B. Ephestia elutella, Ephestia kuehniella, Epinotia spp., Epiphyas postvittana, Erannis spp., Erschoviella musculana, Etiella spp., Eudocima spp., Eulia spp., Eupoecilia ambiguella, Euproctis spp., z. B. Euproctis chrysorrhoea, Euxoa spp., Feltia spp., Galleria mellonella, Gracillaria spp., Grapholitha spp., z. B. Grapholita molesta, Grapholita prunivora, Hedylepta spp., Helicoverpa spp., z. B. Helicoverpa armigera, Helicoverpa zea, Heliothis spp., z. B. Heliothis virescens , Hofmannophila pseudospretella, Homoeosoma spp., Homona spp., Hyponomeuta padella, Kakivoria flavofasciata, Lampides spp., Laphygma spp., Laspeyresia molesta, Leucinodes orbonalis, Leucoptera spp., z. B. Leucoptera coffeella, Lithocolletis spp., z. B. Lithocolletis blancardella, Lithophane antennata, Lobesia spp., z. B. Lobesia botrana, Loxagrotis albicosta, Lymantria spp., z. B. Lymantria dispar, Lyonetia spp., z. B. Lyonetia clerkella, Malacosoma neustria, Maruca testulalis, Mamestra brassicae, Melanitis leda, Mocis spp., Monopis obviella, Mythimna separata, Nemapogon cloacellus, Nymphula spp., Oiketicus spp., Omphisa spp., Operophtera spp., Oria spp., Orthaga spp., Ostrinia spp., z. B. Ostrinia nubilalis, Panolis flammea, Parnara spp., Pectinophora spp., z. B. Pectinophora gossypiella, Perileucoptera spp., Phthorimaea spp., z. B. Phthorimaea operculella, Phyllocnistis citrella, Phyllonorycter spp., z. B. Phyllonorycter blancardella, Phyllonorycter crataegella, Pieris spp., z. B. Pieris rapae, Platynota stultana, Plodia interpunctella, Plusia spp., Plutella xylostella (=Plutella maculipennis), Podesia spp., z. B. Podesia syringae, Prays spp., Prodenia spp., Protoparce spp., Pseudaletia spp., z. B. Pseudaletia unipuncta, Pseudoplusia includens, Pyrausta nubilalis, Rachiplusia nu, Schoenobius spp., z. B. Schoenobius bipunctifer, Scirpophaga spp., z. B. Scirpophaga innotata, Scotia segetum, Sesamia spp., z. B. Sesamia inferens, Sparganothis spp., Spodoptera spp., z. B. Spodoptera eradiana, Spodoptera exigua, Spodoptera frugiperda, Spodoptera praefica, Stathmopoda spp., Stenoma spp., Stomopteryx subsecivella, Synanthedon spp., Tecia solanivora, Thaumetopoea spp., Thermesia gemmatalis, Tinea cloacella, Tinea pellionella, Tineola bisselliella, Tortrix spp., Trichophaga tapetzella, Trichoplusia spp., z. B. Trichoplusia ni, Tryporyza incertulas, Tuta absoluta, Virachola spp.;
aus der Ordnung der Orthoptera oder Saltatoria z. B. Acheta domesticus, Dichroplus spp., Gryllotalpa spp., z. B. Gryllotalpa gryllotalpa, Hieroglyphus spp., Locusta spp., z. B. Locusta migratoria, Melanoplus spp., z. B. Melanoplus devastator, Paratlanticus ussuriensis, Schistocerca gregaria;
aus der Ordnung der Phthiraptera z. B. Damalinia spp., Haematopinus spp., Linognathus spp., Pediculus spp., Phylloxera vastatrix, Phthirus pubis, Trichodectes spp.;
aus der Ordnung der Psocoptera z. B. Lepinotus spp., Liposcelis spp.;
aus der Ordnung der Siphonaptera z. B. Ceratophyllus spp., Ctenocephalides spp., z. B. Ctenocephalides canis, Ctenocephalides felis, Pulex irritans, Tunga penetrans, Xenopsylla cheopis;
aus der Ordnung der Thysanoptera z. B. Anaphothrips obscurus, Baliothrips biformis, Chaetanaphothrips leeuweni, Drepanothrips reuteri, Enneothrips flavens, Frankliniella spp., z. B. Frankliniella fusca, Frankliniella occidentalis, Frankliniella schultzei, Frankliniella tritici, Frankliniella vaccinii, Frankliniella williamsi, Haplothrips spp., Heliothrips spp., Hercinothrips femoralis, Kakothrips spp., Rhipiphorothrips cruentatus, Scirtothrips spp., Taeniothrips cardamomi, Thrips spp., z. B. Thrips palmi, Thrips tabaci;
aus der Ordnung der Zygentoma (= Thysanura), z. B. Ctenolepisma spp., Lepisma saccharina, Lepismodes inquilinus, Thermobia domestica;
aus der Klasse der Symphyla z. B. Scutigerella spp., z. B. Scutigerella immaculata;
Schädlinge aus dem Stamm der Mollusca, z. B. aus der Klasse der Bivalvia, z. B. Dreissena spp.;
sowie aus der Klasse der Gastropoda z. B. Arion spp., z. B. Arion ater rufus, Biomphalaria spp., Bulinus spp., Deroceras spp., z. B. Deroceras laeve, Galba spp., Lymnaea spp., Oncomelania spp., Pomacea spp., Succinea spp.;
Pflanzenschädlinge aus dem Stamm der Nematoda, d. h. pflanzenparasitäre Nematoden, insbesondere Aglenchus spp., z. B. Aglenchus agricola, Anguina spp., z. B. Anguina tritici, Aphelenchoides spp., z. B. Aphelenchoides arachidis, Aphelenchoides fragariae, Belonolaimus spp., z. B. Belonolaimus gracilis, Belonolaimus longicaudatus, Belonolaimus nortoni, Bursaphelenchus spp., z. B. Bursaphelenchus cocophilus, Bursaphelenchus eremus, Bursaphelenchus xylophilus, Cacopaurus spp., z. B. Cacopaurus pestis, Criconemella spp., z. B. Criconemella curvata, Criconemella onoensis, Criconemella ornata, Criconemella rusium, Criconemella xenoplax (= Mesocriconema xenoplax), Criconemoides spp., z. B. Criconemoides ferniae, Criconemoides onoense, Criconemoides ornatum, Ditylenchus spp., z. B. Ditylenchus dipsaci, Dolichodorus spp., Globodera spp., z. B. Globodera pallida, Globodera rostochiensis, Helicotylenchus spp., z. B. Helicotylenchus dihystera, Hemicriconemoides spp., Hemicycliophora spp., Heterodera spp., z. B. Heterodera avenae, Heterodera glycines, Heterodera schachtii, Hirschmaniella spp., Hoplolaimus spp., Longidorus spp., z. B. Longidorus africanus, Meloidogyne spp., z. B. Meloidogyne chitwoodi, Meloidogyne fallax, Meloidogyne hapla, Meloidogyne incognita, Meloinema spp., Nacobbus spp., Neotylenchus spp., Paralongidorus spp., Paraphelenchus spp., Paratrichodorus spp., z. B. Paratrichodorus minor, Paratylenchus spp., Pratylenchus spp., z. B. Pratylenchus penetrans, Pseudohalenchus spp., Psilenchus spp., Punctodera spp., Quinisulcius spp., Radopholus spp., z. B. Radopholus citrophilus, Radopholus similis, Rotylenchulus spp., Rotylenchus spp., Scutellonema spp., Subanguina spp., Trichodorus spp., z. B. Trichodorus obtusus, Trichodorus primitivus, Tylenchorhynchus spp., z. B. Tylenchorhynchus annulatus, Tylenchulus spp., z. B. Tylenchulus semipenetrans, Xiphinema spp., z. B. Xiphinema index.

Die Verbindungen der Formel (I) können gegebenenfalls in bestimmten Konzentrationen bzw. Aufwandmengen auch als Herbizide, Safener, Wachstumsregulatoren oder Mittel zur Verbesserung der Pflanzeneigenschaften, als Mikrobizide oder Gametozide, beispielsweise als Fungizide, Antimykotika, Bakterizide, Virizide (einschließlich Mittel gegen Viroide) oder als Mittel gegen MLO (Mycoplasmalike-organism) und RLO (Rickettsia-like-organism) verwendet werden. Sie lassen sich gegebenenfalls auch als Zwischen- oder Vorprodukte für die Synthese weiterer Wirkstoffe einsetzen.

### Formulierungen

Die vorliegende Erfindung betrifft weiterhin Formulierungen und daraus bereitete Anwendungsformen als Schädlingsbekämpfungsmittel wie z. B. Drench-, Drip- und Spritzbrühen, umfassend mindestens eine Verbindung der Formel (I). Gegebenenfalls enthalten die Anwendungsformen weitere Schädlingsbekämpfungsmittel und/oder die Wirkung verbessernde Adjuvantien wie Penetrationsförderer, z. B. pflanzliche Öle wie beispielsweise Rapsöl, Sonnenblumenöl, Mineralöle wie beispielsweise Paraffinöle, Alkylester pflanzlicher Fettsäuren wie beispielsweise Rapsöl- oder Sojaölmethylester oder Alkanol-alkoxylate und/oder Spreitmittel wie beispielsweise Alkylsiloxane und/oder Salze, z. B. organische oder anorganische Ammonium- oder Phosphoniumsalze wie beispielsweise Ammoniumsulfat oder Diammonium-hydrogenphosphat und/oder die Retention fördernde Mittel wie z. B. Dioctylsulfosuccinat oder Hydroxypropyl-guar-Polymere und/oder Humectants wie z. B. Glycerin und/oder Dünger wie beispielsweise Ammonium, Kalium oder Phosphor enthaltende Dünger.

Übliche Formulierungen sind beispielsweise wasserlösliche Flüssigkeiten (SL), Emulsionskonzentrate (EC), Emulsionen in Wasser (EW), Suspensionskonzentrate (SC, SE, FS, OD), in Wasser dispergierbare Granulate (WG), Granulate (GR) und Kapselkonzentrate (CS); diese und weitere mögliche Formuliertypen sind beispielsweise durch Crop Life International und in Pesticide Specifications, Manual on development and use of FAO and WHO specifications for pesticides, FAO Plant Production and Protection Papers - 173, prepared by the FAO/WHO Joint Meeting on Pesticide Specifications, 2004, ISBN: 9251048576 beschrieben. Gegebenenfalls enthalten die Formulierungen neben einer oder mehreren Verbindungen der Formel (I) weitere agrochemische Wirkstoffe.

Vorzugsweise handelt es sich um Formulierungen oder Anwendungsformen, welche Hilfsstoffe wie beispielsweise Streckmittel, Lösemittel, Spontanitätsförderer, Trägerstoffe, Emulgiermittel, Dispergiermittel, Frostschutzmittel, Biozide, Verdicker und/oder weitere Hilfsstoffe wie beispielsweise Adjuvantien enthalten. Ein Adjuvant in diesem Kontext ist eine Komponente, die die biologische Wirkung der Formulierung verbessert, ohne dass die Komponente selbst eine biologische Wirkung hat. Beispiele für Adjuvantien sind Mittel, die die Retention, das Spreitverhalten, das Anhaften an der Blattoberfläche oder die Penetration fördern.

Diese Formulierungen werden in bekannter Weise hergestellt, z. B. durch Vermischen der Verbindungen der Formel (I) mit Hilfsstoffen wie beispielsweise Streckmitteln, Lösemitteln und/oder festen Trägerstoffen und/oder weiteren Hilfsstoffen wie beispielsweise oberflächenaktiven Stoffen. Die Herstellung der Formulierungen erfolgt entweder in geeigneten Anlagen oder auch vor oder während der Anwendung.

Als Hilfsstoffe können solche Stoffe Verwendung finden, die geeignet sind, der Formulierung der Verbindungen der Formel (I) oder den aus diesen Formulierungen bereiteten Anwendungsformen (wie z. B. gebrauchsfähigen Schädlingsbekämpfungsmitteln wie Spritzbrühen oder Saatgutbeizen) besondere Eigenschaften, wie bestimmte physikalische, technische und/oder biologische Eigenschaften zu verleihen.

Als Streckmittel eignen sich z. B. Wasser, polare und unpolare organische chemische Flüssigkeiten z. B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (Poly-)Ether, der einfachen und substituierten Amine, Amide, Lactame (wie N-Alkylpyrrolidone) und Lactone, der Sulfone und Sulfoxide (wie Dimethylsulfoxid).

Im Falle der Benutzung von Wasser als Streckmittel können z. B. auch organische Lösemittel als Hilfslösemittel verwendet werden. Als flüssige Lösemittel kommen im Wesentlichen infrage: Aromaten wie Xylol, Toluol oder Alkylnaphthaline, chlorierte Aromaten oder chlorierte aliphatische Kohlenwasserstoffe wie Chlorbenzole, Chlorethylene oder Methylenchlorid, aliphatische Kohlenwasserstoffe, wie Cyclohexan oder Paraffine, z. B. Erdölfraktionen, mineralische und pflanzliche Öle, Alkohole wie Butanol oder Glykol sowie deren Ether und Ester, Ketone wie Aceton, Methylethylketon, Methylisobutylketon oder Cyclohexanon, stark polare Lösemittel wie Dimethylformamid und Dimethylsulfoxid sowie Wasser.

Grundsätzlich können alle geeigneten Lösemittel verwendet werden. Geeignete Lösemittel sind beispielsweise aromatische Kohlenwasserstoffe wie z. B. Xylol, Toluol oder Alkylnaphthaline, chlorierte aromatische oder chlorierte aliphatische Kohlenwasserstoffe wie z. B. Chlorbenzol, Chlorethylen, oder Methylenchlorid, aliphatische Kohlenwasserstoffe wie z. B. Cyclohexan, Paraffine, Erdölfraktionen, mineralische und pflanzliche Öle, Alkohole wie z. B. Methanol, Ethanol, iso-Propanol, Butanol oder Glykol sowie deren Ether und Ester, Ketone wie z. B. Aceton, Methylethylketon, Methylisobutylketon oder Cyclohexanon, stark polare Lösemittel wie Dimethylsulfoxid sowie Wasser.

Grundsätzlich können alle geeigneten Trägerstoffe eingesetzt werden. Als Trägerstoffe kommen insbesondere infrage: z. B. Ammoniumsalze und natürliche Gesteinsmehle wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und natürliche oder synthetische Silikate, Harze, Wachse und/oder feste Düngemittel. Mischungen solcher Trägerstoffe können ebenfalls verwendet werden. Als Trägerstoffe für Granulate kommen infrage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Papier, Kokosnussschalen, Maiskolben und Tabakstängel.

Auch verflüssigte gasförmige Streckmittel oder Lösemittel können eingesetzt werden. Insbesondere eignen sich solche Streckmittel oder Trägerstoffe, welche bei normaler Temperatur und unter Normaldruck gasförmig sind, z. B. Aerosol-Treibgase wie Halogenkohlenwasserstoffe sowie Butan, Propan, Stickstoff und Kohlendioxid.

Beispiele für Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe sind Salze von Polyacrylsäure, Salze von Lignosulfonsäure, Salze von Phenolsulfonsäure oder Naphthalinsulfonsäure, Polykondensate von Ethylenoxid mit Fettalkoholen oder mit Fettsäuren oder mit Fettaminen, mit substituierten Phenolen (vorzugsweise Alkylphenole oder Arylphenole), Salze von Sulfobernsteinsäureestern, Taurinderivate (vorzugsweise Alkyltaurate), Phosphorsäureester von polyethoxylierten Alkoholen oder Phenolen, Fettsäureester von Polyolen und Derivate der Verbindungen enthaltend Sulfate, Sulfonate und Phosphate, z. B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist vorteilhaft, wenn eine der Verbindungen der Formel (I) und/oder einer der inerten Trägerstoffe nicht in Wasser löslich ist und wenn die Anwendung in Wasser erfolgt.

Als weitere Hilfsstoffe können in den Formulierungen und den daraus abgeleiteten Anwendungsformen Farbstoffe wie anorganische Pigmente, z. B. Eisenoxid, Titanoxid, Ferrocyanblau und organische Farbstoffe wie Alizarin-, Azo- und Metallphthalocyaninfarbstoffe und Nähr- und Spurennährstoffe wie Salze von Eisen, Mangan, Bor, Kupfer, Kobalt, Molybdän und Zink vorhanden sein.

Weiterhin enthalten sein können Stabilisatoren wie Kältestabilisatoren, Konservierungsmittel, Oxidationsschutzmittel, Lichtschutzmittel oder andere die chemische und/oder physikalische Stabilität verbessernde Mittel. Weiterhin enthalten sein können schaumerzeugende Mittel oder Entschäumer.

Ferner können die Formulierungen und daraus abgeleiteten Anwendungsformen als zusätzliche Hilfsstoffe auch Haftmittel wie Carboxymethylcellulose, natürliche und synthetische pulverige, körnige oder latexförmige Polymere enthalten wie Gummiarabikum, Polyvinylalkohol, Polyvinylacetat sowie natürliche Phospholipide wie Kephaline und Lecithine und synthetische Phospholipide. Weitere Hilfsstoffe können mineralische und pflanzliche Öle sein.

Gegebenenfalls können noch weitere Hilfsstoffe in den Formulierungen und den daraus abgeleiteten Anwendungsformen enthalten sein. Solche Zusatzstoffe sind beispielsweise Duftstoffe, schützende Kolloide, Bindemittel, Klebstoffe, Verdicker, thixotrope Stoffe, Penetrationsförderer, Retentionsförderer, Stabilisatoren, Sequestiermittel, Komplexbildner, Feuchthaltemittel, Spreitmittel. Im Allgemeinen können die Verbindungen der Formel (I) mit jedem festen oder flüssigen Zusatzstoff, welcher für Formulierungszwecke gewöhnlich verwendet wird, kombiniert werden.

Als Retentionsförderer kommen alle diejenigen Substanzen in Betracht, die die dynamische Oberflächenspannung verringern wie beispielsweise Dioctylsulfosuccinat oder die die Visko-Elastizität erhöhen wie beispielsweise Hydroxypropyl-guar-Polymere.

Als Penetrationsförderer kommen im vorliegenden Zusammenhang alle diejenigen Substanzen in Betracht, die üblicherweise eingesetzt werden, um das Eindringen von agrochemischen Wirkstoffen in Pflanzen zu verbessern. Penetrationsförderer werden in diesem Zusammenhang dadurch definiert, dass sie aus der (in der Regel wässerigen) Applikationsbrühe und/oder aus dem Spritzbelag in die Kutikula der Pflanze eindringen und dadurch die Beweglichkeit der Wirkstoffe in der Kutikula erhöhen können. Die in der Literatur (Baur et al., 1997, Pesticide Science 51, 131-152) beschriebene Methode kann zur Bestimmung dieser Eigenschaft eingesetzt werden. Beispielhaft werden genannt Alkoholalkoxylate wie beispielsweise Kokosfettethoxylat (10) oder Isotridecylethoxylat (12), Fettsäureester wie beispielsweise Rapsöl- oder Sojaölmethylester, Fettaminalkoxylate wie beispielsweise Tallowamine-ethoxylat (15) oder Ammonium- und/oder Phosphonium-Salze wie beispielsweise Ammoniumsulfat oder Diammonium-hydrogenphosphat.

Die Formulierungen enthalten bevorzugt zwischen 0,00000001 und 98 Gew.-% der Verbindung der Formel (I), besonders bevorzugt zwischen 0,01 und 95 Gew.-% der Verbindung der Formel (I), ganz besonders bevorzugt zwischen 0,5 und 90 Gew.-% der Verbindung der Formel (I), bezogen auf das Gewicht der Formulierung.

Der Gehalt an der Verbindung der Formel (I) in den aus den Formulierungen bereiteten Anwendungsformen (insbesondere Schädlingsbekämpfungsmittel) kann in weiten Bereichen variieren. Die Konzentration der Verbindung der Formel (I) in den Anwendungsformen kann üblicherweise zwischen 0,00000001 und 95 Gew.-% der Verbindung der Formel (I), vorzugsweise zwischen 0,00001 und 1 Gew.-%, bezogen auf das Gewicht der Anwendungsform, liegen. Die Anwendung geschieht in einer den Anwendungsformen angepaßten üblichen Weise.

### Mischungen

Die Verbindungen der Formel (I) können auch in Mischung mit einem oder mehreren geeigneten Fungiziden, Bakteriziden, Akariziden, Molluskiziden, Nematiziden, Insektiziden, Mikrobiologika, Nützlingen, Herbiziden, Düngemitteln, Vogelrepellentien, Phytotonics, Sterilantien, Safenern, Semiochemicals und/oder Pflanzenwachstumsregulatoren verwendet werden, um so z. B. das Wirkungsspektrum zu verbreitern, die Wirkdauer zu verlängern, die Wirkgeschwindigkeit zu steigern, Repellenz zu verhindern oder Resistenzentwicklungen vorzubeugen. Des Weiteren können solche Wirkstoffkombinationen das Pflanzenwachstum und/oder die Toleranz gegenüber abiotischen Faktoren wie z. B. hohen oder niedrigen Temperaturen, gegen Trockenheit oder gegen erhöhten Wasser- bzw. Bodensalzgehalt verbessern. Auch lässt sich das Blüh- und Fruchtverhalten verbessern, die Keimfähigkeit und Bewurzelung optimieren, die Ernte erleichtern und Ernteertrag steigern, die Reife beeinflussen, die Qualität und/oder der Ernährungswert der Ernteprodukte steigern, die Lagerfähigkeit verlängern und/oder die Bearbeitbarkeit der Ernteprodukte verbessern.

Weiterhin können die Verbindungen der Formel (I) in Mischung mit weiteren Wirkstoffen oder Semiochemicals, wie Lockstoffen und/oder Vogelrepellentien und/oder Pflanzenaktivatoren und/oder Wachstumsregulatoren und/oder Düngemitteln vorliegen. Gleichfalls können die Verbindungen der Formel (I) zur Verbesserung der Pflanzeneigenschaften wie zum Beispiel Wuchs, Ertrag und Qualität des Erntegutes eingesetzt werden.

In einer besonderen erfindungsgemäßen Ausführungsform liegen die Verbindungen der Formel (I) in Formulierungen bzw. in den aus diesen Formulierungen bereiteten Anwendungsformen in Mischung mit weiteren Verbindungen vor, vorzugsweise solchen wie nachstehend beschrieben.

Wenn eine der im Folgenden genannten Verbindungen in verschiedenen tautomeren Formen vorkommen kann, sind auch diese Formen mit umfasst, auch wenn sie sie nicht in jedem Fall explizit genannt wurden. Alle genannten Mischungspartner können außerdem, wenn sie auf Grund ihrer funktionellen Gruppen dazu imstande sind, gegebenenfalls mit geeigneten Basen oder Säuren Salze bilden.

### Insektizide/Akarizide/Nematizide

Die hier mit ihrem "Common Name" genannten Wirkstoffe sind bekannt und beispielsweise im Pestizidhandbuch ("The Pesticide Manual" 16th Ed., British Crop Protection Council 2012) beschrieben oder im Internet recherchierbar (z. B. http://www.alanwood.net/pesticides). Die Klassifizierung basiert auf dem zum Zeitpunkt der Einreichung dieser Patentanmeldung gültigen IRAC Mode of Action Classification Scheme.
(1) Acetylcholinesterase(AChE)-Inhibitoren, wie beispielsweise Carbamate, z. B. Alanycarb, Aldicarb, Bendiocarb, Benfuracarb, Butocarboxim, Butoxycarboxim, Carbaryl, Carbofuran, Carbosulfan, Ethiofencarb, Fenobucarb, Formetanate, Furathiocarb, Isoprocarb, Methiocarb, Methomyl, Metolcarb, Oxamyl, Pirimicarb, Propoxur, Thiodicarb, Thiofanox, Triazamate, Trimethacarb, XMC und Xylylcarb oder Organophosphate, z. B. Acephat, Azamethiphos, Azinphos-ethyl, Azinphos-methyl, Cadusafos, Chlorethoxyfos, Chlorfenvinphos, Chlormephos, Chlorpyrifos-methyl, Coumaphos, Cyanophos, Demeton-S-methyl, Diazinon, Dichlorvos/DDVP, Dicrotophos, Dimethoat, Dimethylvinphos, Disulfoton, EPN, Ethion, Ethoprophos, Famphur, Fenamiphos, Fenitrothion, Fenthion, Fosthiazat, Heptenophos, Imicyafos, Isofenphos, Isopropyl-O-(methoxyaminothio-phosphoryl)salicylat, Isoxathion, Malathion, Mecarbam, Methamidophos, Methidathion, Mevinphos, Monocrotophos, Naled, Omethoate, Oxydemeton-methyl, Parathion-methyl, Phenthoat, Phorat, Phosalon, Phosmet, Phosphamidon, Phoxim, Pirimiphos-methyl, Profenofos, Propetamphos, Prothiofos, Pyraclofos, Pyridaphenthion, Quinalphos, Sulfotep, Tebupirimfos, Temephos, Terbufos, Tetrachlorvinphos, Thiometon, Triazophos, Triclorfon und Vamidothion.
(2) GABA-gesteuerte Chlorid-Kanal-Blocker, wie beispielsweise Cyclodien-organochlorine, z. B. Chlordan und Endosulfan oder Phenylpyrazole (Fiprole), z. B. Ethiprol und Fipronil.
(3) Natrium-Kanal-Modulatoren, wie beispielsweise Pyrethroide, z. B. Acrinathrin, Allethrin, d-cistrans-Allethrin, d-trans-Allethrin, Bifenthrin, Bioallethrin, Bioallethrin-S-cyclopentenyl-Isomer, Bioresmethrin, Cycloprothrin, Cyfluthrin, beta-Cyfluthrin, Cyhalothrin, lambda-Cyhalothrin, gamma-Cyhalothrin, Cypermethrin, alpha-Cypermethrin, beta-Cypermethrin, theta-Cypermethrin, zeta-Cypermethrin, Cyphenothrin [(1R)-trans-Isomer], Deltamethrin, Empenthrin [(EZ)-(1R)-Isomer], Esfenvalerat, Etofenprox, Fenpropathrin, Fenvalerat, Flucythrinat, Flumethrin, tau-Fluvalinat, Halfenprox, Imiprothrin, Kadethrin, Momfluorothrin, Permethrin, Phenothrin [(1R)-trans-Isomer], Prallethrin, Pyrethrine (pyrethrum), Resmethrin, Silafluofen, Tefluthrin, Tetramethrin, Tetramethrin [(1R)-Isomer], Tralomethrin und Transfluthrin oder DDT oder Methoxychlor.
(4) Kompetitive Modulatoren des nicotinischen Acetylcholin-Rezeptors (nAChR), wie beispielsweise Neonicotinoide, z. B. Acetamiprid, Clothianidin, Dinotefuran, Imidacloprid, Nitenpyram, Thiacloprid und Thiamethoxam oder Nicotin oder Sulfoxaflor oder Flupyradifurone.
(5) Allosterische Modulatoren des nicotinischen Acetylcholin-Rezeptors (nAChR), wie beispielsweise Spinosyne, z. B. Spinetoram und Spinosad.
(6) Allosterische Modulatoren des Glutamat-abhängigen Chloridkanals(GluCl), wie beispielsweise Avermectine/Milbemycine, z. B. Abamectin, Emamectin-benzoat, Lepimectin und Milbemectin.
(7) Juvenilhormon-Mimetika, wie beispielsweise Juvenilhormon-Analoge, z. B. Hydropren, Kinopren und Methopren oder Fenoxycarb oder Pyriproxyfen.
(8) Verschiedene nicht spezifische (multi-site) Inhibitoren, wie beispielsweise Alkylhalogenide, z. B. Methylbromid und andere Alkylhalogenide; oder Chloropicrin oder Sulfurylfluorid oder Borax oder Brechweinstein oder Methylisocyanaterzeuger, z. B. Diazomet und Metam.
(9) Modulatoren chordotonaler Organe, z. B. Pymetrozin oder Flonicamid.
(10) Milbenwachstumsinhibitoren, wie z. B. Clofentezin, Hexythiazox und Diflovidazin oder Etoxazol.
(11) Mikrobielle Disruptoren der Insektendarmmembran, wie z. B. *Bacillus thuringiensis* Subspezies *israelensis, Bacillus sphaericus, Bacillus thuringiensis* Subspezies *aizawai, Bacillus thuringiensis* Subspezies *kurstaki, Bacillus thuringiensis* Subspezies *tenebrionis* und *B.t*.-Pflanzenproteine: Cry1Ab, Cry1Ac, Cry1Fa, Cry1A.105, Cry2Ab, VIP3A, mCry3A, Cry3Ab, Cry3Bb, Cry34Ab1/35Ab1.
(12) Inhibitoren der mitochondrialen ATP-Synthase, wie ATP-Disruptoren, wie beispielsweise Diafenthiuron oder Organozinnverbindungen, z. B. Azocyclotin, Cyhexatin und Fenbutatin-oxid oder Propargit oder Tetradifon.
(13) Entkoppler der oxidativen Phoshorylierung durch Störung des Protonengradienten, wie beispielsweise Chlorfenapyr, DNOC und Sulfluramid.
(14) Blocker des nicotinischen Acetylcholinrezeptorkanals, wie beispielsweise Bensultap, Cartaphydrochlorid, Thiocyclam und Thiosultap-Natrium.
(15) Inhibitoren der Chitinbiosynthese, Typ 0, wie beispielsweise Bistrifluron, Chlorfluazuron, Diflubenzuron, Flucycloxuron, Flufenoxuron, Hexaflumuron, Lufenuron, Novaluron, Noviflumuron, Teflubenzuron und Triflumuron.
(16) Inhibitoren der Chitinbiosynthese, Typ 1, wie beispielsweise Buprofezin.
(17) Häutungsdisruptor (insbesondere bei Dipteren, d. h. Zweiflüglern), wie beispielsweise Cyromazin.
(18) Ecdyson-Rezeptor-Agonisten, wie beispielsweise Chromafenozid, Halofenozid, Methoxyfenozid und Tebufenozid.
(19) Oktopamin-Rezeptor-Agonisten, wie beispielsweise Amitraz.
(20) Mitochondriale Komplex-III-Elektronentransportinhibitoren, wie beispielsweise Hydramethylnon oder Acequinocyl oder Fluacrypyrim.
(21) Mitochondriale Komplex-I-Elektronentransportinhibitoren, wie beispielsweise METI-Akarizide, z. B. Fenazaquin, Fenpyroximat, Pyrimidifen, Pyridaben, Tebufenpyrad und Tolfenpyrad oder Rotenon (Derris).
(22) Blocker des spannungsabhängigen Natriumkanals, wie z. B. Indoxacarb oder Metaflumizone.
(23) Inhibitoren der Acetyl-CoA-Carboxylase, wie beispielsweise Tetron- und Tetramsäurederivate, z. B. Spirodiclofen, Spiromesifen und Spirotetramat.
(24) Inhibitoren des mitochondrialen Komplex-IV-Elektronentransports, wie beispielsweise Phosphine, z. B. Aluminiumphosphid, Calciumphosphid, Phosphin und Zinkphosphid oder Cyanide, Calciumcyanid, Kaliumcyanid und Natriumcyanid.
(25) Inhibitoren des mitochondrialen Komplex-II-Elektronentransports, wie beispielsweise beta-Ketonitrilderivate, z. B. Cyenopyrafen und Cyflumetofen und Carboxanilide, wie beispielsweise Pyflubumid.
(28) Ryanodinrezeptor-Modulatoren, wie beispielsweise Diamide, z. B. Chlorantraniliprol, Cyantraniliprol und Flubendiamid,
   weitere Wirkstoffe wie beispielsweise Afidopyropen, Afoxolaner, Azadirachtin, Benclothiaz, Benzoximat, Bifenazat, Broflanilid, Bromopropylat, Chinomethionat, Chloroprallethrin, Cryolit, Cyclaniliprol, Cycloxaprid, Cyhalodiamid, Dicloromezotiaz, Dicofol, epsilon-Metofluthrin, epsilon-Momfluthrin, Flometoquin, Fluazaindolizin, Fluensulfon, Flufenerim, Flufenoxystrobin, Flufiprol, Fluhexafon, Fluopyram, Fluralaner, Fluxametamid, Fufenozid, Guadipyr, Heptafluthrin, Imidaclothiz, Iprodione, kappa-Bifenthrin, kappa-Tefluthrin, Lotilaner, Meperfluthrin, Paichongding, Pyridalyl, Pyrifluquinazon, Pyriminostrobin, Spirobudiclofen, Tetramethylfluthrin, Tetraniliprol, Tetrachlorantraniliprol, Tigolaner, Tioxazafen, Thiofluoximat, Triflumezopyrim und Iodmethan; des Weiteren Präparate auf Basis von *Bacillus firmus* (I-1582, BioNeem, Votivo), sowie folgende Verbindungen: 1-{2-Fluor-4-methyl-5-[(2,2,2-trifluorethyl)sulfinyl]phenyl}-3-(trifluormethyl)-1H-1,2,4-triazol-5-amin (bekannt aus WO2006/043635) (CAS 885026-50-6), {1'-[(2E)-3-(4-Chlorphenyl)prop-2-en-1-yl]-5-fluorspiro[indol-3,4'-piperidin]-1(2H)-yl}(2-chlorpyridin-4-yl)methanon (bekannt aus WO2003/106457) (CAS 637360-23-7), 2-Chlor-N-[2-{1-[(2E)-3-(4-chlorphenyl)prop-2-en-1-yl]piperidin-4-yl}-4-(trifluormethyl)phenyl]isonicotinamid (bekannt aus WO2006/003494) (CAS 872999-66-1), 3-(4-Chlor-2,6-dimethylphenyl)-4-hydroxy-8-methoxy-1,8-diazaspiro[4.5]dec-3-en-2-on (bekannt aus WO 2010052161) (CAS 1225292-17-0), 3-(4-Chlor-2, 6-dimethylphenyl)-8-methoxy-2-oxo-1,8-diazaspiro[4.5]dec-3-en-4-yl-ethylcarbonat (bekannt aus EP 2647626) (CAS-1440516-42-6), 4-(But-2-in-1-yloxy)-6-(3,5-dimethylpiperidin-1-yl)-5-fluorpyrimidin (bekannt aus WO2004/099160) (CAS 792914-58-0), PF1364 (bekannt aus JP2010/018586) (CAS-Reg.No. 1204776-60-2), N-[(2E)-1-[(6-Chlorpyridin-3-yl)methyl]pyridin-2(1H)-yliden]-2,2,2-trifluoracetamid (bekannt aus WO2012/029672) (CAS 1363400-41-2), (3E)-3-[1-[(6-Chlor-3-pyridyl)methyl]-2-pyridyliden]-1,1,1-trifluorpropan-2-on (bekannt aus WO2013/144213) (CAS 1461743-15-6), N-[3-(Benzylcarbamoyl)-4-chlorphenyl]-1-methyl-3-(pentafluorethyl)-4-(trifluormethyl)-1H-pyrazol-5-carboxamid (bekannt aus WO2010/051926) (CAS 1226889-14-0), 5-Brom-4-chlor-N-[4-chlor-2-methyl-6-(methylcarbamoyl)phenyl]-2-(3-chlor-2-pyridyl)pyrazol-3-carboxamid (bekannt aus CN103232431) (CAS 1449220-44-3), 4-[5-(3,5-Dichlorphenyl)-4,5-dihydro-5-(trifluormethyl)-3-isoxazolyl]-2-methyl-N-(cis-1-oxido-3-thietanyl)benzamid, 4-[5-(3,5-Dichlorphenyl)-4,5-dihydro-5-(trifluormethyl)-3-isoxazolyl]-2-methyl-N-(trans-1-oxido-3-thietanyl)benzamid und 4-[(5S)-5-(3,5-Dichlorphenyl)-4,5-dihydro-5-(trifluormethyl)-3-isoxazolyl]-2-methyl-N-(cis-1-oxido-3-thietanyl)benzamid (bekannt aus WO 2013/050317 A1) (CAS 1332628-83-7), N-[3-Chlor-1-(3-pyridinyl)-1H-pyrazol-4-yl]-N-ethyl-3-[(3,3,3-trifluorpropyl)sulfinyl]propanamid, (+)-N-[3-Chlor-1-(3-pyridinyl)-1H-pyrazol-4-yl]-N-ethyl-3-[(3,3,3-trifluorpropyl)sulfinyl]propanamid und (-)-N-[3-Chlor-1-(3-pyridinyl)-1H-pyrazol-4-yl]-N-ethyl-3-[(3,3,3-trifluorpropyl)sulfinyl]propanamid (bekannt aus WO 2013/162715 A2, WO 2013/162716 A2, US 2014/0213448 A1) (CAS 1477923-37-7), 5-[[(2E)-3-Chlor-2-propen-1-yl]amino]-1-[2,6-dichlor-4-(trifluormethyl)phenyl]-4-[(trifluormethyl)sulfinyl]-1H-pyrazol-3-carbonitrile (bekannt aus CN 101337937 A) (CAS 1105672-77-2), 3-Brom-N-[4-chlor-2-methyl-6-[(methylamino)thioxomethyl]phenyl]-1-(3-chlor-2-pyridinyl)-1H-pyrazol-5-carboxamid, (Liudaibenjiaxuanan, bekannt aus CN 103109816 A) (CAS 1232543-85-9); N-[4-Chlor-2-[[(1,1-dimethylethyl)amino]carbonyl]-6-methylphenyl]-1-(3-chlor-2-pyridinyl)-3-(fluormethoxy)-1H-pyrazol-5-carboxamid (bekannt aus WO 2012/034403 A1) (CAS 1268277-22-0), N-[2-(5-Amino-1,3,4-thiadiazol-2-yl)-4-chlor-6-methylphenyl]-3-brom-1-(3-chlor-2-pyridinyl)-1H-pyrazol-5-carboxamid (bekannt aus WO 2011/085575 A1) (CAS 1233882-22-8), 4-[3-[2,6-Dichlor-4-[(3,3-dichlor-2-propen-1-yl)oxy]phenoxy]propoxy]-2-methoxy-6-(trifluormethyl)pyrimidin (bekannt aus CN 101337940 A) (CAS 1108184-52-6); (2E)- und 2(Z)-2-[2-(4-Cyanophenyl)-1-[3-(trifluormethyl)phenyl]ethyliden]-N-[4-(difluormethoxy)phenyl]hydrazincarboxamid (bekannt aus CN 101715774 A) (CAS 1232543-85-9); Cyclopropancarbonsäure-3-(2,2-dichlorethenyl)-2,2-dimethyl-4-(1H-benzimidazol-2-yl)phenylester (bekannt aus CN 103524422 A) (CAS 1542271-46-4); (4aS)-7-Chlor-2,5-dihydro-2-[[(methoxycarbonyl)[4-[(trifluormethyl)thio]phenyl]amino]carbonyl]indeno[1,2-e] [1,3,4]oxadiazin-4a(3H)-carbonsäuremethylester (bekannt aus CN 102391261 A) (CAS 1370358-69-2); 6-Desoxy-3-O-ethyl-2,4-di-O-methyl-1-[N-[4-[1-[4-(1,1,2,2,2-pentafluorethoxy)phenyl]-1H-1,2,4-triazol-3-yl]phenyl]carbamat]-α-L-mannopyranose (bekannt aus US 2014/0275503 A1) (CAS 1181213-14-8); 8-(2-Cyclopropylmethoxy-4-trifluormethylphenoxy)-3-(6-trifluormethylpyridazin-3-yl)-3-azabicyclo[3.2.1]octan (CAS 1253850-56-4), (8-anti)-8-(2-Cyclopropylmethoxy-4-trifluormethylphenoxy)-3-(6-trifluormethylpyridazin-3-yl)-3-azabicyclo[3.2.1]octan (CAS 933798-27-7), (8-syn)-8-(2-Cyclopropylmethoxy-4-trifluormethylphenoxy)-3-(6-trifluormethylpyridazin-3-yl)-3-azabicyclo[3.2.1]octan (bekannt aus WO 2007040280 A1, WO 2007040282 A1) (CAS 934001-66-8), N-[3-Chlor-1-(3-pyridinyl)-1H-pyrazol-4-yl]-N-ethyl-3-[(3,3,3-trifluorpropyl)thio]-propanamid (bekannt aus WO 2015/058021 A1, WO 2015/058028 A1) (CAS 1477919-27-9) und N-[4-(Aminothioxomethyl)-2-methyl-6-[(methylamino)carbonyl]phenyl]-3-bromo-1-(3-chloro-2-pyridinyl)-1*H*-pyrazol-5-carboxamid (bekannt aus CN 103265527 A) (CAS 1452877-50-7), 5-(1,3-Dioxan-2-yl)-4-[[4-(trifluormethyl)phenyl]methoxy]-pyrimidin (bekannt aus WO 2013/115391 A1) (CAS 1449021-97-9), 3-(4-Chlor-2,6-dimethylphenyl)-4-hydroxy-8-methoxy-1-methyl-1,8-diazaspiro[4.5]dec-3-en-2-on (bekannt aus WO 2010/066780 A1, WO 2011/151146 A1) (CAS 1229023-34-0), 3-(4-Chlor-2,6-dimethylphenyl)-8-methoxy-1-methyl-1,8-diazaspiro[4.5]decane-2,4-dion (bekannt aus WO 2014/187846 A1) (CAS 1638765-58-8), 3-(4-Chlor-2,6-dimethylphenyl)-8-methoxy-1-methyl-2-oxo-1, 8-diazaspiro[4.5]dec-3-en-4-yl-carbonsäureethylester (bekannt aus WO 2010/066780 A1, WO 2011151146 A1) (CAS 1229023-00-0), N-[1-[(6-chlor-3-pyridinyl)methyl]-2(1*H*)-pyridinylidene]-2,2, 2-trifluor-acetamide (bekannt aus DE 3639877 A1, WO 2012029672 A1) (CAS 1363400-41-2), [N(*E*)]-N-[1-[(6-chlor-3-pyridinyl)methyl]-2(1*H*)-pyridinylidene]-2,2,2-trifluor-acetamide (bekannt aus WO 2016005276 A1) (CAS 1689566-03-7), [N(Z)]-N-[1-[(6-chlor-3-pyridinyl)methyl]-2(1*H*)-pyridinylidene]-2,2,2-trifluor- acetamide (CAS 1702305-40-5), 3-*endo*-3-[2-Propoxy-4-(trifluormethyl) phenoxy]-9-[[5-(trifluormethyl)-2-pyridinyl]oxy]-9-azabicyclo[3.3.1]nonan (bekannt aus WO 2011/105506 A1, WO 2016/133011 A1) (CAS 1332838-17-1).

### Fungizide

Die hier mit ihrem "Common Name" spezifizierten Wirkstoffe sind bekannt und beispielsweise im "Pesticide Manual" (16. Aufl. British Crop Protection Council) oder im Internet recherchierbar (beispielsweise: http://www.alanwood.net/pesticides) beschrieben.

Alle genannten Mischungspartner der Klassen (1) bis (15) können, wenn sie auf Grund ihrer funktionellen Gruppen dazu imstande sind, gegebenenfalls mit geeigneten Basen oder Säuren Salze bilden. Alle genannten fungiziden Mischungspartner der Klassen (1) bis (15) können gegebenenfalls tautomere Formen einschließen.
1) Inhibitoren der Ergosterol-Biosynthese, beispielsweise (1.001) Cyproconazol, (1.002) Difenoconazol, (1.003) Epoxiconazol, (1.004) Fenhexamid, (1.005) Fenpropidin, (1.006) Fenpropimorph, (1.007) Fenpyrazamin, (1.008) Fluquinconazol, (1.009) Flutriafol, (1.010) Imazalil, (1.011) Imazalil Sulfat, (1.012) Ipconazol, (1.013) Metconazol, (1.014) Myclobutanil, (1.015) Paclobutrazol, (1.016) Prochloraz, (1.017) Propiconazol, (1.018) Prothioconazol, (1.019) Pyrisoxazol, (1.020) Spiroxamin, (1.021) Tebuconazol, (1.022) Tetraconazol, (1.023) Triadimenol, (1.024) Tridemorph, (1.025) Triticonazol, (1.026) (1R,2S,5S)-5-(4-Chlorbenzyl)-2-(chlormethyl)-2-methyl-1-(1H-1,2,4-triazol-1-ylmethyl)cyclopentanol, (1.027) (1S,2R,5R)-5-(4-Chlorbenzyl)-2-(chlormethyl)-2-methyl-1-(1H-1,2,4-triazol-1-ylmethyl)cyclopentanol, (1.028) (2R)-2-(1-Chlorcyclopropyl)-4-[(1R)-2,2-dichlorcyclopropyl]-1-(1H-1,2,4-triazol-1-yl)butan-2-ol (1.029) (2R)-2-(1-Chlorcyclopropyl)-4-[(1S)-2,2-dichlorcyclopropyl]-1-(1H-1,2,4-triazol-1-yl)butan-2-ol, (1.030) (2R)-2-[4-(4-Chlorphenoxy)-2-(trifluormethyl)phenyl]-1-(1H-1,2,4-triazol-1-yl)propan-2-ol, (1.031) (2S)-2-(1-Chlorcyclopropyl)-4-[(1R)-2,2-dichlorcyclopropyl]-1-(1H-1,2,4-triazol-1-yl)butan-2-ol, (1.032) (2S)-2-(1-Chlorcyclopropyl)-4-[(1S)-2,2-dichlorcyclopropyl]-1-(1H-1,2,4-triazol-1-yl)butan-2-ol, (1.033) (2S)-2-[4-(4-Chlorphenoxy)-2-(trifluormethyl)phenyl]-1-(1H-1,2,4-triazol-1-yl)propan-2-ol, (1.034) (R)-[3-(4-Chlor-2-fluorphenyl)-5-(2,4-difluorphenyl)-1,2-oxazol-4-yl](pyridin-3-yl)methanol, (1.035) (S)-[3-(4-Chlor-2-fluorphenyl)-5-(2,4-difluorphenyl)-1,2-oxazol-4-yl](pyridin-3-yl)methanol, (1.036) [3-(4-Chlor-2-fluorphenyl)-5-(2,4-difluorphenyl)-1,2-oxazol-4-yl](pyridin-3-yl)methanol, (1.037) 1-({(2R,4S)-2-[2-Chlor-4-(4-chlorphenoxy)phenyl]-4-methyl-1,3-dioxolan-2-yl}methyl)-1H-1,2,4-triazol, (1.038) 1-({(2S,4S)-2-[2-Chlor-4-(4-chlorphenoxy)phenyl]-4-methyl-1,3-dioxolan-2-yl}methyl)-1H-1,2,4-triazol, (1.039) 1-{[3-(2-Chlorphenyl)-2-(2,4-difluorphenyl)oxiran-2-yl]methyl}-1H-1,2,4-triazol-5-yl-thiocyanat, (1.040) 1-{[rel(2R,3R)-3-(2-Chlorphenyl)-2-(2,4-difluorphenyl)oxiran-2-yl]methyl}-1H-1,2,4-triazol-5-yl-thiocyanat, (1.041) 1-{[rel(2R,3S)-3-(2-Chlorphenyl)-2-(2,4-difluorphenyl)oxiran-2-yl]methyl}-1H-1,2,4-triazol-5-yl-thiocyanat, (1.042) 2-[(2R,4R,5R)-1-(2,4-Dichlorphenyl)-5-hydroxy-2,6,6-trimethylheptan-4-yl]-2,4-dihydro-3H-1,2,4-triazol-3-thion, (1.043) 2-[(2R,4R,5S)-1-(2,4-Dichlorphenyl)-5-hydroxy-2,6,6-trimethylheptan-4-yl]-2,4-dihydro-3H-1,2,4-triazol-3-thion, (1.044) 2-[(2R,4S,5R)-1-(2,4-Dichlorophenyl)-5-hydroxy-2,6,6-trimethylheptan-4-yl]-2,4-dihydro-3H-1,2,4-triazol-3-thion, (1.045) 2-[(2R,4S,5S)-1-(2,4-Dichlorphenyl)-5-hydroxy-2,6,6-trimethylheptan-4-yl]-2,4-dihydro-3H-1,2,4-triazol-3-thion, (1.046) 2-[(2S,4R,5R)-1-(2,4-Dichlorphenyl)-5-hydroxy-2,6,6-trimethylheptan-4-yl]-2,4-dihydro-3H-1,2,4-triazol-3-thion, (1.047) 2-[(2S,4R,5S)-1-(2,4-Dichlorphenyl)-5-hydroxy-2,6,6-trimethylheptan-4-yl]-2,4-dihydro-3H-1,2,4-triazol-3-thion, (1.048) 2-[(2S,4S,5R)-1-(2,4-Dichlorphenyl)-5-hydroxy-2,6,6-trimethylheptan-4-yl]-2,4-dihydro-3H-1,2,4-triazol-3-thion, (1.049) 2-[(2S,4S,5S)-1-(2,4-Dichlorphenyl)-5-hydroxy-2,6,6-trimethylheptan-4-yl]-2,4-dihydro-3H-1,2,4-triazol-3-thion, (1.050) 2-[1-(2,4-Dichlorphenyl)-5-hydroxy-2,6,6-trimethylheptan-4-yl]-2,4-dihydro-3H-1,2,4-triazol-3-thion, (1.051) 2-[2-Chlor-4-(2,4-dichlorophenoxy)phenyl]-1-(1H-1,2,4-triazol-1-yl)propan-2-ol, (1.052) 2-[2-Chlor-4-(4-chlorphenoxy)phenyl]-1-(1H-1,2,4-triazol-1-yl)butan-2-ol, (1.053) 2-[4-(4-Chlorphenoxy)-2-(trifluormethyl)phenyl]-1-(1H-1,2,4-triazol-1-yl)butan-2-ol, (1.054) 2-[4-(4-Chlorphenoxy)-2-(trifluormethyl)phenyl]-1-(1H-1,2,4-triazol-1-yl)pentan-2-ol, (1.055) 2-[4-(4-Chlorphenoxy)-2-(trifluormethyl)phenyl]-1-(1H-1,2,4-triazol-1-yl)propan-2-ol, (1.056) 2-{[3-(2-Chlorphenyl)-2-(2,4-difluorphenyl)oxiran-2-yl]methyl}-2,4-dihydro-3H-1,2,4-triazol-3-thion, (1.057) 2-{[rel(2R,3R)-3-(2-Chlorphenyl)-2-(2,4-difluorphenyl)oxiran-2-yl]methyl}-2,4-dihydro-3H-1,2,4-triazol-3-thion, (1.058) 2-{[rel(2R,3S)-3-(2-Chlorphenyl)-2-(2,4-difluorphenyl)oxiran-2-yl]methyl}-2,4-dihydro-3H-1,2,4-triazol-3-thion, (1.059) 5-(4-Chlorbenzyl)-2-(chlormethyl)-2-methyl-1-(1H-1,2,4-triazol-1-ylmethyl)cyclopentanol, (1.060) 5-(Allylsulfanyl)-1-{[3-(2-chlorphenyl)-2-(2,4-difluorphenyl)oxiran-2-yl]methyl} -1H-1,2,4-triazol, (1.061) 5-(Allylsulfanyl)-1-{[rel(2R,3R)-3-(2-chlorphenyl)-2-(2,4-difluorphenyl)oxiran-2-yl]methyl}-1H-1,2,4-triazol, (1.062) 5-(Allylsulfanyl)-1-{[rel(2R,3S)-3-(2-chlorphenyl)-2-(2,4-difluorphenyl)oxiran-2-yl]methyl}-1H-1,2,4-triazol, (1.063) N'-(2,5-Dimethyl-4-{[3-(1,1,2,2-tetrafluorethoxy)phenyl]sulfanyl}phenyl)-N-ethyl-N-methylimidoformamid, (1.064) N'-(2,5-Dimethyl-4-{[3-(2,2,2-trifluorethoxy)phenyl]sulfanyl}phenyl)-N-ethyl-N-methylimidoformamid, (1.065) N'-(2,5-Dimethyl-4- {[3-(2,2,3,3-tetrafluorpropoxy)phenyl]sulfanyl}phenyl)-N-ethyl-N-methylimidoformamid, (1.066) N'-(2,5-Dimethyl-4-{[3-(pentafluorethoxy)phenyl]sulfanyl}phenyl)-N-ethyl-N-methylimidoformamid, (1.067) N'-(2,5-Dimethyl-4-{3-[(1,1,2,2-tetrafluorethyl)sulfanyl]phenoxy}phenyl)-N-ethyl-N-methylimidoformamid, (1.068) N'-(2,5-Dimethyl-4- {3-[(2,2,2-trifluorethyl)sulfanyl]phenoxy}phenyl)-N-ethyl-N-methylimidoformamid, (1.069) N'-(2,5-Dimethyl-4-{3-[(2,2,3,3-tetrafluorpropyl)sulfanyl]phenoxy}phenyl)-N-ethyl-N-methylimidoformamid, (1.070) N'-(2,5-Dimethyl-4-{3-[(pentafluorethyl)sulfanyl]phenoxy}phenyl)-N-ethyl-N-methylimidoformamid, (1.071) N'-(2,5-Dimethyl-4-phenoxyphenyl)-N-ethyl-N-methylimidoformamid, (1.072) N'-(4-{[3-(Difluormethoxy)phenyl]sulfanyl}-2,5-dimethylphenyl)-N-ethyl-N-methylimidoformamid, (1.073) N'-(4-{3-[(Difluormethyl)sulfanyl]phenoxy}-2,5-dimethylphenyl)-N-ethyl-N-methylimidoformamid, (1.074) N'-[5-Brom-6-(2,3-dihydro-1H-inden-2-yloxy)-2-methylpyridin-3-yl]-N-ethyl-N-methylimidoformamid, (1.075) N'-{4-[(4,5-Dichlor-1,3-thiazol-2-yl)oxy]-2,5-dimethylphenyl}-N-ethyl-N-methylimidoformamid, (1.076) N'-{5-Brom-6-[(1R)-1-(3,5-difluorophenyl)ethoxy]-2-methylpyridin-3-yl}-N-ethyl-N-methylimidoformamid, (1.077) N'-{5-Brom-6-[(1S)-1-(3,5-difluorphenyl)ethoxy]-2-methylpyridin-3-yl}-N-ethyl-N-methylimidoformamid, (1.078) N'-{5-Brom-6-[(cis-4-isopropylcyclohexyl)oxy]-2-methylpyridin-3-yl}-N-ethyl-N-methylimidoformamid, (1.079) N'-{5-Brom-6-[(trans-4-isopropylcyclohexyl)oxy]-2-methylpyridin-3-yl}-N-ethyl-N-methylimidoformamid, (1.080) N'-{5-Bromo-6-[1-(3,5-difluorphenyl)ethoxy]-2-methylpyridin-3-yl}-N-ethyl-N-methylimidoformamid, (1.081) Mefentrifluconazole, (1.082) Ipfentrifluconazole.
2) Inhibitoren der Atmungskette am Komplex I oder II beispielsweise (2.001) Benzovindiflupyr, (2.002) Bixafen, (2.003) Boscalid, (2.004) Carboxin, (2.005) Fluopyram, (2.006) Flutolanil, (2.007) Fluxapyroxad, (2.008) Furametpyr, (2.009) Isofetamid, (2.010) Isopyrazam (anti-epimeres Enantiomer 1R,4S,9S), (2.011) Isopyrazam (anti-epimeres Enantiomer 1S,4R,9R), (2.012) Isopyrazam (anti-epimeres Racemat 1RS,4SR,9SR), (2.013) Isopyrazam (Mischung des syn-epimeren Razemates 1RS,4SR,9RS und des anti-epimeren Razemates 1RS,4SR,9SR), (2.014) Isopyrazam (syn-epimeres Enantiomer 1R,4S,9R), (2.015) Isopyrazam (syn-epimeres Enantiomer 1S,4R,9S), (2.016) Isopyrazam (syn-epimeres Racemat 1RS,4SR,9RS), (2.017) Penflufen, (2.018) Penthiopyrad, (2.019) Pydiflumetofen, (2.020) Pyraziflumid, (2.021) Sedaxane, (2.022) 1,3-Dimethyl-N-(1,1,3-trimethyl-2,3-dihydro-1H-inden-4-yl)-1H-pyrazol-4-carboxamid, (2.023) 1,3-Dimethyl-N-[(3R)-1,1,3-trimethyl-2,3-dihydro-1H-inden-4-yl]-1H-pyrazol-4-carboxamid, (2.024) 1,3-Dimethyl-N-[(3S)-1,1,3-trimethyl-2,3-dihydro-1H-inden-4-yl]-1H-pyrazol-4-carboxamid, (2.025) 1-Methyl-3-(trifluormethyl)-N-[2'-(trifluormethyl)biphenyl-2-yl]-1H-pyrazol-4-carboxamid, (2.026) 2-Fluor-6-(trifluoromethyl)-N-(1,1,3-trimethyl-2,3-dihydro-1H-inden-4-yl)benzamid, (2.027) 3-(Difluormethyl)-1-methyl-N-(1,1,3-trimethyl-2,3-dihydro-1H-inden-4-yl)-1H-pyrazol-4-carboxamid, (2.028) 3-(Difluormethyl)-1-methyl-N-[(3R)-1,1,3-trimethyl-2,3-dihydro-1H-inden-4-yl]-1H-pyrazol-4-carboxamid, (2.029) 3-(Difluormethyl)-1-methyl-N-[(3S)-1,1,3-trimethyl-2,3-dihydro-1H-inden-4-yl]-1H-pyrazol-4-carboxamid, (2.030) 3-(Difluormethyl)-N-(7-fluor-1,1,3-trimethyl-2,3-dihydro-1H-inden-4-yl)-1-methyl-1H-pyrazol-4-carboxamid, (2.031) 3-(Difluormethyl)-N-[(3R)-7-fluor-1,1,3-trimethyl-2,3-dihydro-1H-inden-4-yl]-1-methyl-1H-pyrazol-4-carboxamid, (2.032) 3-(Difluoromethyl)-N-[(3S)-7-fluor-1,1,3-trimethyl-2,3-dihydro-1H-inden-4-yl]-1-methyl-1H-pyrazol-4-carboxamid, (2.033) 5,8-Difluor-N-[2-(2-fluor-4-{[4-(trifluormethyl)pyridin-2-yl]oxy}phenyl)ethyl]quinazolin-4-amin, (2.034) N-(2-Cyclopentyl-5-fluorbenzyl)-N-cyclopropyl-3-(difluormethyl)-5-fluor-1-methyl-1H-pyrazol-4-carboxamid, (2.035) N-(2-tert-Butyl-5-methylbenzyl)-N-cyclopropyl-3-(difluormethyl)-5-fluor-1-methyl-1H-pyrazol-4-carboxamid, (2.036) N-(2-tert-Butylbenzyl)-N-cyclopropyl-3-(difluormethyl)-5-fluor-1-methyl-1H-pyrazol-4-carboxamid, (2.037) N-(5-Chlor-2-ethylbenzyl)-N-cyclopropyl-3-(difluormethyl)-5-fluor-1-methyl-1H-pyrazol-4-carboxamid, (2.038) N-(5-Chlor-2-isopropylbenzyl)-N-cyclopropyl-3-(difluormethyl)-5-fluor-1-methyl-1H-pyrazol-4-carboxamid, (2.039) N-[(1R,4S)-9-(Dichlormethylen)-1,2,3,4-tetrahydro-1,4-methanonaphthalen-5-yl]-3-(difluormethyl)-1-methyl-1 H-pyrazol-4-carboxamid, (2.040) N-[(1S,4R)-9-(Dichlormethylen)-1,2,3,4-tetrahydro-1,4-methanonaphthalen-5-yl]-3-(difluormethyl)-1-methyl-1H-pyrazol-4-carboxamid, (2.041) N-[1-(2,4-Dichlorphenyl)-1-methoxypropan-2-yl]-3-(difluormethyl)-1-methyl-1H-pyrazol-4-carboxamid, (2.042) N-[2-Chlor-6-(trifluormethyl)benzyl]-N-cyclopropyl-3-(difluormethyl)-5-fluor-1-methyl-1H-pyrazol-4-carboxamid, (2.043) N-[3-Chlor-2-fluor-6-(trifluormethyl)benzyl]-N-cyclopropyl-3-(difluormethyl)-5-fluor-1-methyl-1H-pyrazol-4-carboxamid, (2.044) N-[5-Chlor-2-(trifluormethyl)benzyl]-N-cyclopropyl-3-(difluormethyl)-5-fluor-1-methyl-1H-pyrazol-4-carboxamid, (2.045) N-Cyclopropyl-3-(difluormethyl)-5-fluor-1-methyl-N-[5-methyl-2-(trifluormethyl)benzyl]-1H-pyrazol-4-carboxamid, (2.046) N-Cyclopropyl-3-(difluormethyl)-5-fluor-N-(2-fluor-6-isopropylbenzyl)-1-methyl-1H-pyrazol-4-carboxamid, (2.047) N-Cyclopropyl-3-(difluormethyl)-5-fluor-N-(2-isopropyl-5-methylbenzyl)-1-methyl-1H-pyrazol-4-carboxamid, (2.048) N-Cyclopropyl-3-(difluormethyl)-5-fluor-N-(2-isopropylbenzyl)-1-methyl-1H-pyrazol-4-carbothioamid, (2.049) N-Cyclopropyl-3-(difluoromethyl)-5-fluor-N-(2-isopropylbenzyl)-1-methyl-1H-pyrazol-4-carboxamid, (2.050) N-Cyclopropyl-3-(difluormethyl)-5-fluor-N-(5-fluor-2-isopropylbenzyl)-1-methyl-1H-pyrazol-4-carboxamid, (2.051) N-Cyclopropyl-3-(difluormethyl)-N-(2-ethyl-4,5-dimethylbenzyl)-5-fluor-1-methyl-1H-pyrazol-4-carboxamid, (2.052) N-Cyclopropyl-3-(difluormethyl)-N-(2-ethyl-5-fluorbenzyl)-5-fluor-1-methyl-1H-pyrazol-4-carboxamid, (2.053) N-Cyclopropyl-3-(difluormethyl)-N-(2-ethyl-5-methylbenzyl)-5-fluor-1-methyl-1H-pyrazole-4-carboxamid, (2.054) N-Cyclopropyl-N-(2-cyclopropyl-5-fluorbenzyl)-3-(difluormethyl)-5-fluor-1-methyl-1H-pyrazole-4-carboxamid, (2.055) N-Cyclopropyl-N-(2-cyclopropyl-5-methylbenzyl)-3-(difluormethyl)-5-fluor-1-methyl-1H-pyrazole-4-carboxamid, (2.056) N-Cyclopropyl-N-(2-cyclopropylbenzyl)-3-(difluormethyl)-5-fluor-1-methyl-1H-pyrazole-4-carboxamid.
3) Inhibitoren der Atmungskette am Komplex III, beispielsweise (3.001) Ametoctradin, (3.002) Amisulbrom, (3.003) Azoxystrobin, (3.004) Coumethoxystrobin, (3.005) Coumoxystrobin, (3.006) Cyazofamid, (3.007) Dimoxystrobin, (3.008) Enoxastrobin, (3.009) Famoxadon, (3.010) Fenamidon, (3.011) Flufenoxystrobin, (3.012) Fluoxastrobin, (3.013) Kresoxim-Methyl, (3.014) Metominostrobin, (3.015) Orysastrobin, (3.016) Picoxystrobin, (3.017) Pyraclostrobin, (3.018) Pyrametostrobin, (3.019) Pyraoxystrobin, (3.020) Trifloxystrobin (3.021) (2E)-2-{2-[({[(1E)-1-(3-{[(E)-1-Fluor-2-phenylvinyl]oxy}phenyl)ethyliden]amino}oxy)methyl]phenyl}-2-(methoxyimino)-N-methylacetamid, (3.022) (2E,3Z)-5-{[1-(4-Chlorphenyl)-1H-pyrazol-3-yl]oxy}-2-(methoxyimino)-N,3-dimethylpent-3-enamid, (3.023) (2R)-2-{2-[(2,5-Dimethylphenoxy)methyl]phenyl}-2-methoxy-N-methylacetamid, (3.024) (2S)-2-{2-[(2,5-Dimethylphenoxy)methyl]phenyl}-2-methoxy-N-methylacetamid, (3.025) (3S,6S,7R,8R)-8-Benzyl-3-[({3-[(isobutyryloxy)methoxy]-4-methoxypyridin-2-yl}carbonyl)amino]-6-methyl-4,9-dioxo-1,5-dioxonan-7-yl-2-methylpropanoat, (3.026) 2-{2-[(2,5-Dimethylphenoxy)methyl]phenyl}-2-methoxy-N-methylacetamid, (3.027) N-(3-Ethyl-3,5,5-trimethylcyclohexyl)-3-formamido-2-hydroxybenzamid, (3.028) (2E,3Z)-5-{[1-(4-Chlor-2-fluorphenyl)-1H-pyrazol-3-yl]oxy}-2-(methoxyimino)-N,3-dimethylpent-3-enamid, (3.029) Methyl {5-[3-(2,4-dimethylphenyl)-1H-pyrazol-1-yl]-2-methylbenzyl}carbamate.
4) Inhibitoren der Mitose und Zellteilung, beispielsweise (4.001) Carbendazim, (4.002) Diethofencarb, (4.003) Ethaboxam, (4.004) Fluopicolid, (4.005) Pencycuron, (4.006) Thiabendazol, (4.007) Thiophanat-Methyl, (4.008) Zoxamid, , (4.009) 3-Chlor-4-(2,6-difluorphenyl)-6-methyl-5-phenylpyridazin, (4.010) 3-Chlor-5-(4-chlorphenyl)-4-(2,6-difluorphenyl)-6-methylpyridazin, (4.011) 3-Chlor-5-(6-chlorpyridin-3-yl)-6-methyl-4-(2,4,6-trifluorphenyl)pyridazin, (4.012) 4-(2-Brom-4-fluorphenyl)-N-(2,6-difluorphenyl)-1,3-dimethyl-1H-pyrazol-5-amin, (4.013) 4-(2-Brom-4-fluorphenyl)-N-(2-brom-6-fluorphenyl)-1,3-dimethyl-1H-pyrazol-5-amin, (4.014) 4-(2-Brom-4-fluorphenyl)-N-(2-bromphenyl)-1,3-dimethyl-1H-pyrazol-5-amin, (4.015) 4-(2-Brom-4-fluorphenyl)-N-(2-chlor-6-fluorphenyl)-1,3-dimethyl-1H-pyrazol-5-amin, (4.016) 4-(2-Brom-4-fluorphenyl)-N-(2-chlorphenyl)-1,3-dimethyl-1H-pyrazol-5-amin, (4.017) 4-(2-Brom-4-fluorphenyl)-N-(2-fluorphenyl)-1,3-dimethyl-1H-pyrazol-5-amin, (4.018) 4-(2-Chlor-4-fluorphenyl)-N-(2,6-difluorphenyl)-1,3-dimethyl-1H-pyrazol-5-amin, (4.019) 4-(2-Chlor-4-fluorphenyl)-N-(2-chlor-6-fluorphenyl)-1,3-dimethyl-1H-pyrazol-5-amin, (4.020) 4-(2-Chlor-4-fluorphenyl)-N-(2-chlorphenyl)-1,3-dimethyl-1H-pyrazol-5-amin, (4.021) 4-(2-Chlor-4-fluorphenyl)-N-(2-fluorphenyl)-1,3-dimethyl-1H-pyrazol-5-amin, (4.022) 4-(4-Chlorphenyl)-5-(2,6-difluorphenyl)-3,6-dimethylpyridazin, (4.023) N-(2-Brom-6-fluorphenyl)-4-(2-chlor-4-fluorphenyl)-1,3-dimethyl-1H-pyrazol-5-amin, (4.024) N-(2-Bromphenyl)-4-(2-chlor-4-fluorphenyl)-1,3-dimethyl-1H-pyrazol-5-amin, (4.025) N-(4-Chlor-2,6-difluorphenyl)-4-(2-chlor-4-fluorphenyl)-1,3-dimethyl-1H-pyrazol-5-amin.
5) Verbindungen mit Befähigung zu Multisite-Aktivität, beispielsweise (5.001) Bordeauxmischung, (5.002) Captafol, (5.003) Captan, (5.004) Chlorthalonil, (5.005) Kupferhydroxid, (5.006) Kupfernaphthenat, (5.007) Kupferoxid, (5.008) Kupferoxychlorid, (5.009) Kupfer(2+)-sulfat, (5.010) Dithianon, (5.011) Dodin, (5.012) Folpet, (5.013) Mancozeb, (5.014) Maneb, (5.015) Metiram, (5.016) Zinkmetiram, (5.017) Kupfer-Oxin, (5.018) Propineb, (5.019) Schwefel und Schwefelzubereitungen einschließlich Calciumpolysulfid, (5.020) Thiram, (5.021) Zineb, (5.022) Ziram, (5.023) 6-Ethyl-5,7-dioxo-6,7-dihydro-5H-pyrrolo[3',4':5,6][1,4]dithiino[2,3-c][1,2]thiazole-3-carbonitrile.
6) Verbindungen, die zum Auslösen einer Wirtsabwehr befähigt sind, beispielsweise (6.001) Acibenzolar-S-Methyl, (6.002) Isotianil, (6.003) Probenazol, (6.004) Tiadinil.
7) Inhibitoren der Aminosäure- und/oder Protein-Biosynthese, beispielsweise (7.001) Cyprodinil, (7.002) Kasugamycin, (7.003) Kasugamycinhydrochlorid-hydrat, (7.004) Oxytetracyclin (7.005) Pyrimethanil, (7.006) 3-(5-Fluor-3,3,4,4-tetramethyl-3,4-dihydroisochinolin-1-yl)chinolin.
(8) Inhibitoren der ATP-Produktion, beispielsweise (8.001) Silthiofam.
9) Inhibitoren der Zellwandsynthese, beispielsweise (9.001) Benthiavalicarb, (9.002) Dimethomorph, (9.003) Flumorph, (9.004) Iprovalicarb, (9.005) Mandipropamid, (9.006) Pyrimorph, (9.007) Valifenalat, (9.008) (2E)-3-(4-tert.-Butylphenyl)-3-(2-chlorpyridin-4-yl)-1-(morpholin-4-yl)prop-2-en-1-on, (9.009) (2Z)-3-(4-tert.-Butylphenyl)-3-(2-chlorpyridin-4-yl)-1-(morpholin-4-yl)prop-2-en-1-on.
10) Inhibitoren der Lipid- und Membran-Synthese, beispielsweise (10.001) Propamocarb, (10.002) Propamocarbhydrochlorid, (10.003) Tolclofos-Methyl.
11) Inhibitoren der Melanin-Biosynthese, beispielsweise (11.001) Tricyclazol, (11.002) 2,2,2-Trifluorethyl-{3-methyl-1-[(4-methylbenzoyl)amino]butan-2-yl}carbamat.
12) Inhibitoren der Nukleinsäuresynthese, beispielsweise (12.001) Benalaxyl, (12.002) Benalaxyl-M (Kiralaxyl), (12.003) Metalaxyl, (12.004) Metalaxyl-M (Mefenoxam).
13) Inhibitoren der Signaltransduktion, beispielsweise (13.001) Fludioxonil, (13.002) Iprodion, (13.003) Procymidon, (13.004) Proquinazid, (13.005) Quinoxyfen, (13.006) Vinclozolin.
14) Verbindungen, die als Entkoppler wirken können, beispielsweise (14.001) Fluazinam, (14.002) Meptyldinocap.
15) Weitere Verbindungen, beispielsweise (15.001) Abscisinsäure, (15.002) Benthiazol, (15.003) Bethoxazin, (15.004) Capsimycin, (15.005) Carvon, (15.006) Chinomethionat, (15.007) Cufraneb, (15.008) Cyflufenamid, (15.009) Cymoxanil, (15.010) Cyprosulfamid, (15.011) Flutianil, (15.012) Fosetyl-Aluminium, (15.013) Fosetyl-Calcium, (15.014) Fosetyl-Natrium, (15.015) Methylisothiocyanat, (15.016) Metrafenon, (15.017) Mildiomycin, (15.018) Natamycin, (15.019) Nickel-Dimethyldithiocarbamat, (15.020) Nitrothal-Isopropyl, (15.021) Oxamocarb, (15.022) Oxathiapiprolin, (15.023) Oxyfenthiin, (15.024) Pentachlorphenol und Salze, (15.025) Phosphonsäure und deren Salze, (15.026) Propamocarb-fosetylat, (15.027) Pyriofenone (Chlazafenone) (15.028) Tebufloquin, (15.029) Tecloftalam, (15.030) Tolnifanide, (15.031) 1-(4-{4-[(5R)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, (15.032) 1-(4-{4-[(5S)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, (15.033) 2-(6-Benzylpyridin-2-yl)quinazolin, (15.034) 2,6-Dimethyl-1H,5H-[1,4]dithiino[2,3-c:5,6-c']dipyrrol-1,3,5,7(2H,6H)-tetron, (15.035) 2-[3,5-Bis(difluormethyl)-1H-pyrazol-1-yl]-1-[4-(4-{5-[2-(prop-2-in-1-yloxy)phenyl]-4,5-dihydro-1,2-oxazol-3-yl}-1,3-thiazol-2-yl)piperidin-1-yl]ethanon, (15.036) 2-[3,5-Bis(difluormethyl)-1H-pyrazol-1-yl]-1-[4-(4-{5-[2-chlor-6-(prop-2-in-1-yloxy)phenyl]-4,5-dihydro-1,2-oxazol-3-yl}-1,3-thiazol-2-yl)piperidin-1-yl]ethanon, (15.037) 2-[3,5-Bis(difluormethyl)-1H-pyrazol-1-yl]-1-[4-(4-{5-[2-fluor-6-(prop-2-in-1-yloxy)phenyl]-4,5-dihydro-1,2-oxazol-3-yl}-1,3-thiazol-2-yl)piperidin-1-yl]ethanon, (15.038) 2-[6-(3-Fluor-4-methoxyphenyl)-5-methylpyridin-2-yl]quinazolin, (15.039) 2-{(5R)-3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenyl methanesulfonat, (15.040) 2-{(5S)-3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenyl methanesulfonat, (15.041) 2-{2-[(7,8-Difluor-2-methylquinolin-3-yl)oxy]-6-fluorphenyl}propan-2-ol, (15.042) 2-}2-Fluor-6-[(8-fluor-2-methylquinolin-3-yl)oxy]phenyl}propan-2-ol, (15.043) 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenyl-methansulfonat, (15.044) 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}phenyl methanesulfonat, (15.045) 2-Phenylphenol und deren Salze, (15.046) 3-(4,4,5-Trifluor-3,3-dimethyl-3,4-dihydroisoquinolin-1-yl)quinolin, (15.047) 3-(4,4-Difluor-3,3-dimethyl-3,4-dihydroisoquinolin-1-yl)quinolin, (15.048) 4-Amino-5-fluorpyrimidin-2-ol (Tautomere Form: 4-Amino-5-fluorpyrimidin-2(1H)-on), (15.049) 4-Oxo-4-[(2-phenylethyl)amino]buttersäure, (15.050) 5-Amino-1,3,4-thiadiazol-2-thiol, (15.051) 5-Chlor-N'-phenyl-N'-(prop-2-yn-1-yl)thiophen-2-sulfonohydrazid, (15.052) 5-Fluor-2-[(4-fluorbenzyl)oxy]pyrimidin-4-amin, (15.053) 5-Fluor-2-[(4-methylbenzyl)oxy]pyrimidin-4-amin, (15.054) 9-Fluor-2,2-dimethyl-5-(quinolin-3-yl)-2,3-dihydro-1,4-benzoxazepin, (15.055) But-3-yn-1-yl {6-[({[(Z)-(1-methyl-1H-tetrazol-5-yl)(phenyl)methylen]amino}oxy)methyl]pyridin-2-yl}carbamat, (15.056) Ethyl (2Z)-3-amino-2-cyano-3-phenylacrylat, (15.057) Phenazin-1-carbonsäure, (15.058) Propyl 3,4,5-trihydroxybenzoat, (15.059) Quinolin-8-ol, (15.060) Quinolin-8-ol sulfat (2:1), (15.061) tert-Butyl {6-[({[(1-methyl-1H-tetrazol-5-yl)(phenyl)methylene]amino}oxy)methyl]pyridin-2-yl}carbamat, (15.062) 5-Fluor-4-imino-3-methyl-1-[(4-methylphenyl)sulfonyl] -3,4-dihydropyrimidin-2(1H)-one.

### Biologische Schädlingsbekämpfungsmittel als Mischungskomponenten

Die Verbindungen der Formel (I) können mit biologischen Schädlingsbekämpfungsmitteln kombiniert werden.

Biologische Schädlingsbekämpfungsmittel umfassen insbesondere Bakterien, Pilze, Hefen, Pflanzenextrakte und solche Produkte, die von Mikroorganismen gebildet wurden inklusive Proteine und sekundäre Stoffwechselprodukte.

Biologische Schädlingsbekämpfungsmittel umfassen Bakterien wie sporenbildende Bakterien, wurzelbesiedelnde Bakterien und Bakterien, die als biologische Insektizide, Fungizide oder Nematizide wirken.

Beispiele für solche Bakterien, die als biologische Schädlingsbekämpfungsmittel eingesetzt werden bzw. verwendet werden können, sind:
*Bacillus amyloliquefaciens,* Stamm FZB42 (DSM 231179), oder *Bacillus cereus,* insbesondere *B. cereus* Stamm CNCM I-1562 oder *Bacillus firmus,* Stamm I-1582 (Accession number CNCM I-1582) oder *Bacillus pumilus,* insbesondere Stamm GB34 (Accession No. ATCC 700814) und Stamm QST2808 (Accession No. NRRL B-30087), oder *Bacillus subtilis,* insbesondere Stamm GB03 (Accession No. ATCC SD-1397), oder *Bacillus subtilis* Stamm QST713 (Accession No. NRRL B-21661) oder *Bacillus subtilis* Stamm OST 30002 (Accession No. NRRL B-50421), *Bacillus thuringiensis,* insbesondere *B. thuringiensis* Subspezies *israelensis* (Serotyp H-14), Stamm AM65-52 (Accession No. ATCC 1276), oder *B. thuringiensis subsp. aizawai,* insbesondere Stamm ABTS-1857 (SD-1372), oder *B. thuringiensis subsp. kurstaki* Stamm HD-1, oder *B. thuringiensis subsp. tenebrionis* Stamm NB 176 (SD-5428), *Pasteuria penetrans, Pasteuria spp.* (Rotylenchulus reniformis nematode)-PR3 (Accession Number ATCC SD-5834), *Streptomyces microflavus* Stamm AQ6121 (= QRD 31.013, NRRL B-50550), *Streptomyces galbus* Stamm AQ 6047 (Acession Number NRRL 30232).

Beispiele für Pilze und Hefen, die als biologische Schädlingsbekämpfungsmittel eingesetzt werden bzw. verwendet werden können, sind:
*Beauveria bassiana,* insbesondere Stamm ATCC 74040, *Coniothyrium minitans,* insbesondere Stamm CON/M/91-8 (Accession No. DSM-9660), *Lecanicillium spp.,* insbesondere Stamm HRO LEC 12, *Lecanicillium lecanii* (ehemals bekannt als *Verticillium lecanii*)*,* insbesondere Stamm KV01, *Metarhizium anisopliae,* insbesondere Stamm F52 (DSM3884/ ATCC 90448), *Metschnikowia fructicola,* insbesondere Stamm NRRL Y-30752, *Paecilomyces fumosoroseus (*neu: *Isaria fumosorosea),* insbesondere Stamm IFPC 200613, oder Stamm Apopka 97 (Accesion No. ATCC 20874), *Paecilomyces lilacinus,* insbesondere *P. lilacinus* Stamm 251 (AGAL 89/030550), *Talaromyces flavus,* insbesondere Stamm V117b, *Trichoderma atroviride,* insbesondere Stamm SC1 (Accession Number CBS 122089), *Trichoderma harzianum,* insbesondere *T. harzianum rifai T39.* (Accession Number CNCM I-952).

Beispiele für Viren, die als biologische Schädlingsbekämpfungsmittel eingesetzt werden bzw. verwendet werden können, sind:
*Adoxophyes orana* (Apfelschalenwickler) Granulosevirus (GV), *Cydia pomonella* (Apfelwickler) Granulosevirus (GV), *Helicoverpa armigera* (Baumwollkapselwurm) Nuklear Polyhedrosis Virus (NPV), *Spodoptera exigua* (Zuckerrübeneule) mNPV, *Spodoptera frugiperda* (Heerwurm) mNPV, *Spodoptera littoralis* (Afrikanischer Baumwollwurm) NPV.

Es sind auch Bakterien und Pilze umfasst, die als 'Inokulant' Pflanzen oder Pflanzenteilen oder Pflanzenorganen beigegeben werden und durch ihre besonderen Eigenschaften das Pflanzenwachstum und die Pflanzengesundheit fördern. Als Beispiele sind genannt:
*Agrobacterium spp., Azorhizobium caulinodans, Azospirillum spp., Azotobacter spp., Bradyrhizobium spp., Burkholderia spp.,* insbesondere *Burkholderia cepacia* (ehemals bekannt als *Pseudomonas cepacia*)*, Gigaspora spp.,* oder *Gigaspora monosporum, Glomus spp., Laccaria spp., Lactobacillus buchneri, Paraglomus spp., Pisolithus tinctorus, Pseudomonas spp., Rhizobium spp.,* insbesondere *Rhizobium trifolii, Rhizopogon spp., Scleroderma spp., Suillus spp., Streptomyces spp..*

Beispiele für Pflanzenextrakte und solche Produkte, die von Mikroorganismen gebildet wurden inklusive Proteine und sekundäre Stoffwechselprodukte, die als biologische Schädlingsbekämpfungsmittel eingesetzt werden bzw. verwendet werden können, sind:
Allium sativum, Artemisia absinthium, Azadirachtin, Biokeeper WP, Cassia nigricans, Celastrus angulatus, Chenopodium anthelminticum, Chitin, Armour-Zen, Dryopteris filix-mas, Equisetum arvense, Fortune Aza, Fungastop, Heads Up (Chenopodium quinoa-Saponinextrakt), Pyrethrum/Pyrethrine, Quassia amara, Quercus, Quillaja, Regalia, "Requiem™ Insecticide", Rotenon, Ryania/Ryanodine, Symphytum officinale, Tanacetum vulgare, Thymol, Triact 70, TriCon, Tropaeulum majus, Urtica dioica, Veratrin, Viscum album, Brassicacaeen-Extrakt, insbesondere Raps- oder Senfpulver.

### Safener als Mischungskomponenten

Die Verbindungen der Formel (I) können mit Safenern kombiniert werden, wie zum Beispiel Benoxacor, Cloquintocet (-mexyl), Cyometrinil, Cyprosulfamide, Dichlormid, Fenchlorazole (-ethyl), Fenclorim, Flurazole, Fluxofenim, Furilazole, Isoxadifen (-ethyl), Mefenpyr (-diethyl), Naphthalic anhydride, Oxabetrinil, 2-Methoxy-N-({4-[(methylcarbamoyl)amino]phenyl]sulfonyl)benzamid (CAS 129531-12-0), 4-(Dichloracetyl)-1-oxa-4-azaspiro[4.5]decan (CAS 71526-07-3), 2,2,5-Trimethyl-3-(dichloracetyl)-1,3-oxazolidin (CAS 52836-31-4).

### Pflanzen und Pflanzenteile

Erfindungsgemäß können alle Pflanzen und Pflanzenteile behandelt werden. Unter Pflanzen werden hierbei alle Pflanzen und Pflanzenpopulationen verstanden wie erwünschte und unerwünschte Wildpflanzen oder Kulturpflanzen (einschließlich natürlich vorkommender Kulturpflanzen), beispielsweise Getreide (Weizen, Reis, Triticale, Gerste, Roggen, Hafer), Mais, Soja, Kartoffel, Zuckerrüben, Zuckerrohr, Tomaten, Paprika, Gurke, Melone, Möhre, Wassermelone, Zwiebel, Salat, Spinat, Porree, Bohnen, *Brassica oleracea* (z. B. Kohl) und andere Gemüsesorten, Baumwolle, Tabak, Raps, sowie Obstpflanzen (mit den Früchten Äpfel, Birnen, Zitrusfrüchte und Weintrauben). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzen sollen alle Entwicklungsstadien wie Saatgut, Stecklinge, junge (unausgereifte) Pflanzen bis hin zu ausgereiften Pflanzen verstanden werden. Unter Pflanzenteilen sollen alle oberirdischen und unterirdischen Teile und Organe der Pflanzen wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehören auch geerntete Pflanzen oder geerntete Pflanzenteile sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Verbindungen der Formel (I) erfolgt direkt oder durch Einwirkung der Verbindungen auf die Umgebung, den Lebensraum oder den Lagerraum nach den üblichen Behandlungsmethoden, z. B. durch Eintauchen, Spritzen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden wie Kreuzung oder Protoplastenfusion erhaltene Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert. Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die durch konventionelle Züchtung, durch Mutagenese oder durch rekombinante DNA-Techniken erhalten worden sind. Dies können Sorten, Rassen, Bio- und Genotypen sein.

### Transgene Pflanze, Saatgutbehandlung und Integrationsereignisse

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehrfähigkeit der Pflanzen gegen tierische und mikrobielle Schädlinge, wie Insekten, Spinnentiere, Nematoden, Milben, Schnecken, bewirkt z. B. durch in den Pflanzen entstehende Toxine, insbesondere solche, die durch das genetische Material aus Bacillus Thuringiensis (z. B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden, ferner eine erhöhte Abwehrfähigkeit der Pflanzen gegen pflanzenpathogene Pilze, Bakterien und/oder Viren, bewirkt z. B. durch Systemisch Akquirierte Resistenz (SAR), Systemin, Phytoalexine, Elicitoren sowie Resistenzgene und entsprechend exprimierte Proteine und Toxine, sowie eine erhöhte Toleranz der Pflanzen gegen bestimmte herbizide Wirkstoffe, beispielsweise Imidazolinone, Sulfonylharnstoffe, Glyphosat oder Phosphinotricin (z. B. "PAT"-Gen). Die jeweils die gewünschten Eigenschaften ("Traits") verleihenden Gene können auch in Kombinationen miteinander in den transgenen Pflanzen vorkommen. Als Beispiele transgener Pflanzen werden die wichtigen Kulturpflanzen, wie Getreide (Weizen, Reis, Triticale, Gerste, Roggen, Hafer), Mais, Soja, Kartoffel, Zuckerrüben, Zuckerrohr, Tomaten, Erbsen und andere Gemüsesorten, Baumwolle, Tabak, Raps, sowie Obstpflanzen (mit den Früchten Äpfel, Birnen, Zitrusfrüchte und Weintrauben) erwähnt, wobei Mais, Soja, Weizen, Reis, Kartoffel, Baumwolle, Zuckerrohr, Tabak und Raps besonders hervorgehoben werden. Als Eigenschaften ("Traits") werden besonders hervorgehoben die erhöhte Abwehrfähigkeit der Pflanzen gegen Insekten, Spinnentiere, Nematoden und Schnecken.

### Pflanzenschutz - Behandlungsarten

Die Behandlung der Pflanzen und Pflanzenteile mit den Verbindungen der Formel (I) erfolgt direkt oder durch Einwirkung auf deren Umgebung, Lebensraum oder Lagerraum nach den üblichen Behandlungsmethoden, z. B. durch Tauchen, Spritzen, Sprühen, Berieseln, Verdampfen, Zerstäuben, Vernebeln, Verstreuen, Verschäumen, Bestreichen, Verstreichen, Injizieren, Gießen (drenchen), Tröpfchenbewässerung und bei Vermehrungsmaterial, insbesondere bei Saatgut, weiterhin durch Trockenbeizen, Nassbeizen, Schlämmbeizen, Inkrustieren, ein- oder mehrschichtiges Umhüllen, usw. Es ist ferner möglich, die Verbindungen der Formel (I) nach dem Ultra-Low-Volume-Verfahren auszubringen oder die Anwendungsform oder die Verbindung der Formel (I) selbst in den Boden zu injizieren.

Eine bevorzugte direkte Behandlung der Pflanzen ist die Blattapplikation, d. h. die Verbindungen der Formel (I) werden auf das Blattwerk aufgebracht, wobei die Behandlungsfrequenz und die Aufwandmenge auf den Befallsdruck des jeweiligen Schädlings abgestimmt sein sollte.

Bei systemisch wirksamen Wirkstoffen gelangen die Verbindungen der Formel (I) auch über das Wurzelwerk in die Pflanzen. Die Behandlung der Pflanzen erfolgt dann durch Einwirkung der Verbindungen der Formel (I) auf den Lebensraum der Pflanze. Das kann beispielsweise durch Drenchen, Einmischen in den Boden oder die Nährlösung sein, d. h. der Standort der Pflanze (z. B. Boden oder hydroponische Systeme) wird mit einer flüssigen Form der Verbindungen der Formel (I) getränkt, oder durch die Bodenapplikation, d. h. die erfindungsgemäßen Verbindungen der Formel (I) werden in fester Form (z. B. in Form eines Granulats) in den Standort der Pflanzen eingebracht. Bei Wasserreiskulturen kann das auch durch Zudosieren der Verbindung der Formel (I) in einer festen Anwendungsform (z. B. als Granulat) in ein überflutetes Reisfeld sein.

### Saatgutbehandlung

Die Bekämpfung von tierischen Schädlingen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufriedenstellend gelöst werden können. So ist es erstrebenswert, Verfahren zum Schutz des Saatguts und der keimenden Pflanze zu entwickeln, die das zusätzliche Ausbringen von Schädlingsbekämpfungsmitteln bei der Lagerung, nach der Saat oder nach dem Auflaufen der Pflanzen überflüssig machen oder zumindest deutlich verringern. Es ist weiterhin erstrebenswert, die Menge des eingesetzten Wirkstoffs dahingehend zu optimieren, dass das Saatgut und die keimende Pflanze vor dem Befall durch tierische Schädlinge bestmöglich geschützt werden, ohne jedoch die Pflanze selbst durch den eingesetzten Wirkstoff zu schädigen. Insbesondere sollten Verfahren zur Behandlung von Saatgut auch die intrinsischen insektiziden bzw. nematiziden Eigenschaften schädlingsresistenter bzw. -toleranter transgener Pflanzen einbeziehen, um einen optimalen Schutz des Saatguts und auch der keimenden Pflanze bei einem minimalen Aufwand an Schädlingsbekämpfungsmitteln zu erreichen.

Die vorliegende Erfindung bezieht sich daher insbesondere auch auf ein Verfahren zum Schutz von Saatgut und keimenden Pflanzen vor dem Befall von Schädlingen, indem das Saatgut mit einer der Verbindungen der Formel (I) behandelt wird. Das erfindungsgemäße Verfahren zum Schutz von Saatgut und keimenden Pflanzen vor dem Befall von Schädlingen umfasst ferner ein Verfahren, in dem das Saatgut gleichzeitig in einem Vorgang oder sequentiell mit einer Verbindung der Formel (I) und einer Mischungskomponente behandelt wird. Es umfasst ferner auch ein Verfahren, in dem das Saatgut zu unterschiedlichen Zeiten mit einer Verbindung der Formel (I) und einer Mischungskomponente behandelt wird.

Die Erfindung bezieht sich ebenfalls auf die Verwendung der Verbindungen der Formel (I) zur Behandlung von Saatgut zum Schutz des Saatguts und der daraus entstehenden Pflanze vor tierischen Schädlingen.

Weiterhin bezieht sich die Erfindung auf Saatgut, welches zum Schutz vor tierischen Schädlingen mit einer erfindungsgemäßen Verbindung der Formel (I) behandelt wurde. Die Erfindung bezieht sich auch auf Saatgut, welches zur gleichen Zeit mit einer Verbindung der Formel (I) und einer Mischungskomponente behandelt wurde. Die Erfindung bezieht sich weiterhin auf Saatgut, welches zu unterschiedlichen Zeiten mit einer Verbindung der Formel (I) und einer Mischungskomponente behandelt wurde. Bei Saatgut, welches zu unterschiedlichen Zeiten mit einer Verbindung der Formel (I) und einer Mischungskomponente behandelt wurde, können die einzelnen Substanzen in unterschiedlichen Schichten auf dem Saatgut vorhanden sein. Dabei können die Schichten, die eine Verbindung der Formel (I) und Mischungskomponenten enthalten, gegebenenfalls durch eine Zwischenschicht getrennt sein. Die Erfindung bezieht sich auch auf Saatgut, bei dem eine Verbindung der Formel (I) und eine Mischungskomponente als Bestandteil einer Umhüllung oder als weitere Schicht oder weitere Schichten zusätzlich zu einer Umhüllung aufgebracht sind.

Des Weiteren bezieht sich die Erfindung auf Saatgut, welches nach der Behandlung mit einer Verbindung der Formel (I) einem Filmcoating-Verfahren unterzogen wird, um Staubabrieb am Saatgut zu vermeiden.

Einer der auftretenden Vorteile, wenn eine Verbindung der Formel (I) systemisch wirkt, ist es, dass die Behandlung des Saatguts nicht nur das Saatgut selbst, sondern auch die daraus hervorgehenden Pflanzen nach dem Auflaufen vor tierischen Schädlingen schützt. Auf diese Weise kann die unmittelbare Behandlung der Kultur zum Zeitpunkt der Aussaat oder kurz danach entfallen.

Ein weiterer Vorteil ist darin zu sehen, dass durch die Behandlung des Saatguts mit einer Verbindung der Formel (I) Keimung und Auflauf des behandelten Saatguts gefördert werden können.

Ebenso ist es als vorteilhaft anzusehen, dass Verbindungen der Formel (I) insbesondere auch bei transgenem Saatgut eingesetzt werden können.

Verbindungen der Formel (I) können ferner in Kombination mit Mitteln der Signaltechnologie eingesetzt werden, wodurch eine bessere Besiedlung mit Symbionten, wie zum Beispiel Rhizobien, Mycorrhiza und/oder endophytischen Bakterien oder Pilzen, stattfindet und/oder es zu einer optimierten Stickstofffixierung kommt.

Die Verbindungen der Formel (I) eignen sich zum Schutz von Saatgut jeglicher Pflanzensorte, die in der Landwirtschaft, im Gewächshaus, in Forsten oder im Gartenbau eingesetzt wird. Insbesondere handelt es sich dabei um Saatgut von Getreide (z. B. Weizen, Gerste, Roggen, Hirse und Hafer), Mais, Baumwolle, Soja, Reis, Kartoffeln, Sonnenblume, Kaffee, Tabak, Canola, Raps, Rübe (z. B. Zuckerrübe und Futterrübe), Erdnuss, Gemüse (z. B. Tomate, Gurke, Bohne, Kohlgewächse, Zwiebeln und Salat), Obstpflanzen, Rasen und Zierpflanzen. Besondere Bedeutung kommt der Behandlung des Saatguts von Getreide (wie Weizen, Gerste, Roggen und Hafer), Mais, Soja, Baumwolle, Canola, Raps, Gemüse und Reis zu.

Wie vorstehend bereits erwähnt, kommt auch der Behandlung von transgenem Saatgut mit einer Verbindung der Formel (I) eine besondere Bedeutung zu. Dabei handelt es sich um das Saatgut von Pflanzen, die in der Regel zumindest ein heterologes Gen enthalten, das die Expression eines Polypeptids mit insbesondere insektiziden bzw. nematiziden Eigenschaften steuert. Die heterologen Gene in transgenem Saatgut können dabei aus Mikroorganismen wie Bacillus, Rhizobium, Pseudomonas, Serratia, Trichoderma, Clavibacter, Glomus oder Gliocladium stammen. Die vorliegende Erfindung eignet sich besonders für die Behandlung von transgenem Saatgut, das zumindest ein heterologes Gen enthält, das aus Bacillus sp. stammt. Besonders bevorzugt handelt es sich dabei um ein heterologes Gen, das aus Bacillus thuringiensis stammt.

Im Rahmen der vorliegenden Erfindung wird die Verbindung der Formel (I) auf das Saatgut aufgebracht. Vorzugsweise wird das Saatgut in einem Zustand behandelt, in dem es so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hüllen, Wolle oder Fruchtfleisch befreit wurde. So kann zum Beispiel Saatgut verwendet werden, das geerntet, gereinigt und bis zu einem lagerfähigen Feuchtigkeitsgehalt getrocknet wurde. Alternativ kann auch Saatgut verwendet werden, das nach dem Trocknen z. B. mit Wasser behandelt und dann erneut getrocknet wurde, zum Beispiel Priming. Im Fall von Reis-Saatgut ist es auch möglich, Saatgut zu verwenden, das getränkt wurde, zum Beispiel in Wasser bis zu einem bestimmten Stadium des Reisembryos ("Pigeon Breast Stage"), wodurch die Keimung und ein einheitlicheres Auflaufen stimuliert wird.

Im Allgemeinen muss bei der Behandlung des Saatguts darauf geachtet werden, dass die Menge der auf das Saatgut aufgebrachten Verbindung der Formel (I) und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Die Verbindungen der Formel (I) werden in der Regel in Form einer geeigneten Formulierung auf das Saatgut aufgebracht. Geeignete Formulierungen und Verfahren für die Saatgutbehandlung sind dem Fachmann bekannt.

Die Verbindungen der Formel (I) können in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen.

Diese Formulierungen werden in bekannter Weise hergestellt, indem man die Verbindungen der Formel (I) mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser.

Als Farbstoffe, die in den erfindungsgemäß verwendbaren Beizmittel-Formulierungen enthalten sein können, kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Solvent Red 1 bekannten Farbstoffe.

Als Netzmittel, die in den erfindungsgemäß verwendbaren Beizmittel-Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalinsulfonate, wie Diisopropyl- oder Diisobutylnaphthalinsulfonate.

Als Dispergiermittel und/oder Emulgatoren, die in den erfindungsgemäß verwendbaren Beizmittel-Formulierungen enthalten sein können, kommen alle zur Formulierung von agrochemischen Wirkstoffen üblichen nichtionischen, anionischen und kationischen Dispergiermittel in Betracht. Vor-zugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid-Blockpolymere, Alkylphenolpolyglykolether sowie Tristryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den erfindungsgemäß verwendbaren Beizmittel-Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfindungsgemäß verwendbaren Beizmittel-Formulierungen alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den erfindungsgemäß verwendbaren Beizmittel-Formulierungen enthalten sein können, kommen alle für derartige Zwecke in agrochemischen Mitteln einsetzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Beizmittel-Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose.

Als Gibberelline, die in den erfindungsgemäß verwendbaren Beizmittel-Formulierungen enthalten sein können, kommen vorzugsweise die Gibberelline A1, A3 (= Gibberellinsäure), A4 und A7 infrage, besonders bevorzugt verwendet man die Gibberellinsäure. Die Gibberelline sind bekannt (vgl. R. Wegler "Chemie der Pflanzenschutz- und Schädlingsbekämpfungsmittel", Bd. 2, Springer Verlag, 1970, S. 401-412).

Die erfindungsgemäß verwendbaren Beizmittel-Formulierungen können entweder direkt oder nach vorherigem Verdünnen mit Wasser zur Behandlung von Saatgut der verschiedensten Art eingesetzt werden. So lassen sich die Konzentrate oder die daraus durch Verdünnen mit Wasser erhältlichen Zubereitungen einsetzen zur Beizung des Saatgutes von Getreide, wie Weizen, Gerste, Roggen, Hafer und Triticale, sowie des Saatgutes von Mais, Reis, Raps, Erbsen, Bohnen, Baumwolle, Sonnenblumen, Soja und Rüben oder auch von Gemüsesaatgut der verschiedensten Natur. Die erfindungsgemäß verwendbaren Beizmittel-Formulierungen oder deren verdünnte Anwendungsformen können auch zum Beizen von Saatgut transgener Pflanzen eingesetzt werden.

Zur Behandlung von Saatgut mit den erfindungsgemäß verwendbaren Beizmittel-Formulierungen oder dem daraus durch Zugabe von Wasser hergestellten Anwendungsformen kommen alle üblicherweise für die Beizung einsetzbaren Mischgeräte in Betracht. Im Einzelnen geht man bei der Beizung so vor, dass man das Saatgut in einen Mischer im diskontinuierlichen oder kontinuierlichen Betrieb gibt, die jeweils gewünschte Menge an Beizmittel-Formulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

Die Aufwandmenge an den erfindungsgemäß verwendbaren Beizmittel-Formulierungen kann inner-halb eines größeren Bereiches variiert werden. Sie richtet sich nach dem jeweiligen Gehalt der Verbindungen der Formel (I) in den Formulierungen und nach dem Saatgut. Die Aufwandmengen bei der Verbindung der Formel (I) liegen im Allgemeinen zwischen 0,001 und 50 g pro Kilogramm Saatgut, vorzugsweise zwischen 0,01 und 15 g pro Kilogramm Saatgut.

### Tiergesundheit

Auf dem Gebiet der Tiergesundheit, d. h. dem Gebiet der Tiermedizin, sind die Verbindungen der Formel (I) gegen Tierparasiten, insbesondere Ektoparasiten oder Endoparasiten, wirksam. Der Begriff Endoparasit umfasst insbesondere Helminthen und Protozoen wie Kokzidien. Ektoparasiten sind typischerweise und bevorzugt Arthropoden, insbesondere Insekten oder Akariden.

Auf dem Gebiet der Tiermedizin eignen sich die Verbindungen der Formel (I), die eine günstige Toxizität gegenüber Warmblütern aufweisen, für die Bekämpfung von Parasiten, die in der Tierzucht und Tierhaltung bei Nutztieren, Zuchttieren, Zootieren, Laboratoriumstieren, Versuchstieren und Haustieren auftreten. Sie sind gegen alle oder einzelne Entwicklungsstadien der Parasiten wirksam.

Zu den landwirtschaftlichen Nutztieren zählen zum Beispiel Säugetiere wie Schafe, Ziegen, Pferde, Esel, Kamele, Büffel, Kaninchen, Rentiere, Damhirsche und insbesondere Rinder und Schweine; oder Geflügel wie Truthähne, Enten, Gänse und insbesondere Hühner; oder Fische oder Krustentiere, z. B. in der Aquakultur, oder gegebenenfalls Insekten wie Bienen.

Zu den Haustieren zählen zum Beispiel Säugetiere wie Hamster, Meerschweinchen, Ratten, Mäuse, Chinchillas, Frettchen und insbesondere Hunde, Katzen, Stubenvögel; Reptilien, Amphibien oder Aquariumfische.

Gemäß einer bestimmten Ausführungsform werden die Verbindungen der Formel (I) an Säugetiere verabreicht.

Gemäß einer weiteren bestimmten Ausführungsform werden die Verbindungen der Formel (I) an Vögel, nämlich Stubenvögel oder insbesondere Geflügel, verabreicht.

Durch Verwendung der Verbindungen der Formel (I) für die Bekämpfung von Tierparasiten sollen Krankheit, Todesfälle und Leistungsminderungen (bei Fleisch, Milch, Wolle, Häuten, Eiern, Honig und dergleichen) verringert bzw. vorgebeugt werden, so dass eine wirtschaftlichere und einfachere Tierhaltung ermöglicht wird und ein besseres Wohlbefinden der Tiere erzielbar ist.

In Bezug auf das Gebiet der Tiergesundheit bedeutet der Begriff "Bekämpfung" oder "bekämpfen" im vorliegenden Zusammenhang, dass durch die Verbindungen der Formel (I) wirksam das Auftreten des jeweiligen Parasiten in einem Tier, das mit solchen Parasiten in einem harmlosen Ausmaß infiziert ist, reduziert wird. Genauer gesagt bedeutet "bekämpfen" im vorliegenden Zusammenhang, dass die Verbindungen der Formel (I) den jeweiligen Parasiten abtöten, sein Wachstum verhindern oder seine Vermehrung verhindern.

Zu den Arthropoden zählen beispielsweise, ohne hierauf beschränkt zu sein,
aus der Ordnung Anoplurida zum Beispiel Haematopinus spp., Linognathus spp., Pediculus spp., Phtirus spp., Solenopotes spp.;
aus der Ordnung Mallophagida und den Unterordnungen Amblycerina und Ischnocerina, zum Beispiel Bovicola spp., Damalina spp., Felicola spp.; Lepikentron spp., Menopon spp., Trichodectes spp., Trimenopon spp., Trinoton spp., Werneckiella spp;
aus der Ordnung Diptera und den Unterordnungen Nematocerina und Brachycerina, zum Beispiel Aedes spp., Anopheles spp., Atylotus spp., Braula spp., Calliphora spp., Chrysomyia spp., Chrysops spp., Culex spp., Culicoides spp., Eusimulium spp., Fannia spp., Gasterophilus spp., Glossina spp., Haematobia spp., Haematopota spp., Hippobosca spp., Hybomitra spp., Hydrotaea spp., Hypoderma spp., Lipoptena spp., Lucilia spp., Lutzomyia spp., Melophagus spp., Morellia spp., Musca spp., Odagmia spp., Oestrus spp., Philipomyia spp., Phlebotomus spp., Rhinoestrus spp., Sarcophaga spp., Simulium spp., Stomoxys spp., Tabanus spp., Tipula spp., Wilhelmia spp., Wohlfahrtia spp.;
aus der Ordnung Siphonapterida, zum Beispiel Ceratophyllus spp., Ctenocephalides spp., Pulex spp., Tunga spp., Xenopsylla spp.;
aus der Ordnung Heteropterida, zum Beispiel Cimex spp., Panstrongylus spp., Rhodnius spp., Triatoma spp.; sowie Lästlinge und Hygieneschädlinge aus der Ordnung Blattarida.

Weiterhin sind bei den Arthropoden beispielhaft, ohne hierauf beschränkt zu sein, die folgenden Akari zu nennen:
Aus der Unterklasse Akari (Acarina) und der Ordnung Metastigmata, zum Beispiel aus der Familie Argasidae, wie Argas spp., Ornithodorus spp., Otobius spp., aus der Familie Ixodidae, wie Amblyomma spp., Dermacentor spp., Haemaphysalis spp., Hyalomma spp., Ixodes spp., Rhipicephalus (Boophilus) spp., Rhipicephalus spp. (die ursprüngliche Gattung der mehrwirtigen Zecken); aus der Ordnung Mesostigmata, wie Dermanyssus spp., Ornithonyssus spp., Pneumonyssus spp., Raillietia spp., Sternostoma spp., Tropilaelaps spp., Varroa spp.; aus der Ordnung Actinedida (Prostigmata), zum Beispiel Acarapis spp., Cheyletiella spp., Demodex spp., Listrophorus spp., Myobia spp., Neotrombicula spp., Ornithocheyletia spp., Psorergates spp., Trombicula spp.; und aus der Ordung der Acaridida (Astigmata), zum Beispiel Acarus spp., Caloglyphus spp., Chorioptes spp., Cytodites spp., Hypodectes spp., Knemidocoptes spp., Laminosioptes spp., Notoedres spp., Otodectes spp., Psoroptes spp., Pterolichus spp., Sarcoptes spp., Trixacarus spp., Tyrophagus spp.

Zu Beispielen für parasitäre Protozoen zählen, ohne hierauf beschränkt zu sein:
Mastigophora (Flagellata), wie:
   Metamonada: aus der Ordnung Diplomonadida zum Beispiel Giardia spp., Spironucleus spp.
   Parabasala: aus der Ordnung Trichomonadida zum Beispiel Histomonas spp., Pentatrichomonas spp., Tetratrichomonas spp., Trichomonas spp., Tritrichomonas spp.
   Euglenozoa: aus der Ordnung Trypanosomatida zum Beispiel Leishmania spp., Trypanosoma spp.

Sarcomastigophora (Rhizopoda), wie Entamoebidae, zum Beispiel Entamoeba spp., Centramoebidae, zum Beispiel Acanthamoeba sp., Euamoebidae, z. B. Hartmanella sp.

Alveolata wie Apicomplexa (Sporozoa): z. B. Cryptosporidium spp.; aus der Ordnung Eimeriida zum Beispiel Besnoitia spp., Cystoisospora spp., Eimeria spp., Hammondia spp., Isospora spp., Neospora spp., Sarcocystis spp., Toxoplasma spp.; aus der Ordnung Adeleida z. B. Hepatozoon spp., Klossiella spp.; aus der Ordnung Haemosporida z. B. Leucocytozoon spp., Plasmodium spp.; aus der Ordnung Piroplasmida z. B. Babesia spp., Ciliophora spp., Echinozoon spp., Theileria spp.; aus der Ordnung Vesibuliferida z. B. Balantidium spp., Buxtonella spp.

Microspora wie Encephalitozoon spp., Enterocytozoon spp., Globidium spp., Nosema spp., und außerdem z. B. Myxozoa spp.

Zu den für Menschen oder Tiere pathogenen Helminthen zählen zum Beispiel Acanthocephala, Nematoden, Pentastoma und Platyhelminthen (z.B. Monogenea, Cestodes und Trematodes).

Zu beispielhaften Helminthen zählen, ohne hierauf beschränkt zu sein:
Monogenea: z. B.: Dactylogyrus spp., Gyrodactylus spp., Microbothrium spp., Polystoma spp., Troglecephalus spp.;
Cestodes: aus der Ordnung Pseudophyllidea zum Beispiel: Bothridium spp., Diphyllobothrium spp., Diplogonoporus spp. Ichthyobothrium spp., Ligula spp., Schistocephalus spp., Spirometra spp.

Aus der Ordnung Cyclophyllida zum Beispiel: Andyra spp., Anoplocephala spp., Avitellina spp., Bertiella spp., Cittotaenia spp., Davainea spp., Diorchis spp., Diplopylidium spp., Dipylidium spp., Echinococcus spp., Echinocotyle spp., Echinolepis spp., Hydatigera spp., Hymenolepis spp., Joyeuxiella spp., Mesocestoides spp., Moniezia spp., Paranoplocephala spp., Raillietina spp., Stilesia spp., Taenia spp., Thysaniezia spp., Thysanosoma spp.

Trematodes: aus der Klasse Digenea zum Beispiel: Austrobilharzia spp., Brachylaima spp., Calicophoron spp., Catatropis spp., Clonorchis spp. Collyriclum spp., Cotylophoron spp., Cyclocoelum spp., Dicrocoelium spp., Diplostomum spp., Echinochasmus spp., Echinoparyphium spp., Echinostoma spp., Eurytrema spp., Fasciola spp., Fasciolides spp., Fasciolopsis spp., Fischoederius spp., Gastrothylacus spp., Gigantobilharzia spp., Gigantocotyle spp., Heterophyes spp., Hypoderaeum spp., Leucochloridium spp., Metagonimus spp., Metorchis spp., Nanophyetus spp., Notocotylus spp., Opisthorchis spp., Ornithobilharzia spp., Paragonimus spp., Paramphistomum spp., Plagiorchis spp., Posthodiplostomum spp., Prosthogonimus spp., Schistosoma spp., Trichobilharzia spp., Troglotrema spp., Typhlocoelum spp.

Nematoden: aus der Ordnung Trichinellida zum Beispiel: Capillaria spp., Trichinella spp., Trichomosoides spp., Trichuris spp.

Aus der Ordnung Tylenchida zum Beispiel: Micronema spp., Parastrangyloides spp., Strongyloides spp.

Aus der Ordnung Rhabditina zum Beispiel: Aelurostrongylus spp., Amidostomum spp., Ancylostoma spp., Angiostrongylus spp., Bronchonema spp., Bunostomum spp., Chabertia spp., Cooperia spp., Cooperioides spp., Crenosoma spp., Cyathostomum spp., Cyclococercus spp., Cyclodontostomum spp., Cylicocyclus spp., Cylicostephanus spp., Cylindropharynx spp., Cystocaulus spp., Dictyocaulus spp., Elaphostrongylus spp., Filaroides spp., Globocephalus spp., Graphidium spp., Gyalocephalus spp., Haemonchus spp., Heligmosomoides spp., Hyostrongylus spp., Marshallagia spp., Metastrongylus spp., Muellerius spp., Necator spp., Nematodirus spp., Neostrongylus spp., Nippostrongylus spp., Obeliscoides spp., Oesophagodontus spp., Oesophagostomum spp., Ollulanus spp.; Ornithostrongylus spp., Oslerus spp., Ostertagia spp., Paracooperia spp., Paracrenosoma spp., Parafilaroides spp., Parelaphostrongylus spp., Pneumocaulus spp., Pneumostrongylus spp., Poteriostomum spp., Protostrongylus spp., Spicocaulus spp., Stephanurus spp., Strongylus spp., Syngamus spp., Teladorsagia spp., Trichonema spp., Trichostrongylus spp., Triodontophorus spp., Troglostrongylus spp., Uncinaria spp.

Aus der Ordnung Spirurida zum Beispiel: Acanthocheilonema spp., Anisakis spp., Ascaridia spp.; Ascaris spp., Ascarops spp., Aspiculuris spp., Baylisascaris spp., Brugia spp., Cercopithifilaria spp., Crassicauda spp., Dipetalonema spp., Dirofilaria spp., Dracunculus spp.; Draschia spp., Enterobius spp., Filaria spp., Gnathostoma spp., Gongylonema spp., Habronema spp., Heterakis spp.; Litomosoides spp., Loa spp., Onchocerca spp., Oxyuris spp., Parabronema spp., Parafilaria spp., Parascaris spp., Passalurus spp., Physaloptera spp., Probstmayria spp., Pseudofilaria spp., Setaria spp., Skjrabinema spp., Spirocerca spp., Stephanofilaria spp., Strongyluris spp., Syphacia spp., Thelazia spp., Toxascaris spp., Toxocara spp., Wuchereria spp.

Acanthocephala: aus der Ordnung Oligacanthorhynchida z.B: Macracanthorhynchus spp., Prosthenorchis spp.; aus der Ordnung Moniliformida zum Beispiel: Moniliformis spp.,
Aus der Ordnung Polymorphida zum Beispiel: Filicollis spp.; aus der Ordnung Echinorhynchida zum Beispiel Acanthocephalus spp., Echinorhynchus spp., Leptorhynchoides spp.

Pentastoma: aus der Ordnung Porocephalida zum Beispiel Linguatula spp.

Auf dem Gebiet der Tiermedizin und der Tierhaltung erfolgt die Verabreichung der Verbindungen der Formel (I) nach allgemein fachbekannten Verfahren, wie enteral, parenteral, dermal oder nasal in Form von geeigneten Präparaten. Die Verabreichung kann prophylaktisch; metaphylaktisch oder therapeutisch erfolgen.

So bezieht sich eine Ausführungsform der vorliegenden Erfindung auf die Verbindungen der Formel (I) zur Verwendung als Arzneimittel.

Ein weiterer Aspekt bezieht sich auf die Verbindungen der Formel (I) zur Verwendung als Antiendoparasitikum.

Ein weiterer spezieller Aspekt der Erfindung betrifft die Verbindungen der Formel (I) zur Verwendung als Antihelminthikum, insbesondere zur Verwendung als Nematizid, Platymelminthizid, Acanthocephalizid oder Pentastomizid.

Ein weiterer spezieller Aspekt der Erfindung betrifft die Verbindungen der Formel (I) zur Verwendung als Antiprotozoikum.

Ein weiterer Aspekt betrifft die Verbindungen der Formel (I) zur Verwendung als Antiektoparasitikum, insbesondere ein Arthropodizid, ganz besonders ein Insektizid oder ein Akarizid.

Weitere Aspekte der Erfindung sind veterinärmedizinische Formulierungen, die eine wirksame Menge mindestens einer Verbindung der Formel (I) und mindestens einen der folgenden umfassen: einen pharmazeutisch unbedenklichen Exzipienten (z.B. feste oder flüssige Verdünnungsmittel), ein pharmazeutisch unbedenkliches Hilfsmittel (z.B. Tenside), insbesondere einen herkömmlicherweise in veterinärmedizinischen Formulierungen verwendeten pharmazeutisch unbedenklichen Exzipienten und/oder ein herkömmlicherweise in veterinärmedizinischen Formulierungen verwendetes pharmazeutisch unbedenkliches Hilfsmittel.

Ein verwandter Aspekt der Erfindung ist ein Verfahren zur Herstellung einer wie hier beschriebenen veterinärmedizinischen Formulierung, welches den Schritt des Mischens mindestens einer Verbindung der Formel (I) mit pharmazeutisch unbedenklichen Exzipienten und/oder Hilfsmitteln, insbesondere mit herkömmlicherweise in veterinärmedizinischen Formulierungen verwendeten pharmazeutisch unbedenklichen Exzipienten und/oder herkömmlicherweise in veterinärmedizinischen Formulierungen verwendeten Hilfsmitteln umfasst.

Ein anderer spezieller Aspekt der Erfindung sind veterinärmedizinische Formulierungen ausgewählt aus der Gruppe ektoparasitizider und endoparasitizider Formulierungen, insbesondere ausgewählt aus der Gruppe anthelmintischer, antiprotozolischer und arthropodizider Formulierungen, ganz besonders ausgewählt aus der Gruppe nematizider, platyhelminthizider, acanthocephalizider, pentastomizider, insektizider und akkarizider Formulierungen, gemäß den erwähnten Aspekten, sowie Verfahren zu ihrer Herstellung.

Ein anderer Aspekt bezieht sich auf ein Verfahren zur Behandlung einer parasitischen Infektion, insbesondere einer Infektion durch einen Parasiten ausgewählt aus der Gruppe der hier erwähnten Ektoparasiten und Endoparasiten, durch Anwendung einer wirksamen Menge einer Verbindung der Formel (I) bei einem Tier, insbesondere einem nichthumanen Tier, das dessen bedarf.

Ein anderer Aspekt bezieht sich auf ein Verfahren zur Behandlung einer parasitischen Infektion, insbesondere einer Infektion durch einen Parasiten ausgewählt aus der Gruppe der hier erwähnten Ektoparasiten und Endoparasiten, durch Anwendung einer wie hier definierten veterinärmedizinischen Formulierung bei einem Tier, insbesondere einem nichthumanen Tier, das dessen bedarf.

Ein anderer Aspekt bezieht sich auf die Verwendung der Verbindungen der Formel (I) bei der Behandlung einer Parasiteninfektion, insbesondere einer Infektion durch einen Parasiten ausgewählt aus der Gruppe der hier erwähnten Ektoparasiten und Endoparasiten, bei einem Tier, insbesondere einem nichthumanen Tier.

Im vorliegenden tiergesundheitlichen oder veterinärmedizinischen Zusammenhang schließt der Begriff "Behandlung" die prophylaktische, die metaphylaktische und die therapeutische Behandlung ein.

Bei einer bestimmten Ausführungsform werden hiermit Mischungen mindestens einer Verbindung der Formel (I) mit anderen Wirkstoffen, insbesondere mit Endo- und Ektoparasitiziden, für das veterinärmedizinische Gebiet bereitgestellt.

Auf dem Gebiet der Tiergesundheit bedeutet "Mischung" nicht nur, dass zwei (oder mehr) verschiedene Wirkstoffe in einer gemeinsamen Formulierung formuliert werden und entsprechend zusammen angewendet werden, sondern bezieht sich auch auf Produkte, die für jeden Wirkstoff getrennte Formulierungen umfassen. Dementsprechend können, wenn mehr als zwei Wirkstoffe angewendet werden sollen, alle Wirkstoffe in einer gemeinsamen Formulierung formuliert werden oder alle Wirkstoffe in getrennten Formulierungen formuliert werden; ebenfalls denkbar sind gemischte Formen, bei denen einige der Wirkstoffe gemeinsam formuliert und einige der Wirkstoffe getrennt formuliert sind. Getrennte Formulierungen erlauben die getrennte oder aufeinanderfolgende Anwendung der in Rede stehenden Wirkstoffe.

Die hier mit ihrem "Common Name" spezifizierten Wirkstoffe sind bekannt und beispielsweise im "Pesticide Manual" (siehe oben) beschrieben oder im Internet recherchierbar (z.B. http://www.alanwood.net/pesticides).

Beispielhafte Wirkstoffe aus der Gruppe der Ektoparasitizide als Mischungspartner schließen, ohne dass dies eine Einschränkung darstellen soll, die oben ausführlich aufgelisteten Insektizide und Akkarizide ein. Weitere verwendbare Wirkstoffe sind unten gemäß der oben erwähnten Klassifikation, die auf dem aktuellen IRAC Mode of Action Classification Scheme beruht, aufgeführt: (1) Acetylcholinesterase (AChE)-Inhibitoren; (2) GABA-gesteuerte Chlorid-Kanal-Blocker; (3) Natrium-Kanal-Modulatoren; (4) kompetitive Modulatoren des nicotinischen Acetylcholin-Rezeptors (nAChR); (5) allosterische Modulatoren des nicotinischen Acetylcholin-Rezeptors (nAChR); (6) allosterische Modulatoren des Glutamat-abhängigen Chloridkanals (GluCl); (7) Juvenilhormon-Mimetika; (8) verschiedene nichtspezifische (Multi-Site) Inhibitoren; (9) Modulatoren Chordotonaler Organe; (10) Milbenwachstumsinhibitoren; (12) Inhibitoren der mitochondrialen ATP-Synthase, wie ATP-Disruptoren; (13) Entkoppler der oxidativen Phosphorylierung durch Störung des Protonengradienten; (14) Blocker des nicotinischen Acetylcholinrezeptorkanals; (15) Inhibitoren der Chitinbiosynthese, Typ 0; (16) Inhibitoren der Chitinbiosynthese, Typ 1; (17) Häutungsdisruptor (insbesondere bei Dipteren, d.h. Zweiflüglern); (18) Ecdyson-Rezeptor-Agonisten; (19) Octopamin-Rezeptor-Agonisten; (21) mitochondriale Komplex-I-Elektronentransportinhibitoren; (25) mitochondriale Komplex-II-Elektronentransportinhibitoren; (20) mitochondriale Komplex-III-Elektronentransportinhibitoren; (22) Blocker des spannungsabhängigen Natriumkanals; (23) Inhibitoren der Acetyl-CoA-Carboxylase; (28) Ryanodinrezeptor-Modulatoren;

Wirkstoffe mit unbekannten oder nicht spezifischen Wirkmechanismen, z. B. Fentrifanil, Fenoxacrim, Cyclopren, Chlorobenzilat, Chlordimeform, Flubenzimin, Dicyclanil, Amidoflumet, Quinomethionat, Triarathen, Clothiazoben, Tetrasul, Kaliumoleat, Petroleum, Metoxadiazon, Gossyplur, Flutenzin, Brompropylat, Cryolit;
Verbindungen aus anderen Klassen, z.B. Butacarb, Dimetilan, Cloethocarb, Phosphocarb, Pirimiphos(-ethyl), Parathion(-ethyl), Methacrifos, Isopropyl-o-salicylat, Trichlorfon, Tigolaner, Sulprofos, Propaphos, Sebufos, Pyridathion, Prothoat, Dichlofenthion, Demeton-S-methylsulfon, Isazofos, Cyanofenphos, Dialifos, Carbophenothion, Autathiofos, Aromfenvinfos(-methyl), Azinphos(-ethyl), Chlorpyrifos(-ethyl), Fosmethilan, Iodofenphos, Dioxabenzofos, Formothion, Fonofos, Flupyrazofos, Fensulfothion, Etrimfos;
Organochlorverbindungen, z. B. Camphechlor, Lindan, Heptachlor; oder Phenylpyrazole, z. B. Acetoprol, Pyrafluprol, Pyriprol, Vaniliprol, Sisapronil; oder Isoxazoline, z. B. Sarolaner, Afoxolaner, Lotilaner, Fluralaner;
Pyrethroide, z. B. (cis-, trans-)Metofluthrin, Profluthrin, Flufenprox, Flubrocythrinat, Fubfenprox, Fenfluthrin, Protrifenbut, Pyresmethrin, RU15525, Terallethrin, cis-Resmethrin, Heptafluthrin, Bioethanomethrin, Biopermethrin, Fenpyrithrin, cis-Cypermethrin, cis-Permethrin, Clocythrin, Cyhalothrin (lambda-), Chlovaporthrin, oder halogenierte Kohlenwasserstoffverbindungen (HCHs),
Neonicotinoide, z. B. Nithiazin
Dicloromezotiaz, Triflumezopyrim makrocyclische Lactone, z. B. Nemadectin, Ivermectin, Latidectin, Moxidectin, Selamectin, Eprinomectin, Doramectin, Emamectinbenzoat; Milbemycinoxim
Tripren, Epofenonan, Diofenolan;
Biologicals, Hormone oder Pheromone, zum Beispiel natürliche Produkte, z.B. Thuringiensin, Codlemon oder Neem-Komponenten
Dinitrophenole, z. B. Dinocap, Dinobuton, Binapacryl;
Benzoylharnstoffe, z. B. Fluazuron, Penfluron,
Amidinderivate, z. B. Chlormebuform, Cymiazol, Demiditraz
Bienenstockvarroa-Akarizide, zum Beispiel organische Säuren, z.B. Ameisensäure, Oxalsäure.

Zu beispielhaften Wirkstoffen aus der Gruppe der Endoparasitizide, als Mischungspartner, zählen, ohne hierauf beschränkt zu sein, anthelmintische Wirkstoffe und antiprotozoische Wirkstoffe.

Zu den anthelmintischen Wirkstoffen zählen, ohne hierauf beschränkt zu sein, die folgenden nematiziden, trematiziden und/oder cestoziden Wirkstoffe:
aus der Klasse der makrocyclischen Lactone zum Beispiel: Eprinomectin, Abamectin, Nemadectin, Moxidectin, Doramectin, Selamectin, Lepimectin, Latidectin, Milbemectin, Ivermectin, Emamectin, Milbemycin;
aus der Klasse der Benzimidazole und Probenzimidazole zum Beispiel: Oxibendazol, Mebendazol, Triclabendazol, Thiophanat, Parbendazol, Oxfendazol, Netobimin, Fenbendazol, Febantel, Thiabendazol, Cyclobendazol, Cambendazol, Albendazol-sulfoxid, Albendazol, Flubendazol;
aus der Klasse der Depsipeptide, vorzugsweise cyclischen Depsipetide, insbesondere 24-gliedrigen cyclischen Depsipeptide, zum Beispiel: Emodepsid, PF1022A;
aus der Klasse der Tetrahydropyrimidine zum Beispiel: Morantel, Pyrantel, Oxantel;
aus der Klasse der Imidazothiazole zum Beispiel: Butamisol, Levamisol, Tetramisol;
aus der Klasse der Aminophenylamidine zum Beispiel: Amidantel, deacyliertes Amidantel (dAMD), Tribendimidin;
aus der Klasse der Aminoacetonitrile zum Beispiel: Monepantel;
aus der Klasse der Paraherquamide zum Beispiel: Paraherquamid, Derquantel;
aus der Klasse der Salicylanilide zum Beispiel: Tribromsalan, Bromoxanid, Brotianid, Clioxanid, Closantel, Niclosamid, Oxyclozanid, Rafoxanid;
aus der Klasse der substituierten Phenole zum Beispiel: Nitroxynil, Bithionol, Disophenol, Hexachlorophen, Niclofolan, Meniclopholan;
aus der Klasse der Organophosphate zum Beispiel: Trichlorfon, Naphthalofos, Dichlorvos/DDVP, Crufomat, Coumaphos, Haloxon;
aus der Klasse der Piperazinone/Chinoline zum Beispiel: Praziquantel, Epsiprantel;
aus der Klasse der Piperazine zum Beispiel: Piperazin, Hydroxyzin;
aus der Klasse der Tetracycline zum Beispiel: Tetracyclin, Chlorotetracyclin, Doxycyclin, Oxytetracyclin, Rolitetracyclin;
aus diversen anderen Klassen zum Beispiel: Bunamidin, Niridazol, Resorantel, Omphalotin, Oltipraz, Nitroscanat, Nitroxynil, Oxamniquin, Mirasan, Miracil, Lucanthon, Hycanthon, Hetolin, Emetin, Diethylcarbamazin, Dichlorophen, Diamfenetid, Clonazepam, Bephenium, Amoscanat, Clorsulon.

Antiprotozoische Wirkstoffe, darunter, ohne hierauf beschränkt zu sein, die folgenden Wirkstoffe:
aus der Klasse der Triazine zum Beispiel: Diclazuril, Ponazuril, Letrazuril, Toltrazuril;
aus der Klasse Polyletherionophor zum Beispiel: Monensin, Salinomycin, Maduramicin, Narasin;
aus der Klasse der makrocyclischen Lactone zum Beispiel: Milbemycin, Erythromycin;
aus der Klasse der Chinolone zum Beispiel: Enrofloxacin, Pradofloxacin;
aus der Klasse der Chinine zum Beispiel: Chloroquin;
aus der Klasse der Pyrimidine zum Beispiel: Pyrimethamin;
aus der Klasse der Sulfonamide zum Beispiel: Sulfachinoxalin, Trimethoprim, Sulfaclozin;
aus der Klasse der Thiamine zum Beispiel: Amprolium;
aus der Klasse der Lincosamide zum Beispiel: Clindamycin;
aus der Klasse der Carbanilide zum Beispiel: Imidocarb;
aus der Klasse der Nitrofurane zum Beispiel: Nifurtimox;
aus der Klasse der Chinazolinonalkaloide zum Beispiel: Halofuginon;
aus diversen anderen Klassen zum Beispiel: Oxamniquin, Paromomycin;
aus der Klasse der Vakzine oder Antigene aus Mikroorganismen zum Beispiel: Babesia canis rossi, Eimeria tenella, Eimeria praecox, Eimeria necatrix, Eimeria mitis, Eimeria maxima, Eimeria brunetti, Eimeria acervulina, Babesia canis vogeli, Leishmania infantum, Babesia canis canis, Dictyocaulus viviparus.

Alle genannten Mischungspartner können außerdem, wenn sie auf Grund ihrer funktionellen Gruppen dazu imstande sind, gegebenenfalls mit geeigneten Basen oder Säuren Salze bilden.

### Vektorbekämpfung

Die Verbindungen der Formel (I) können auch in der Vektorbekämpfung eingesetzt werden. Ein Vektor im Sinne der vorliegenden Erfindung ist ein Arthropode, insbesondere ein Insekt oder Arachnide, der in der Lage ist, Krankheitserreger wie z. B. Viren, Würmer, Einzeller und Bakterien aus einem Reservoir (Pflanze, Tier, Mensch, etc.) auf einen Wirt zu übertragen. Die Krankheitserreger können entweder mechanisch (z. B. Trachoma durch nicht-stechende Fliegen) auf einem Wirt, oder nach Injektion (z. B. Malaria-Parasiten durch Mücken) in einen Wirt übertragen werden.

Beispiele für Vektoren und die von ihnen übertragenen Krankheiten bzw. Krankheitserreger sind:
1) Mücken
   - Anopheles: Malaria, Filariose;
   - Culex: Japanische Encephalitis, Filariasis, weitere virale Erkrankungen, Übertragung von anderen Würmern;
   - Aedes: Gelbfieber, Dengue-Fieber, weitere virale Erkrankungen, Filariasis;
   - Simulien: Übertragung von Würmern, insbesondere Onchocerca volvulus;
   - Psychodidae: Übertragung von Leishmaniose
2) Läuse: Hautinfektionen, epidemisches Fleckfieber;
3) Flöhe: Pest, endemisches Fleckfieber, Bandwürmer;
4) Fliegen: Schlafkrankheit (Trypanosomiasis); Cholera, weitere bakterielle Erkrankungen;
5) Milben: Acariose, epidemisches Fleckfieber, Rickettsipocken, Tularämie, Saint-Louis-Enzephalitis, Frühsommer-Meningoenzephalitis (FSME), Krim-Kongo-Fieber, Borreliose;
6) Zecken: Borelliosen wie Borrelia bungdorferi sensu lato., Borrelia duttoni, Frühsommer-Meningoenzephalitis, Q-Fieber (Coxiella burnetii), Babesien (Babesia canis canis), Ehrlichiose.

Beispiele für Vektoren im Sinne der vorliegenden Erfindung sind Insekten, zum Beispiel Aphiden, Fliegen, Zikaden oder Thripse, die Pflanzenviren auf Pflanzen übertragen können. Weitere Vektoren, die Pflanzenviren übertragen können, sind Spinnmilben, Läuse, Käfer und Nematoden.

Weitere Beispiele für Vektoren im Sinne der vorliegenden Erfindung sind Insekten und Arachniden wie Mücken, insbesondere der Gattungen Aedes, Anopheles, z. B. A. gambiae, A. arabiensis, A. funestus, A. dirus (Malaria) und Culex, Psychodide wie Phlebotomus, Lutzomyia, Läuse, Flöhe, Fliegen, Milben und Zecken, die Krankheitserreger auf Tiere und/oder Menschen übertragen können.

Eine Vektorbekämpfung ist auch möglich, wenn die Verbindungen der Formel (I) Resistenz-brechend sind.

Verbindungen der Formel (I) sind zur Verwendung in der Prävention von Krankheiten und/oder Krankheitserregern, die durch Vektoren übertragen werden, geeignet. Somit ist ein weiterer Aspekt der vorliegenden Erfindung die Verwendung von Verbindungen der Formel (I) zur Vektorbekämpfung, z. B. in der Landwirtschaft, im Gartenbau, in Forsten, in Gärten und Freizeiteinrichtungen sowie im Vorrats- und Materialschutz.

### Schutz von technischen Materialen

Die Verbindungen der Formel (I) eignen sich zum Schutz von technischen Materialien gegen Befall oder Zerstörung durch Insekten, z. B. aus den Ordnungen Coleoptera, Hymenoptera, Isoptera, Lepidoptera, Psocoptera und Zygentoma.

Unter technischen Materialien sind im vorliegenden Zusammenhang nicht lebende Materialien zu verstehen, wie vorzugsweise Kunststoffe, Klebstoffe, Leime, Papiere und Kartone, Leder, Holz, Holzverarbeitungsprodukte und Anstrichmittel. Die Anwendung der Erfindung zum Schutz von Holz ist besonders bevorzugt.

In einer weiteren Ausführungsform werden die Verbindungen der Formel (I) zusammen mit mindestens einem weiteren Insektizid und/oder mindestens einem Fungizid eingesetzt.

In einer weiteren Ausführungsform liegen die Verbindungen der Formel (I) als ein anwendungsfertiges (ready-to-use) Schädlingsbekämpfungsmittel vor, d. h., sie können ohne weitere Änderungen auf das entsprechende Material aufgebracht werden. Als weitere Insektizide oder Fungizide kommen insbesondere die oben genannten in Frage.

Überraschenderweise wurde auch gefunden, dass die Verbindungen der Formel (I) zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, verwendet werden können. Gleichfalls können die Verbindungen der Formel (I) allein oder in Kombinationen mit anderen Wirkstoffen als Antifouling-Mittel eingesetzt werden.

### Bekämpfung von tierischen Schädlingen auf dem Hygienesektor

Die Verbindungen der Formel (I) eignen sich zur Bekämpfung von tierischen Schädlingen auf dem Hygienesektor. Insbesondere kann die Erfindung im Haushalts-, Hygiene- und Vorratsschutz verwendet werden, vor allem zur Bekämpfung von Insekten, Spinnentieren, Zecken und Milben, die in geschlossenen Räumen, wie beispielsweise Wohnungen, Fabrikhallen, Büros, Fahrzeugkabinen, Tierzuchtanlagen vorkommen. Zur Bekämpfung der tierischen Schädlinge werden die Verbindungen der Formel (I) allein oder in Kombination mit anderen Wirk- und/oder Hilfsstoffen verwendet. Bevorzugt werden sie in Haushaltsinsektizid-Produkten verwendet. Die Verbindungen der Formel (I) sind gegen sensible und resistente Arten sowie gegen alle Entwicklungsstadien wirksam.

Zu diesen Schädlingen gehören beispielsweise Schädlinge aus der Klasse Arachnida, aus den Ordnungen Scorpiones, Araneae und Opiliones, aus den Klassen Chilopoda und Diplopoda, aus der Klasse Insecta die Ordnung Blattodea, aus den Ordnungen Coleoptera, Dermaptera, Diptera, Heteroptera, Hymenoptera, Isoptera, Lepidoptera, Phthiraptera, Psocoptera, Saltatoria oder Orthoptera, Siphonaptera und Zygentoma und aus der Klasse Malacostraca die Ordnung Isopoda.

Die Anwendung erfolgt beispielsweise in Aerosolen, drucklosen Sprühmitteln, z. B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

### Erläuterung der Verfahren und Zwischenprodukte:

Die erfindungsgemäßen Verbindungen der Formel (I) können durch die in den folgenden Schemata dargestellten Verfahren erhalten werden:

### Verfahren A:

Die in Verfahren **A** angegebenen erfindungsgemäßen Verbindungen der Formel (I-a) können durch die Abfolge literaturbekannter Methoden gemäß folgendem Schema hergestellt werden.

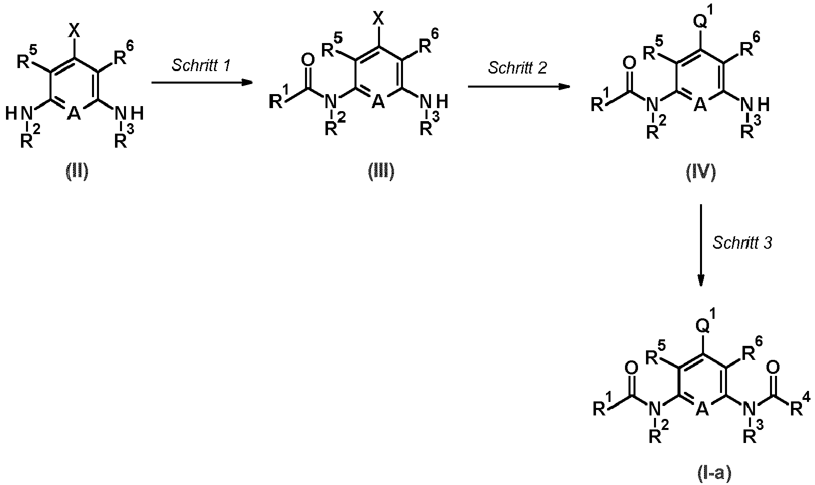

Die Reste A, R¹, R², R³, R⁴, R⁵, R⁶ und Q¹ haben die oben beschriebenen Bedeutungen. X steht für Brom oder Iod.

### Schritt 1:

Die Umsetzung von Verbindungen der Formel (II) zu Verbindungen der Formel (III) gelingt nach literaturbekannten Verfahren, wie z. B. durch Reaktion von (II) mit einem Säurechlorid (R¹COCl) in Anwesenheit geeigneter Basen, beispielsweise Pyridin, Triethylamin oder Kaliumcarbonat, in einem geeigneten inerten Lösungsmittel, wie z. B. Dichlormethan oder 2-Butanon. Analoge Verfahren wurden z. B. beschrieben in EP1714966, US5403816 oder WO2004/35545.

Alternativ lassen sich Verbindungen der Formel (III) auch durch Umsetzung von (II) mit Carbonsäuren (R¹COOH) in Anwesenheit geeigneter Kupplungsreagenzien, z. B. HATU (*O*-(7-Azabenzotriazol-1-yl)-*N*,*N*,*N*',*N*'-tetramethyluronium-hexafluorphosphat]) oder EDCI (1-Ethyl-3-(3-dimethylaminopropyl)carbodiimid), und geeigneter Basen, z. B. Triethylamin oder N,N-Diisopropylethylamin, in geeigneten inerten Lösungsmitteln wie z. B. DMF (Dimethylformamid) oder Dichlormethan, erhalten (vergleiche in Analogie z. B. US2011/301181 oder WO2007/122258).

Verbindungen der Formel (II) sind kommerziell erhältlich oder lassen sich durch literaturbekannte Verfahren (vergleiche in Analogie z.B. Journal of Materials Chemistry B, 2013, vol. 1, # 46 p. 6359 - 6364, US2012/22092 A1, Bioorganic and Medicinal Chemistry Letters, 2011 , vol. 21, # 10 p. 3113 - 3116, Bioorganic and Medicinal Chemistry Letters, 2011 , vol. 21, # 11 p. 3237 - 3242, Journal of the Chemical Society, 1952 , p. 4443, Acta Chemica Scandinavica, Series B: Organic Chemistry and Biochemistry, 1984 , vol. 38, # 10 p. 877 - 884) herstellen.

Die Carbonsäuren sind entweder kommerziell erhältlich oder können nach bekannten Methoden hergestellt werden.

### Schritt 2:

Halogenierte Derivate der Formel **(III)** lassen sich mit Aryl- und Hetaryl-boronsäuren oder Aryl- und Hetarylboronestern nach bekannten Methoden zu Verbindungen der Formel **(IV)** kuppeln. Diese Umsetzung kann in einem geeigneten Lösungsmittel, z. B. Dimethylformamid, oder einem geeigneten Lösungsmittelgemisch, wie z. B. Dimethylformamid/Wasser, 1,4-Dioxan/Wasser oder Isopropanol/Toluol/Wasser, durch Palladium-Katalyse, z.B. mit Tetrakis(triphenylphosphin)-palladium(0) [vergleiche z. B. US2015/210671], Tris-(dibenzylideneaceton)-dipalladium(0) [vergleiche z. B. WO2013/61081] oder (1,1'-Bis(diphenyl-phosphino)-ferrocene)-palladium(II)-dichlorid [vergleiche z. B. US2011/9410] als Palladium-Quelle und in Anwesenheit einer geeigneten Base, z. B. Kaliumcarbonat oder Natriumcarbonat, erfolgen.

Die benötigten Boronsäurederivate bzw. Boronsäureesterderivate sind teilweise bekannt und/oder kommerziell erhältlich bzw. können nach allgemein bekannten Methoden hergestellt werden (vgl. Boronic Acids (Eds.: D. G. Hall), 2nd ed., Wiley-VCH, Weinheim, 2011).

Eine Kupplung der halogenierten Derivate der Formel **(III)** mit NH-haltigen Heteroaromaten, wie z. B. Pyrrolen, Imidazolen oder Pyrazolen, kann z. B. in Analogie zu US2013/165464 oder WO2012/64815 unter Inertgasatmosphäre durch Katalyse mit Kupfer(I)salzen, beispielsweise Kupfer(I)iodid, in Anwesenheit eines geeigneten Liganden, z. B. *(trans)*-N,N'-Dimethylcyclohexan-1,2-diamin oder R-(+)-Prolin, und einer geeigneten Base, z. B. Kaliumcarbonat, in einem geeigneten Lösungsmittel, wie z. B. 1,4-Dioxan oder Dimethylsulfoxid, erfolgen, wodurch Verbindungen der Formel **(IV)** erhalten werden.

### Schritt 3:

Die Herstellung der Verbindungen der Formel **(I-a)** gelingt in Analogie zu den unter Verfahren **A,** Schritt 1, beschriebenen Methoden durch Umsetzung von **(IV)** mit einem geeigneten Säurechlorid (R⁴COCl) in Anwesenheit geeigneter Basen, z. B. Pyridin, Triethylamin oder Kaliumcarbonat, in einem geeigneten inerten Lösungsmittel, wie z. B. Dichlormethan.

Alternativ lassen sich Verbindungen der Formel **(I-a)** auch durch Umsetzung von **(IV)** mit Carbonsäuren (R⁴COOH) in Anwesenheit geeigneter Kupplungsreagenzien, z. B. HATU oder EDCI, und geeigneter Basen, z. B. Triethylamin oder N,N-Diisopropylethylamin, in geeigneten inerten Lösungsmitteln wie z. B. DMF oder Dichlormethan, erhalten (vergleiche in Analogie z. B. US2011/301181 oder WO2007/122258).

Die Carbonsäuren sind entweder kommerziell erhältlich oder können nach bekannten Methoden hergestellt werden.

Um Verbindungen der Formel **(I-a)** aus Verbindungen der Formel **(II)** zu erhalten, ist die Sequenz aus den Schritten 1, 2 und 3 auch in der Reihenfolge austauschbar.

### Verfahren B:

Die in Verfahren **B** angegebenen erfindungsgemäßen Verbindungen der Formel **(I-a),** in denen R1 und R4 gleich sind, können durch die Abfolge literaturbekannter Methoden gemäß folgendem Schema hergestellt werden.

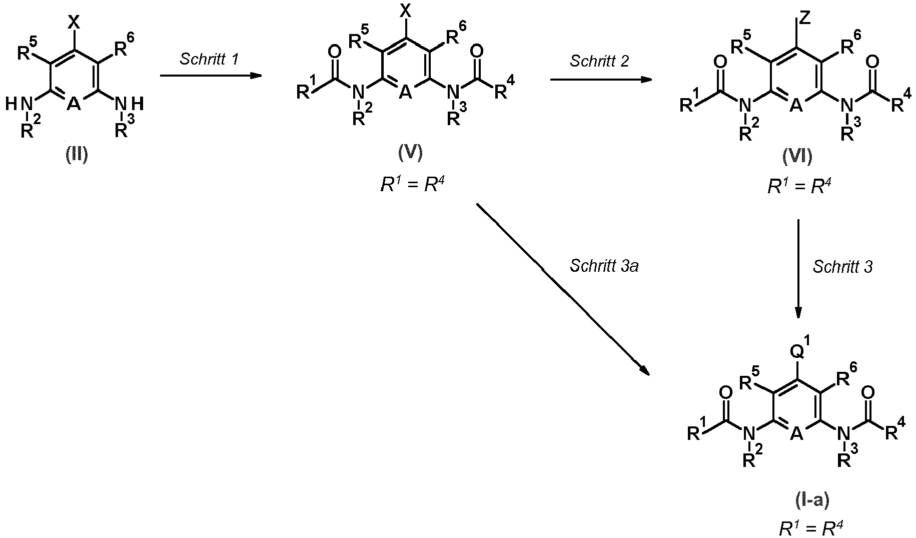

Die Reste A, R¹, R², R³, R⁴, R⁵, R⁶ und Q¹ haben die oben beschriebenen Bedeutungen. X steht für Brom oder Iod.

Z steht für

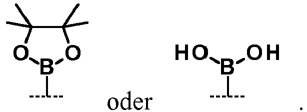

### Schritt 1:

Die Herstellung der Verbindungen der Formel **(V)** gelingt in Analogie zu den unter Verfahren A, Schritt 1, beschriebenen Methoden durch Umsetzung von **(II)** mit mindestens zwei Äquivalenten eines geeigneten Säurechlorids in Anwesenheit geeigneter Basen, z. B. Pyridin, Triethylamin oder Kaliumcarbonat, in einem geeigneten inerten Lösungsmittel, wie z. B. Dichlormethan.

Alternativ lassen sich Verbindungen der Formel **(V)** auch durch Umsetzung von **(II)** mit mindestens zwei Äquivalenten einer geeigneten Carbonsäure in Anwesenheit geeigneter Kupplungsreagenzien, z. B. HATU oder EDCI, und geeigneter Basen, z. B. Triethylamin oder N,N-Diisopropylethylamin, in geeigneten inerten Lösungsmitteln wie z. B. DMF oder Dichlormethan, erhalten (vergleiche in Analogie z. B. US2011/301181 oder WO2007/122258).

### Schritt 2:

Zum Erhalt der entsprechenden Boronsäuren oder Dioxoborolan-Derivate **(VI)** aus den halogenierten Verbindungen **(V)** können ebenfalls literaturbekannte Methoden angewendet werden. So lassen sich z. B. 4-Brom-pyridin-2-carboxamide durch Umsetzung mit Bis(pinacolato)diboron [4,4,4',4',5,5,5',5'-Octamethyl-2,2'-bis-1,3,2-dioxaborolan] in Anwesenheit von Kaliumacetat und geeigneten Katalysatoren, wie z. B. Dichlor(1,1'-bis(diphenylphosphanyl)ferrocen)-palladium(II) [ggf. als Dichlormethan-Addukt] oder Dichlor-bis(triphenylphosphin)-palladium(II), in geeigneten inerten Lösungsmitteln, z. B. Dimethylformamid, Dioxan oder Toluol und unter Inertatmosphäre in die entsprechenden Boronsäure- oder Dioxoborolan-Verbindungen überführen (vergleiche in Analogie z. B. US2004/67982, US2007/197553 oder WO2011/159554).

### Schritt 3:

Verbindungen der Formel **(VI)** lassen sich mit Aryl- oder Hetaryl-halogeniden, insbesondere Chloriden, Bromiden oder Iodiden, oder mit Aryl- oder Hetaryl-triflaten, nach bekannten Methoden zu Verbindungen der Formel **(I-a)** kuppeln. Diese Umsetzung kann in einem geeigneten Lösungsmittel, z. B. Dimethylformamid, oder einem geeigneten Lösungsmittelgemisch, wie z. B. Dimethylformamid/Wasser, 1,4-Dioxan/Wasser oder Isopropanol/Toluol/Wasser, durch Palladium-Katalyse, z.B. mit Tetrakis(triphenylphosphin)-palladium(0) [vergleiche z. B. WO2014/170821], Tris-(dibenzylideneaceton)-dipalladium(0) [vergleiche z. B. WO2013/61081] oder (1,1'-Bis(diphenyl-phosphino)-ferrocene)-palladium(II)-dichlorid [vergleiche z. B. US2011/9410] als Palladium-Quelle und in Anwesenheit einer geeigneten Base, z. B. Kaliumcarbonat oder Natriumcarbonat, erfolgen.

Die benötigten Aryl- oder Hetaryl-halogenide sind entweder kommerziell erhältlich oder können nach bekannten Methoden hergestellt werden (vgl. Synthese z.B. analog 4-Brom-2-fluor-1-(pentafluorethyl)benzol in WO2012010573).

### Schritt 3a:

Eine Kupplung der halogenierten Derivate der Formel **(V)** mit NH-haltigen Heteroaromaten, wie z. B. Pyrrole, Imidazole oder Pyrazole, kann z. B. in Analogie zu US2013/165464 oder WO2012/64815 unter Inertgasatmosphäre durch Katalyse mit Kupfer(I)salzen, beispielsweise Kupfer(I)iodid, in Anwesenheit eines geeigneten Liganden, z. B. *(trans)*-N,N'-Dimethylcyclohexan-1,2-diamin oder *R*-(+)-Prolin, und einer geeigneten Base, z. B. Kaliumcarbonat, in einem geeigneten Lösungsmittel, wie z. B. 1,4-Dioxan oder Dimethylsulfoxid, erfolgen, wodurch Verbindungen der Formel **(I-a)** erhalten werden.

Um Verbindungen der Formel **(I-a)** aus Verbindungen der Formel **(II)** zu erhalten, ist die Sequenz aus den Schritten 1 und 3a in der Reihenfolge austauschbar sowie die Sequenz aus den Schritten 1 → 2 → 3 auch in der Reihenfolge 2 → 1 → 3 und 2 → 3 → 1 durchführbar.

### Verfahren C

Die in Verfahren C angegebenen erfindungsgemäßen Verbindungen der Formel **(I-a),** in denen R² oder R³ für Alkyl steht und der jeweils andere für Wasserstoff steht oder in denen beide (R² und R³) für Alkyl stehen, können durch literaturbekannte Methoden gemäß folgendem Schema hergestellt werden

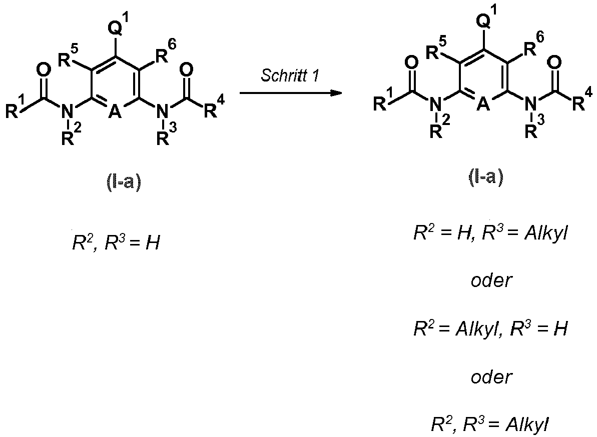

Die Reste A, R¹, R⁴, R⁵, R⁶, V¹, V² und Q¹ haben die oben beschriebenen Bedeutungen. R² und R³ haben die im Schema angegebenen Bedeutungen.

### Schritt 1:

Zum Erhalt der entsprechende *N*-Alkyl-Derivate der Verbindungen **(I-a)** können literaturbekannte Methoden angewendet werden. So lassen sich Carboxamide durch Umsetzung mit Halogenalkanen, z.B. Iodmethan in Anwesenheit einer Base, z.B. Natriumhydrid in geeigneten inerten Lösungsmitteln, wie z.B. *N,N*-Dimethylformamid in die entsprechenden Alkyl-Derivate der Caboxamide überführen (vergleiche in Analogie z.B. US4039543 A1).

### Verfahren D

Die in Verfahren D angegebenen erfindungsgemäßen Verbindungen der Formel (I), in denen V¹ oder V² oder beide (V¹ und V²) für Schwefel stehen, können durch literaturbekannte Methoden gemäß folgendem Schema hergestellt werden.

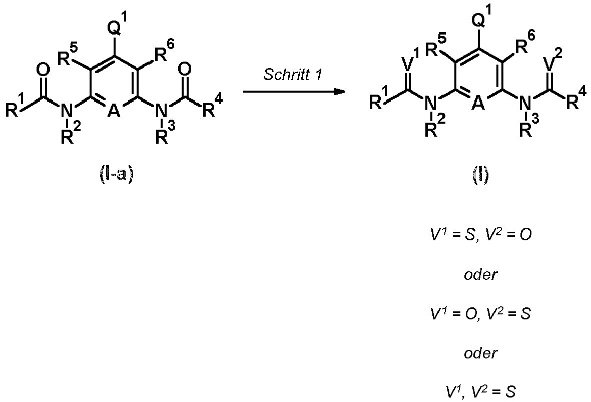

Die Reste A, R¹, R², R³, R⁴, R⁵, R⁶ und Q¹ haben die oben beschriebenen Bedeutungen. V¹ und V² haben die im Schema angegebenen Bedeutungen.

### Schritt 1:

Thioamide der Formel **(I),** in denen V¹ oder V² oder beide (V¹ und V²) für Schwefel stehen, lassen sich aus Amiden der Formel **(I-a)** durch Umsetzung mit einem geeigneten Schwefelungsreagenz, beispielsweise Lawessons Reagenz (vergleiche in Analogie z. B. WO2005/9435) oder P₄S₁₀ (vergleiche in Analogie z. B. European Journal of Medicinal Chemistry 1995 30, 915 - 924), in geeigneten Lösungsmitteln wie z.B. Toluol oder Xylol, herstellen.

### Verfahren E

Die in Verfahren E angegebenen erfindungsgemäßen Verbindungen der Formel **(I-a),** in denen R² oder R³ für C(O)OAlkyl steht und der jeweils andere für Wasserstoff steht oder in denen beide (R² und R³) für C(O)OAlkyl stehen, können durch literaturbekannte Methoden gemäß folgendem Schema hergestellt werden

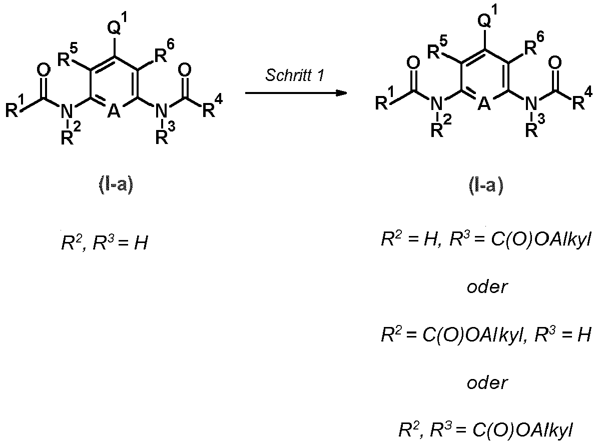

Die Reste A, R¹, R⁴, R⁵, R⁶ und Q¹ haben die oben beschriebenen Bedeutungen. R² und R³ haben die im Schema angegebenen Bedeutungen.

### Schritt 1

Carbamate der Formel **(I-a),** in denen R² oder R³ für C(O)OAlkyl steht und der jeweils andere für H steht oder in denen beide (R² und R³) für C(O)OAlkyl stehen, lassen sich aus Amiden der Formel **(I-a),** in denen R² und R³ für Wasserstoff stehen, durch Umsetzung mit Chlorameisensäureestern und einer Base, beispielsweise Natriumhydrid (vergleiche in Analogie z. B. EP1932836), in geeigneten Lösungsmitteln wie z.B. DMF oder Tetrahydrofuran, herstellen.

### Herstellungsbeispiele

### N-(6-Amino-4-brompyridin-2-yl)cyclopropancarboxamid

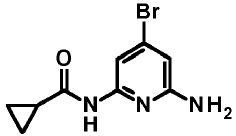

500 mg (2,65 mmol) 4-Brom-2,6-diaminopyridin wurde in 50 ml Dichlormethan vorgelegt und mit 1,39 ml (7,97 mmol) *N,N*-Diisopropylethylamin versetzt. Anschließend wurde unter Kühlung bei 0 °C 278 mg (2,65mmol) Cyclopropancarbonsäurechlorid zugetropft. Es wurde über Nacht bei Raumtemperatur nachgerührt und anschließend das Reaktionsgemsich mit Wasser gewaschen. Die organische Phase wurde über Magnesiumsulfat getrocknet und unter vermindertem Druck vom Lösungsmittel befreit. Der Rückstand wurde durch säulenchromatographische Aufreinigung mit einem Cyclohexan / Aceton Gradienten als Laufmittel gereinigt. Es wurden 310 mg (44% d.Th.) *N*-(6-Amino-4-brompyridin-2-yl)cyclopropancarboxamid erhalten.

logP (neutral): 1,61; MH⁺: 258; ¹H-NMR(600 MHz, D₆-DMSO) δ ppm: 0,76-0,77 (m, 4H), 1,94-2,01 (m, 1H), 6,07 (s, 2H), 6,36 (d, 1H), 7,43 (d, 1H), 10,40 (s, 1H)

### N-{6-Amino-4-[4-(trifluormethoxy)phenyl]pyridin-2-yl}cyclopropancarboxamid

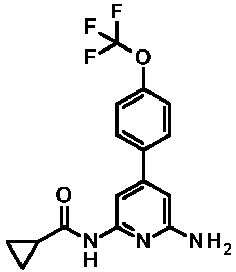

2 g (7,8 mmol) *N*-(6-Amino-4-brompyridin-2-yl)cyclopropancarboxamid, 1,61 g (7,8 mmol) [4-(Trifluormethoxy)phenyl]borsäure und 270 mg (0,23 mmol) Tetrakis-(triphenylphosphin)-palladium wurden unter Schutzgas vorgelegt. Es wurde 40 ml entgastes Dioxan und 15,6 ml entgaste wässrige Natriumcarbonatlösung (2M) zugegeben und über Nacht bei 96 °C nachgerührt. Nach dem Abkühlen wurde das Lösungsmittel unter Vakuum abdestilliert. Der Rückstand wurde mit Dichlormethan versetzt, mit wenig Wasser gewaschen und die organische Phase über Magnesiumsulfat getrocknet. Das Lösungsmittel wurde unter Vakuum abdestilliert. Der Rückstand wurde durch säulenchromatographische Aufreinigung mit einem Cyclohexan / Ethylacetat Gradienten als Laufmittel gereinigt. Es wurden 2,59 g (98% d.Th.) *N*-{6-Amino-4-[4-(trifluormethoxy)phenyl]pyridin-2-yl}cyclopropancarboxamid erhalten.

logP (neutral): 2,79; MH⁺: 338; ¹H-NMR(600 MHz, D₆-DMSO) δ ppm: 0,73-0,81 (m, 4H), 1,98-2,05 (m, 1H), 5,91 (s, 2H), 6,42 (d, 1H), 7,70-7,46 (m, 5H), 10,33 (s, 1H)

### N-16-[(Cyclopropylcarbonyl)amino]-4-[4-(trifluormethoxy)phenyl]pyridin-2-yl}-1-fluorcyclopropancarboxamid (1-12)

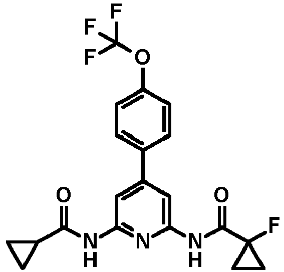

55,5 mg (0,53 mmol) 1-Fluorcyclopropancarbonsäure und 65,2 mg (0,53 mmol) 4 Dimethylaminopyridin wurden in 7 ml Dioxan vorgelegt, mit 102 mg (0,53 mmol) *N*-[3-(Dimethylamino)propyl]-*N'*-ethylcarbodiimidhydrochlorid (1:1) versetzt und 1 h bei Raumtemperatur nachgerührt. Anschließend wurde 150 mg (0,44 mmol) *N*-{6-Amino-4-[4-(trifluormethoxy)phenyl]pyridin-2-yl}cyclopropancarboxamid hinzugegeben und über Nacht bei Raumtemperatur nachgerührt. Das Lösungsmittel wurde unter Vakuum abdestilliert und der Rückstand gegen Dichlormethan extrahiert. Die organische Phase wurde über Magnesiumsulfat getrocknet und unter vermindertem Druck vom Lösungsmittel befreit. Der Rückstand wurde durch säulenchromatographische Aufreinigung mit einem Cyclohexan / Ethylacetat Gradienten als Laufmittel gereinigt. Es wurden 135 mg (72% d.Th.) *N*-{6-[(Cyclopropylcarbonyl)amino]-4-[4-(trifluormethoxy)phenyl]pyridin-2-yl}-1-fluorcyclopropancarboxamid erhalten.

logP (neutral): 3,88; MH⁺: 424; ¹H-NMR(600 MHz, D₆-DMSO) δ ppm: 0,88-0,81 (m, 4H), 1,42-1,34 (m, 2H), 1,53-1,45 (m, 2H), 2,10-2,04 (m, 1H), 7,81-7,51 (m, 4H), 7,87 (d, 1H), 8,18 (d, 1H), 9,82 (s, 1H), 10,81 (s, 1H)

### N,N-(4-Brompyridin-2,6-diyl)dicyclopropancarboxamid

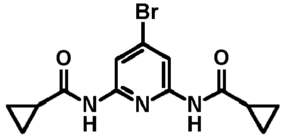

4,5 g (23,9 mmol) 4-Brom-2,6-diaminopyridin wurde in 450 ml Dichlormethan vorgelegt und mit 12,5 ml (71,7 mmol) *N,N*-Diisopropylethylamin versetzt. Anschließend wurde unter Kühlung bei 0 °C 2,5 g (39,9 mmol) Cyclopropancarbonsäurechlorid zugetropft. Es wurde über Nacht bei Raumtemperatur nachgerührt und anschließend das Reaktionsgemsich mit Wasser gewaschen. Die organische Phase wurde über Magnesiumsulfat getrocknet und unter vermindertem Druck vom Lösungsmittel befreit. Der Rückstand wurde durch säulenchromatographische Aufreinigung mit einem Cyclohexan / Aceton Gradienten als Laufmittel gereinigt. Es wurden 768 mg (10% d.Th.) *N,N'*-(4-Brompyridin-2,6-diyl)dicyclopropancarboxamid erhalten.

logP (neutral): 2,35; MH⁺: 326; ¹H-NMR(600 MHz, D₆-DMSO) δ ppm: 0,83 (d, 8H), 2,10-2,04 (m,2 H), 7,97 (s, 2H), 10,60 (s, 2H)

### N,N'-[4-(4,4,5,5-Tetramethyl-1,3,2-dioxaborolan-2-yl)pyridin-2,6-diyl]dicyclopropancarboxamid

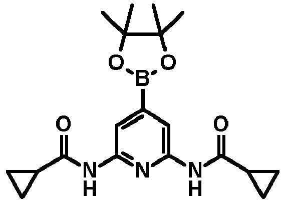

1,5 g (4,62 mmol) *N,N'*-(4-Brompyridin-2,6-diyl)dicyclopropancarboxamid, 1,23 g (4,85 mmol) 4,4,4',4',5,5,5',5'-octamethyl-2,2'-bi-1,3,2-dioxaborolan, 1,36 g (13,8 mmol) Kaliumacetat, 113 mg (0,13 mmol) [1,1'-Bis(diphenylphosphino)ferrocen]palladium(II)chlorid und 77 mg (0,13 mmol) 1,1'-Bis(diphenylphosphino)ferrocen wurden unter Schutzgas unter Schutzgas vorgelegt. Es wurden 9 ml entgastes Dioxan hinzugegeben und über Nacht bei 96 °C nachgerührt. Das Reaktionsgemisch wurde unter vermindertem Druck vom Lösungsmittel befreit, der Rückstand mit Wasser versetzt und gegen Dichlormethan extrahiert. Die organische Phase wurde über Magnesiumsulfat getrocknet und unter vermindertem Druck vom Lösungsmittel befreit. Es wurden 3,03 g (160% d.Th.) Rohprodukt erhalten, das ohne weitere Reinigung im Folgeschritt umgesetzt wurde.

logP (neutral): 0,89; MH⁺: 290; ¹H-NMR(600 MHz, D₆-DMSO) δ ppm: 0,82-0,80 (m, 8H), 1,29 (s, 12H), 2,10-2,02 (m, 2H), 7,98 (d, 2H), 10,35 (s, 2H)

### N,N'-{4-[3-Fluor-4-(pentafluorethyl)phenyl]pyridin-2,6-diyl}dicyclopropancarboxamid (1-35)

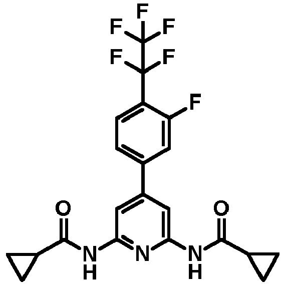

500 mg (1,70 mmol) 4-Brom-2-fluor-1-(pentafluorethyl)benzol, 643 mg (1,70 mmol) *N,N'*-[4-(4,4,5,5-Tetramethyl-1,3,2-dioxaborolan-2-yl)pyridin-2,6-diyl]dicyclopropancarboxamid und 58,9 mg (0,05 mmol) Tetrakis-(triphenylphosphin)-palladium wurden unter Schutzgasatmosphäre vorgelegt, 15 ml entgastes Dioxan und 6,81 ml entgaste wässrige Natriumcarbonatlösung (1M) wurden hinzugefügt. Es wurde über Nacht bei 92°C nachgerührt. Nach dem Abkühlen wurde das Lösungsmittel unter vermindertem Druck abdestilliert. Der Rückstand wurde mit Dichlormethan aufgenommen und mit wenig Wasser gewaschen. Die organische Phase wurde über Magnesiumsulfat getrocknet und unter vermindertem Druck vom Lösungsmittel befreit. Der Rückstand wurde durch säulenchromatographische Aufreinigung mit einem Cyclohexan / Aceton Gradienten als Laufmittel gereinigt. Es wurden 386 mg (50% d.Th.) *N,N'*-{4-[3-Fluor-4-(pentafluorethyl)phenyl]pyridin-2,6-diyl}dicyclopropancarboxamid erhalten.

logP (neutral): 4,06; MH⁺: 458; ¹H-NMR(600 MHz, D₆-DMSO) δ ppm: 0,85-0,83 (m, 8H), 2,14-2,08 (m,2 H), 7,76-7,69 (m, 2H), 7,89 (t, 1H), 8,02 (s, 2H), 10,59 (s, 2H)

### Dimethyl-{4-[4-(trifluormethoxy)phenyl]pyridin-2,6-diyl}bis[(cyclopropylcarbonyl)carbamat] (I-37)

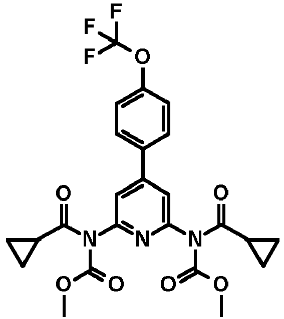

150 mg (0,37 mmol) *N,N'*-{4-[4-(Trifluormethoxy)phenyl]pyridin-2,6-diyl}dicyclopropancarboxamid wurde unter Schutzgasatmosphäre in 4 ml *N,N*-Dimethylformamid vorgelegt, unter Kühlung wurde bei einer Temperatur von 0 °C 29,6 mg (0,74 mmol, 60%ig) Natriumhydrid hinzugegeben und 1 h bei Raumtemperatur nachgerührt. Anschließend wurde 52,5 mg (0,55 mmol) Methylcarbonochloridat hinzudosiert und über 60 h bei Raumtemperatur nachgerührt. Die Reaktion wurde mit Wasser beendet und dreimal gegen Ethylacetat extrahiert. Die organische Phase wurde mit gesättigter wässriger Natriumchlorid Lösung gewaschen, über Magnesiumsulfat getrocknet und unter vermindertem Druck vom Lösungsmittel befreit. Der Rückstand wurde durch säulenchromatographische Aufreinigung mit einem Wasser / Acetonitril Gradienten als Laufmittel und anschließend durch säulenchromatographische Aufreinigung mit einem Cyclohexan / Ethylacetat Gradienten als Laufmittel gereinigt. Es wurden 50,1 mg (17% d.Th.) Dimethyl-{4-[4-(trifluormethoxy)phenyl]pyridin-2,6-diyl}bis[(cyclopropylcarbonyl)carbamat] erhalten.

logP (neutral): 3,73; MH⁺: 522; ¹H-NMR(600 MHz, D₆-DMSO) δ ppm: 1,02-0,95 (m, 8H), 2,61-2,57 (m,2 H), 3,72 (s, 6H), 8,01-7,52 (m, 6H)

### N,N'-{4-[4-(Trifluormethoxy)phenyl]pyridin-2,6-diyl}bis(N-ethylcyclopropancarboxamid) (1-29)

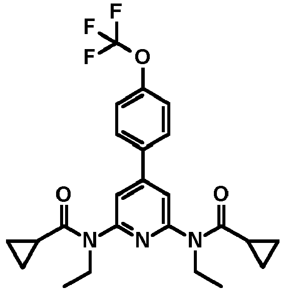

150 mg (0,37 mmol) *N,N'*-{4-[4-(Trifluormethoxy)phenyl]pyridin-2,6-diyl}dicyclopropancarboxamid wurde in *N,N*-Dimethylformamid vorgelegt, unter Kühlung wurde bei einer Temperatur von 0 °C 31 mg (0,77 mmol, 60%ig) Natriumhydrid hinzugegeben und 15 Minuten nachgerührt. Bei Raumtemperatur wurde 115 mg (0,74 mmol) Iodethan hinzudosiert und es wurde 2 h nachgerührt. Das Reaktionsgemisch wurde mit Wasser versetzt und gegen Dichlormethan extrahiert. Die organische Phase wurde mit gesättigter wässriger Natriumchlorid Lösung gewaschen, über Magnesiumsulfat getrocknet und unter vermindertem Druck vom Lösungsmittel befreit. Es wurden 170 mg (100% d.Th.) *N,N'*-{4-[4-(Trifluormethoxy)phenyl]pyridin-2,6-diyl}bis(N-ethylcyclopropancarboxamid) erhalten.

logP (neutral): 4,05; MH⁺: 462; ¹H-NMR(600 MHz, D₆-DMSO) δ ppm: 0,76-0,75 (m, 4H), 0,92-0,84 (m, 4H), 1,14 (t, 6H), 1,72-1,66 (m, 2H), 3,92 (q, 4H), 8,00-7,53 (m, 6H)

### N-{6-[(Cyclopropylcarbonyl)amino]-4-[4-(trifluormethoxy)phenyl]pyridin-2-yl}-N-methylcyclopropancarboxamid (I-31)

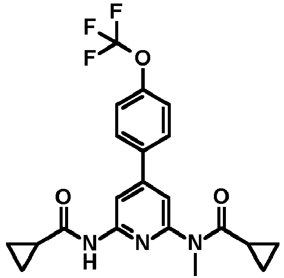

150 mg (0,37 mmol) *N,N'*-{4-[4-(Trifluormethoxy)phenyl]pyridin-2,6-diyl}dicyclopropancarboxamid **(1-1)** wurde in *N,N*-Dimethylformamid vorgelegt, unter Kühlung wurde bei einer Temperatur von 0 °C 17,8 mg (0,44 mmol, 60%ig) Natriumhydrid hinzugegeben und 15 Minuten nachgerührt. Bei Raumtemperatur wurde 52,5 mg (0,37 mmol) Iodmethan hinzudosiert und es wurde 2 h nachgerührt. Das Reaktionsgemisch wurde mit Wasser versetzt und gegen Dichlormethan extrahiert. Die organische Phase wurde mit gesättigter wässriger Natriumchlorid Lösung gewaschen, über Magnesiumsulfat getrocknet und unter vermindertem Druck vom Lösungsmittel befreit. Der Rückstand wurde durch säulenchromatographische Aufreinigung mit einem Cyclohexan / Ethylacetat Gradienten als Laufmittel und anschließend durch säulenchromatographische Aufreinigung mit einem Wasser / Acetonitril Gradienten als Laufmittel gereinigt Es wurden 18,5 mg (12% d.Th.) *N*-{6-[(Cyclopropylcarbonyl)amino]-4-[4-(trifluormethoxy)phenyl]pyridin-2-yl}-*N-*methylcyclopropancarboxamid erhalten.

logP (neutral): 3,66; MH⁺: 420; ¹H-NMR(600 MHz, D₆-DMSO) δ ppm: 0,75-0,73 (m, 2H), 0,87-0,81 (m, 6H), 1,72-1,66 (m, 1H), 2,08-2,02 (m, 1H), 3,31 (s, 3H), 7,88-7,50 (m, 5H), 8,34 (d, 1H), 11,04 (s, 1H)

### N,N'-{4-[4-(Trifluormethoxy)phenyl]pyridin-2,6-diyl}dicyclopropancarbothioamid (1-32)

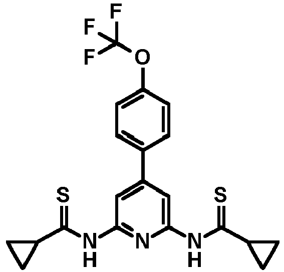

150 mg (0,37 mmol) *N,N'*-{4-[4-(Trifluormethoxy)phenyl]pyridin-2,6-diyl}dicyclopropancarboxamid **(I-1)** wurde in 5 ml Toluol vorgelegt, 225 mg (0,55 mmol) 4-Methoxyphenyldithiophosphonsaeureanhydrid (Lawessons Reagenz) wurde hinzugegeben. Die Reaktionssuspension wurde auf 110 °C erwärmt und über Nacht nachgerührt. Nach dem Abkühlen wurde das Reaktionsgemisch unter vermindertem Druck vom Lösungsmittel befreit. Der Rückstand wurde durch säulenchromatographische Aufreinigung mit einem Cyclohexan / Ethylacetat Gradienten als Laufmittel gereinigt. Es wurden 58,8 mg (30% d.Th.) *N,N'*-{4-[4-(Trifluormethoxy)phenyl]pyridin-2,6-diyl} dicyclopropancarbothioamid erhalten.

logP (neutral): 4,98; MH⁺: 438; ¹H-NMR(600 MHz, D₆-DMSO) δ ppm: 1,05-0,98 (m, 4H), 1,17 (m, 4H), 2,57 (s, 2H), 7,85-7,55 (m, 4H), 8,65 (s, 2H), 12,20 (s, 2H)

### N-16-[(Cyclopropylcarbonothioyl)amino]-4-[4-(trifluormethoxy)phenyl]pyridin-2-yl}cyclopropancarboxamid (1-33)

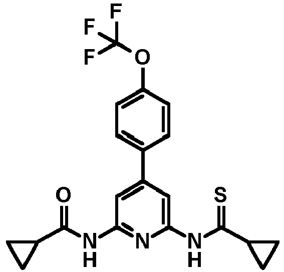

450 mg (1,11 mmol) *N,N'*-{4-[4-(Trifluormethoxy)phenyl]pyridin-2,6-diyl}dicyclopropancarboxamid wurde in 15 ml Toluol vorgelegt, 180 mg (0,44 mmol) 4-Methoxyphenyldithiophosphonsaeureanhydrid wurde hinzugegeben. Die Reaktionssuspension wurde auf 100 °C erwärmt und 3 h nachgerührt. Nach dem Abkühlen wurde das Reaktionsgemisch unter vermindertem Druck vom Lösungsmittel befreit. Der Rückstand wurde durch säulenchromatographische Aufreinigung mit einem Cyclohexan / Ethylacetat Gradienten als Laufmittel gereinigt. Es wurden 200 mg (43% d.Th.) *N*-{6-[(Cyclopropylcarbonothioyl)amino]-4-[4-(trifluormethoxy)phenyl]pyridin-2-yl}cyclopropancarboxamid erhalten.

logP (neutral): 4,44; MH⁺: 422; ¹H-NMR(600 MHz, D₆-DMSO) δ ppm: 0,88-0,81 (m, 4H), 1,02-1,00 (m, 2H), 1,16 (m, 2H), 2,09-2,07 (m, 1H), 2,56 (m, 1H), 7,81-7,52 (m, 4H), 8,23 (s, 1H), 8,38 (s, 1H), 10,77 (s, 1H), 11,99 (s, 1H)

### 3'-Fluor-4'-(pentafluorethyl)biphenyl-3,5-diamin

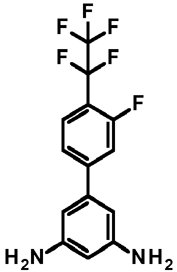

1,93 g (6,60 mmol) 4-Brom-2-fluor-1-(pentafluorethyl)benzol, 1 g (6,60 mmol) (3,5-Diaminophenyl)borsäure und 228 mg (0,19 mmol) Tetrakis-(triphenylphosphin)-palladium wurden unter Schutzgasatmophäre vorgelegt und mit 26,3 ml entgastem Dioxan und 13,2 ml (26,3 mmol) ebenfalls entgaster wässriger Natriumcarbonatlösung (2M) versetzt. Es wurde bei 96 °C über Nacht nachgerührt. Nach dem Abkühlen wurde unter vermindertem Druck das Lösungsmittel entfernt. Der Rückstand wurde mit Wasser versetzt und die ausgefallenen Kristalle wurden abgesaugt. Es wurden 2 g (67% d.Th.) 3'-Fluor-4'-(pentafluorethyl)biphenyl-3,5-diamin erhalten.

logP (neutral): 2,83; MH⁺: 321; ¹H-NMR(600 MHz, D₆-DMSO) δ ppm: 4,93 (s, 4H), 5,93 (d, 1H), 6,14 (d, 2H), 7,76-7,36 (m, 3H)

### N-[5-Amino-3'-fluor-4'-(pentafluorethyl)biphenyl-3-yl]-2-methylpropanamid

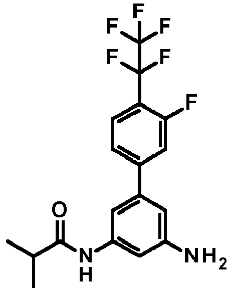

995 mg (3,10 mmol) 3'-Fluor-4'-(pentafluorethyl)biphenyl-3,5-diamin wurde in 30 mL Dichlormethan vorgelegt, mit 1,21 g (9,32 mmol) *N,N*-Diisopropylethylamin versetzt und unter Kühlung bei 0°C 331 mg (3,10 mmol) 2-Methylpropanoylchlorid zugetropft. Das Reaktionsgemisch wurde über Nacht bei Raumtemperatur nachgerührt und anschließend mit Wasser gewaschen. Die organische Phase wurde über Magnesiumsulfat getrocknet und unter vermindertem Druck vom Lösungsmittel befreit. Der Rückstand wurde mit Dichlormethan versetzt und die ausgefallenen Kristalle abgesaugt. Es wurden 123 mg (10% d.Th.) *N*-[5-Amino-3'-fluor-4'-(pentafluorethyl)biphenyl-3-yl]-2-methylpropanamid erhalten.

logP (neutral): 3,32; MH⁺: 391; ¹H-NMR(600 MHz, D₆-DMSO) δ ppm: 1,09 (d, 6H), 2,62-2,55 (m, 1H), 5,30 (s, 2H), 5,76 (s, 1H), 6,60 (s, 1H), 7,01 (s,1H), 7,14 (s, 1H), 7,59 (s, 1H), 7,79 (t, 1H), 9,68 (s, 1H)

### N-[3'-Fluor-5-(isobutyrylamino)-4'-(pentafluorethyl)biphenyl-3-yl]cyclopropancarboxamid (1-60)

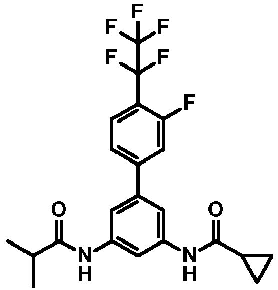

11 mg (0,12 mmol) Cyclopropancarbonsäure wurden in 1,9 ml Dioxan vorgelegt und mit 24,2 mg (0,12 mmol) 1-(3-Dimethylaminopropyl)-3-ethylcarbodiimidhydrochlorid versetzt und 1 h bei Raumtemperatur nachgerührt. Anschließend wurden 41 mg (0,10 mmol) *N*-[5-Amino-3'-fluor-4'-(pentafluorethyl)biphenyl-3-yl]-2-methylpropanamid zugegeben und über Nacht bei Raumtemperatur nachgerührt. Das Reaktionsgemisch wurde unter vermindertem Druck vom Lösungsmittel befreit, mit Wasser versetzt und gegen Dichlormethan extrahiert. Die organische Phase wurde über Magnesiumsulfat getrocknet und unter vermindertem Druck vom Lösungsmittel befreit. Es wurden 22,8 mg (47% d.Th.) *N*-[3'-Fluor-5-(isobutyrylamino)-4'-(pentafluorethyl)biphenyl-3-yl]cyclopropancarboxamid erhalten.

logP (HCOOH): 3,7; MH⁺: 459; ¹H-NMR(600 MHz, D₆-DMSO) δ ppm: 0,83-0,81 (m, 4H), 1,12 (d, 6H), 1,84-1,81 (m, 1H), 2,68-2,59 (m, 1H), 7,70-7,63 (m, 4H), 7,85 (t, 1H), 8,01 (s, 1H), 10,00 (s, 1H), 10,37 (s, 1H)

### 4-Brom-3-chlorpyridin-2,6-diamin

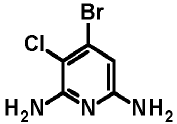

2 g (10,6 mmol) 4-Brom-2,6-diaminopyridin wurden in 100 ml Trichlormethan vorgelegt. Unter Kühlung bei einer Temperatur von 0 °C wurden 1,42 g (10,6 mmol) *N*-Chlorsuccinimid portionsweise hinzugegeben, 1 h bei 0 °C und anschließend über Nacht bei Raumtemperatur nachgerührt. Die Reaktionssuspension wurde filtriert und die Mutterlauge wurde mit Wasser und mit wässrige Natriumthiosulfatlösung gewaschen. Die organische Phase wurde über Magnesiumsulfat getrocknet und unter vermindertem Druck vom Lösungsmittel befreit. Der Rückstand wurde durch säulenchromatographische Aufreinigung mit einem Cyclohexan / Aceton Gradienten als Laufmittel gereinigt. Es wurden 441 mg (18% d.Th.) 4-Brom-3-chlorpyridin-2,6-diamin erhalten.

logP (neutral): 1,46; MH⁺: 224; ¹H-NMR(600 MHz, D₆-DMSO) δ ppm: 5,81 (s, 2H), 5,99 (s, 2H), 6,01 (s, 1H)

In Analogie zu den Beispielen und gemäß den oben beschriebenen Herstellverfahren lassen sich folgende Verbindungen der Formel **(I)** erhalten:

| **Beispielnummer** | **Struktur** | **¹H-NMR-Daten** |
|---|---|---|
| **I-01** | 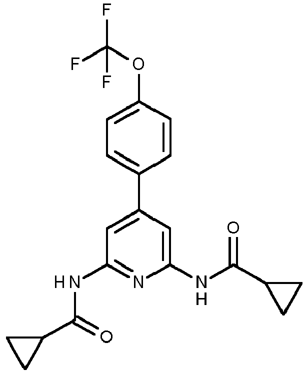 | I-01: ¹H-NMR(400.0 MHz, d₆-DMSO): δ= 10.5076(8.9);8.0460(15.0);7.7559(6. 2);7.7341(7.5);7.5159(5.1);7.4951(4.3); 3.3216(28.0);2.6727(0.3);2.5080(41.4);2 .5037(53.6);2.4995(40.4);2.3304(0.3);2. 1353(0.7);2.1177(2.0);2.1050(3.1);2.092 3(2.1);2.0742(0.8);1.9895(1.1);1.3976(2 .6);1.1760(0.6);0.8749(0.6);0.8516(11.9 );0.8434(16.0);0.8250(8.5);0.8039(0.8);-0.0002(1.0) |
| **I-02** | 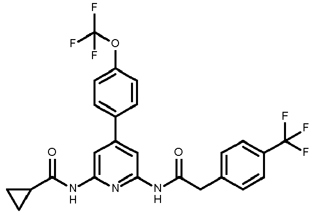 | I-02: ¹H-NMR(400.0 MHz, d₆-DMSO): δ= 10.5635(7.8);10.5258(8.0);8.3178(0. 9);8.0714(8.7);8.0687(9.0);8.0086(4.3); 7.7497(10.9);7.7277(13.8);7.7196(9.2);7 .6987(10.6);7.5924(9.8);7.5721(7.3);7.5 103(8.5);7.4897(7.3);3.9187(12.1);3.568 4(1.6);3.3299(243.8);2.6761(2.0);2.6718 (2.8);2.6676(2.1);2.5249(7.1);2.5072(31 5.0);2.5028(405.1);2.4984(299.5);2.333 8(2.0);2.3295(2.6);2.3251(2.0);2.1218(0 .6);2.1042(1.8);2.0905(2.7);2.0765(1.8); 2.0600(0.7); 1.3976(0.7); 1.3511(2.3); 1.3 358(0.9); 1.2982(0.4); 1.2586(0.8); 1.2493 (1.4);1.2314(3.0);0.8494(16.0);0.8403(1 0.4);0.8292(8.0);0.0077(0.5);-0.0002(16.0) |
| **I-03** | 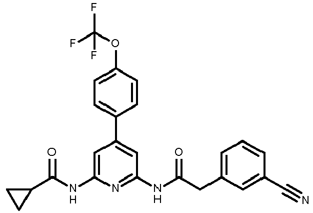 | I-03: ¹H-NMR(400.0 MHz, d₆-DMSO): δ= 10.5631(8.4);10.5205(8.8);8.0767(9. 1);8.0738(9.8);8.0114(4.3);7.8165(8.4); 7.7604(5.8);7.7550(13.2);7.7501(4.9);7. 7380(9.2);7.7330(15.0);7.7259(2.0);7.71 94(1.0);7.7081(4.0);7.6882(5.6);7.6661( 0.7);7.6325(0.3);7.6128(0.5);7.5813(5.2 );7.5619(8.2);7.5540(1.1);7.5426(3.4);7. 5337(0.7);7.5102(9.0);7.4898(7.7);7.459 6(0.3);6.4199(0.4);6.4167(0.5);5.9166(0 .5);5.7592(10.5);4.0390(0.4);4.0211(0.4 );3.8964(13.5);3.6911(2.3);3.3344(69.8) ;2.6784(0.5);2.6739(0.7);2.6697(0.5);2. 5272(1.9);2.5135(43.2);2.5094(85.1);2.5 050(110.7);2.5005(81.4);2.3362(0.5);2.3 317(0.7);2.3271(0.6);2.1255(0.6);2.1084 (1.8);2.0950(2.8);2.0815(1.9);2.0639(0. 8);1.9906(1.8);1.3969(3.3);1.3526(1.7); 1.3368(0.6);1.2588(0.5);1.2494(0.9);1.2 309(2.2);1.1939(0.5);1.1762(1.0);1.1584 (0.5);0.8521(16.0);0.8441(10.9);0.8323( 8.3);0.8102(0.7);0.7972(0.4);0.7891(0.5 );0.7767(0.7);0.7564(0.4);-0.0002(4.9) |
| **I-04** | 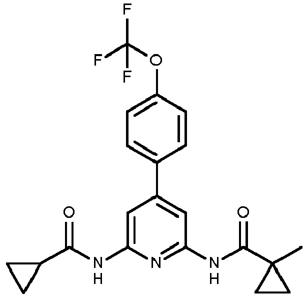 | I-04: ¹H-NMR(400.0 MHz, d₆-DMSO): δ= 10.7603(3.3);9.0907(3.6);8.3176(0.5 );8.1101(3.9);8.1071(4.2);7.9361(4.8);7. 9328(4.9);7.7783(5.0);7.7615(1.9);7.756 3(6.2);7.7494(0.7);7.5236(3.9);7.5035(3 .3);3.3303(166.6);2.6759(0.9);2.6716(1. 2);2.6671(1.0);2.5248(3.0);2.5111(69.4) ;2.5070(140.2);2.5025(185.4);2.4981(13 8.8);2.3337(0.9);2.3292(1.2);2.3249(0.9 );2.0622(0.8);2.0492(1.2);2.0359(0.8);2. 0179(0.3);1.4411(16.0);1.3507(0.5);1.24 92(0.4);1.2331(0.9);1.1828(2.3);1.1592( 1.5); 1.1500(4.2); 1.1432(4.5); 1.1345(1.7 ); 1.0377(0.5); 1.0306(0.5);0.8353(6.5);0. 8286(4.8);0.8153(3.4);0.7212(1.8);0.711 9(4.9);0.7049(5.0);0.6953(1.7);0.6560(0 .5);0.6487(0.5);-0.0002(6.7) |
| **I-05** | 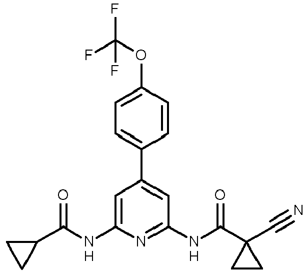 | I-05: ¹H-NMR(400.0 MHz, d₆-DMSO): δ= 10.8537(6.6);9.8201(7.1);8.3174(0.9 );8.1768(7.8);8.1738(8.4);7.8491(9.2);7. 8458(9.6);7.7845(9.6);7.7797(4.0);7.767 6(3.8);7.7626(11.8);7.5326(7.4);7.5123( 6.3);5.7577(0.4);3.3290(233.5);2.6757(2 .0);2.6713(2.8);2.6668(2.2);2.5245(7.2); 2.5067(317.5);2.5023(421.7);2.4978(32 2.7);2.3334(2.0);2.3289(2.8);2.3246(2.1 );2.0827(0.6);2.0662(1.6);2.0520(2.4);2. 0355(1.8);2.0214(0.7);1.7600(1.7);1.743 4(4.9); 1.7346(9.5); 1.7284(7.3); 1.7052(6 .9); 1.6986(9.5); 1.6898(5.5); 1.6733(1.7); 1.3508(0.6); 1.3353(0.7); 1.2980(0.4); 1.2 583(0.7);1.2490(1.1);1.2340(3.4);0.8487 (16.0);0.8377(9.2);0.8296(7.9);0.8059(0 .6);0.0079(0.5);-0.0003(13.8) |
| **I-06** | 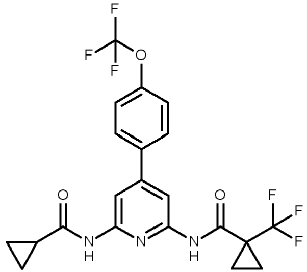 | I-06: ¹H-NMR(400.0 MHz, d₆-DMSO): δ= 13.4013(0.3);10.7643(5.6);9.8356(5. 3);8.3171(3.0);8.1599(6.7);8.1568(6.9); 7.8441(8.1);7.8408(8.0);7.7943(8.4);7.7 894(3.1);7.7775(3.3);7.7723(10.2);7.765 1(1.1);7.5325(6.1);7.5123(5.3);3.3297(8 87.3);3.2798(0.6);2.6756(5.8);2.6712(7. 9);2.6668(5.9);2.5963(0.4);2.5678(0.9); 2.5549(1.1);2.5244(19.7);2.5109(456.6); 2.5067(921.4);2.5022(1207.4);2.4977(8 90.0);2.4934(443.1);2.4251(0.6);2.4204( 0.6);2.4111(0.6);2.3866(0.5);2.3820(0.5 );2.3631(0.3);2.3514(0.4);2.3377(2.8);2. 3334(5.8);2.3289(8.0);2.3244(6.0);2.301 1(0.3);2.2929(0.3);2.1972(0.6);2.1787(1 .1);2.1604(0.6);2.1483(0.4);2.0975(0.6); 2.0788(1.3);2.0666(2.2);2.0498(1.4);2.0 344(0.8);1.5410(1.4);1.5244(5.8);1.4785 (0.4); 1.4728(0.6); 1.4160(0.5); 1.3970(0. 4); 1.3770(3.8); 1.3640(7.0); 1.3583(7.3); 1.3440(3.6); 1.3344(2.3); 1.2975(1.8); 1.2 898(1.7);1.2813(1.7);1.2700(1.5);1.2581 (1.9);1.2349(16.0);0.8535(3.3);0.8412(1 1.5);0.8334(8.0);0.8215(6.0);0.0079(1.3 );-0.0002(42.9);-0.0084(1.8) |
| **I-07** | 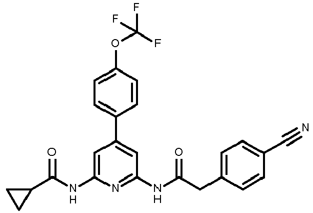 | I-07: ¹H-NMR(400.0 MHz, d₆-DMSO): δ= 10.5533(8.1);10.5231(8.3);8.3157(0. 4);8.0675(9.4);7.9977(4.4);7.8230(11.2) ;7.8025(13.4);7.7495(10.8);7.7276(13.1) ;7.5684(12.1);7.5479(10.4);7.5104(8.9); 7.4898(7.6);5.7564(0.9);3.9199(12.9);3. 3208(134.0);2.6710(2.1);2.5064(294.7); 2.5021(382.6);2.4978(280.1);2.3288(2.1 );2.1203(0.7);2.1021(1.9);2.0863(5.1);2. 0752(2.3);2.0580(0.8);0.8488(16.0);0.84 02(10.7);0.8290(8.2);0.8067(0.7);0.1460 (0.5);-0.0002(97.9);-0.1494(0.5) |
| **I-08** | 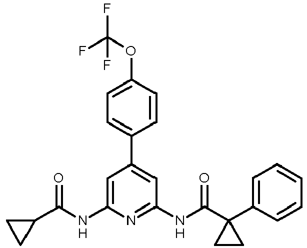 | I-08: ¹H-NMR(400.0 MHz, d₆-DMSO): δ= 10.7540(8.2);8.3170(4.4);8.0916(8.9 );8.0883(12.6);8.0749(13.3);8.0715(9.7) ;7.7710(10.8);7.7617(12.7);7.7565(4.6); 7.7448(4.3);7.7397(14.9);7.5870(5.1);7. 5830(7.6);7.5660(11.4);7.5258(9.2);7.51 76(6.4);7.5004(13.5);7.4813(6.5);7.4631 (4.9);7.4520(1.6);7.4455(5.2);7.4381(1. 0);7.4273(1.3);3.5417(0.4);3.5295(0.4); 3.5153(0.4);3.5057(0.4);3.4761(0.5);3.4 677(0.5);3.3302(2090.8);3.2930(1.0);2.6 759(7.5);2.6714(10.5);2.6669(7.8);2.619 4(0.7);2.5247(26.0);2.5198(40.5);2.5112 (569.4);2.5068(1177.1);2.5023(1564.0); 2.4978(1142.4);2.4934(558.9);2.3380(3. 2);2.3336(7.0);2.3290(9.9);2.3246(7.2); 1.9475(0.6);1.9308(1.9);1.9171(3.0);1.9 041(2.0); 1.8864(0.9); 1.5873(3.7); 1.5773 (8.8); 1.5698(9.8); 1.5608(4.0); 1.3978(6. 4); 1.3507(1.7); 1.3354(0.8); 1.2985(0.5); 1.2590(0.9); 1.2496(1.5); 1.2292(6.6); 1.2 199(9.8);1.2121(9.6);1.2021(3.7);1.1810 (0.4);0.8525(0.7);0.8347(0.6);0.8216(0. 6);0.7886(16.0);0.7795(10.2);0.7685(8.0 );0.1462(0.5);0.0080(4.0);-0.0002(129.9);-0.0083(4.4);-0.1498(0.6) |
| **I-09** | 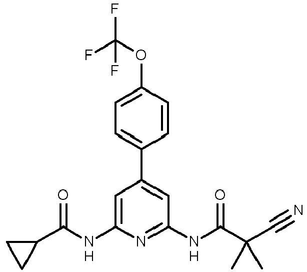 | I-09: ¹H-NMR(400.0 MHz, d₆-DMSO): δ= 10.7590(1.6);10.3110(1.6);8.3170(1. 7);8.1651(1.9);7.8411(2.2);7.8380(2.2); 7.8113(2.3);7.7893(2.8);7.5376(1.6);7.5 172(1.5);6.8951(0.6);5.7575(6.7);3.3306 (1090.1);2.7336(0.4);2.6757(3.7);2.6711 (5.1);2.6667(3.8);2.5244(13.6);2.5109(2 97.8);2.5066(599.5);2.5021(780.8);2.49 76(560.2);2.4933(268.5);2.3334(3.6);2.3 289(4.9);2.3244(3.6);2.0658(0.6);1.6888 (16.0);1.3982(0.3);1.3505(5.2);1.3358(1 .8);1.3138(0.5);1.2978(1.4);1.2584(2.1); 1.2491(2.8);1.2334(6.8);1.1726(0.6);0.8 504(3.5);0.8396(2.6);0.8275(1.9);0.0080 (2.0);-0.0002(59.9);-0.0086(1.7) |
| **I-10** | 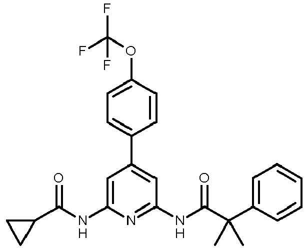 | I-10: ¹H-NMR(400.0 MHz, d₆-DMSO): δ= 10.6725(2.0);8.5635(2.1);8.3175(3.7 );8.0740(2.4);8.0708(2.6);7.9793(2.9);7. 9760(2.7);7.7884(2.9);7.7832(1.1);7.766 4(3.6);7.5324(2.2);7.5125(1.9);7.4545(0 .8);7.4503(1.3);7.4328(3.4);7.4300(3.1); 7.4209(2.4);7.4028(2.6);7.3827(1.1);7.3 235(0.9);7.3056(1.1);7.2887(0.4);3.4124 (0.4);3.4031(0.5);3.3944(0.6);3.3776(0. 8);3.3288(738.2);3.3052(1.9);2.6948(0.3 );2.6756(4.6);2.6710(6.3);2.6666(4.7);2. 6107(0.5);2.5243(16.1);2.5108(345.1);2. 5065(704.8);2.5020(931.1);2.4975(680. 3);2.4932(332.1);2.3333(4.2);2.3288(5.9 );2.3244(4.4); 1.9874(0.4); 1.9833(0.4); 1. 9681(0.7);1.9553(0.5);1.6074(16.0);1.35 08(0.6); 1.2494(0.4); 1.2339(1.0);0.7966( 3.2);0.7768(2.0);0.1460(2.9);0.0079(20. 9);-0.0002(659.6);-0.0083(25.0);-0.0203(1.5);-0.0312(0.7);-0.0579(0.4);-0.1497(3.0) |
| **I-11** | 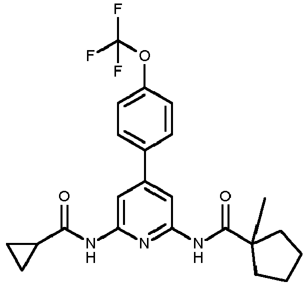 | I-11: ¹H-NMR(400.0 MHz, d₆-DMSO): δ= 12.0136(1.1);10.6706(1.6);10.5297(3 .7);10.1046(3.8);9.4314(1.6);8.3176(0.5 );8.0821(1.9);8.0499(6.6);8.0460(5.9);7. 9217(2.0);7.9190(1.9);7.7902(2.1);7.766 8(6.4);7.7444(5.7);7.5232(5.3);7.5023(4 .5);5.7577(4.0);3.5685(1.1);3.3315(120. 3);2.6721(1.4);2.5932(0.7);2.5648(1.5); 2.5570(1.2);2.5069(175.2);2.5030(218.0 );2.3297(1.4);2.1009(1.7);2.0933(1.9);2. 0558(0.8);2.0429(0.5);2.0002(1.4);1.984 0(1.3);1.9703(1.3);1.9556(0.8);1.8260(2 .0); 1.7970(2.3); 1.7667(1.8); 1.7349(2.2); 1.6651(2.0); 1.6462(2.4); 1.6383(2.6); 1.6 222(3.4); 1.6096(4.4); 1.5729(1.6); 1.5590 (1.1);1.5416(1.0);1.5290(1.0);1.4469(0. 8);1.4160(2.3);1.3924(3.3);1.3865(3.3); 1.3634(3.0); 1.3535(2.6); 1.3276(8.2); 1.2 844(2.0); 1.2517(2.4); 1.2334(2.6); 1.2234 (2.4); 1.1942(1.2); 1.1566(16.0); 1.0774(1 .9);1.0565(0.6);0.9949(0.6);0.9179(0.3); 0.8776(0.7);0.8385(9.9);0.8193(5.2);0.1 462(0.4);-0.0002(75.7);-0.1495(0.4) |
| **I-12** | 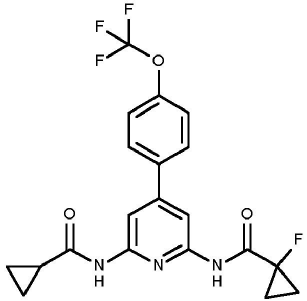 | I-12: ¹H-NMR(400.0 MHz, d₆-DMSO): δ= 10.8076(8.1);9.8213(4.8);8.3181(0.7 );8.1827(9.1);8.1799(9.3);7.8714(12.1); 7.8681(12.3);7.8057(1.3);7.7983(13.0);7 .7933(4.5);7.7815(4.6);7.7763(16.0);7.7 690(1.8);7.5347(9.4);7.5145(8.0);5.7582 (9.2);3.3357(179.8);2.6774(0.6);2.6729( 0.9);2.6684(0.6);2.5263(2.1);2.5215(3.3 );2.5127(48.8);2.5084(101.2);2.5039(13 4.0);2.4994(97.2);2.4950(47.3);2.3352(0 .6);2.3306(0.8);2.3262(0.6);2.0975(0.6); 2.0801(1.7);2.0667(2.6);2.0531(1.8);2.0 359(0.7);1.5281(1.4);1.5118(3.7);1.5042 (4.5); 1.4920(2.4); 1.4828(1.6); 1.4659(3. 3); 1.4582(4.6); 1.4468(2.7); 1.4176(0.7); 1.3971(5.3);1.3828(4.7);1.3748(6.2);1.3 619(5.0);1.3556(4.0);1.3378(1.3);0.8748 (0.4);0.8469(15.9);0.8395(11.2);0.8270( 8.4);0.8050(0.6);0.0079(1.9);-0.0002(64.2);-0.0085(2.3) |
| **I-13** | 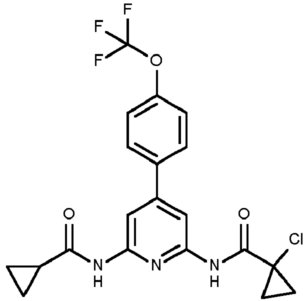 | I-13: ¹H-NMR(400.0 MHz, d₆-DMSO): δ= 10.9257(6.7);9.4710(7.6);8.3181(0.4 );8.1946(8.5);8.1915(8.7);7.9038(9.5);7. 9005(9.5);7.7992(1.0);7.7918(9.9);7.786 9(3.6);7.7749(3.6);7.7698(12.1);7.7627( 1.5);7.5333(7.4);7.5131(6.4);5.7581(1.1 );3.3318(121.2);2.6770(0.7);2.6725(1.0) ;2.6680(0.7);2.5259(2.3);2.5079(112.8); 2.5035(148.5);2.4990(108.8);2.4948(54. 2);2.3346(0.7);2.3302(1.0);2.3258(0.7); 2.0602(0.6);2.0435(1.6);2.0296(2.5);2.0 133(1.7);1.9989(0.7);1.6851(3.1);1.6711 (7.8);1.6632(8.6);1.6512(4.1);1.6089(0. 4);1.5323(0.4);1.4899(4.3);1.4777(8.2); 1.4699(8.4);1.4558(3.1);0.8477(16.0);0. 8367(8.6);0.8285(7.4);0.8057(0.4);0.008 0(2.2);-0.0002(69.5);-0.0083(2.9) |
| **I-14** | 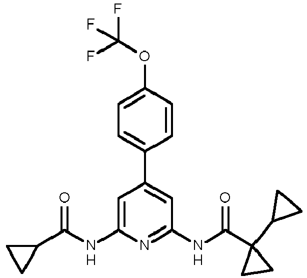 | I-14: ¹H-NMR(400.0 MHz, d₆-DMSO): δ= 12.0913(0.5);10.8514(9.3);9.1184(10 .3);8.3144(0.4);8.1406(11.1);8.1377(11. 9);8.0537(12.7);8.0506(12.2);7.7804(13. 3);7.7585(16.0);7.5254(10.3);7.5048(8.9 );5.7543(5.2);3.3197(66.3);2.6762(0.9); 2.6719(1.2);2.6672(0.9);2.5250(3.2);2.5 072(135.5);2.5028(178.2);2.4985(134.0) ;2.3339(0.9);2.3296(1.2);2.3254(0.9);2. 0854(0.8);2.0721(1.9);2.0674(2.1);2.061 9(1.8);2.0546(3.5);2.0414(2.2);2.0373(2 .2);2.0235(0.9);1.6151(0.8);1.6025(1.8); 1.5952(2.0);1.5829(3.5);1.5703(2.2);1.5 629(1.9); 1.5502(0.9); 1.4273(0.4); 1.4140 (0.8);1.4065(0.9);1.3980(1.1);1.3934(1. 8); 1.3855(0.6); 1.3803(0.9); 1.3726(0.9); 1.3596(0.5);1.2332(1.1);1.1595(1.9);1.0 700(2.8);1.0330(4.0);1.0233(10.5);1.016 2(11.4); 1.0073(4.6);0.9031(1.7);0.8933( 5.0);0.8861(5.4);0.8769(2.2);0.8673(0.7 );0.8373(16.0);0.8175(9.0);0.7960(0.8); 0.6549(4.8);0.6457(12.7);0.6377(14.9);0 .6214(4.4);0.6151(6.9);0.6121(7.2);0.60 16(2.3);0.4977(1.8);0.4884(5.0);0.4812( 4.9);0.4714(1.7);0.3803(0.9);0.3688(2.9 );0.3655(3.0);0.3599(1.3);0.3544(1.3);0. 3481(2.9);0.3447(2.9);0.3337(1.1);0.287 2(2.3);0.2738(8.8);0.2622(8.3);0.2498(2 .2);0.1461(1.1);0.0078(8.8);-0.0002(220.4);-0.0328(1.2);-0.0466(3.9);-0.0595(3.6);-0.0715(1.0);-0.1495(1.1) |
| **I-15** | 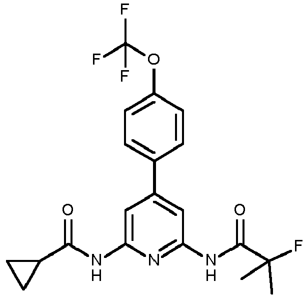 | I-15: ¹H-NMR(400.0 MHz, d₆-DMSO): δ= 10.8016(2.8);9.4373(1.5);9.4257(1.4 );8.3139(1.2);8.1750(3.6);8.1724(3.7);7. 8981(4.0);7.8951(4.0);7.8033(4.5);7.781 5(5.4);7.5358(3.5);7.5154(3.0);3.3177(2 1.7);3.2942(0.4);2.6713(0.4);2.6675(0.3 );2.5241(1.4);2.5067(52.8);2.5023(69.0) ;2.4979(52.1);2.3335(0.3);2.3290(0.4);2 .3247(0.3);2.0612(0.7);2.0483(1.1);2.03 46(0.7); 1.9887(0.5); 1.6438(16.0); 1.5885 (16.0);1.3979(10.3);0.8453(6.0);0.8379( 4.3);0.8254(3.1);0.0077(1.7);-0.0002(45.2);-0.0080(2.2) |
| **I-16** | 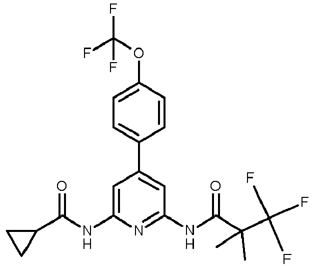 | I-16: ¹H-NMR(400.0 MHz, d₆-DMSO): δ= 10.7642(1.8);9.7867(1.8);8.1680(2.4 );7.8093(2.6);7.8011(2.7);7.7982(2.8);7. 7875(3.1);7.5301(2.1);7.5093(1.8);3.317 5(43.4);2.6755(0.4);2.6711(0.5);2.5063( 57.4);2.5020(76.8);2.4977(60.3);2.3291( 0.5);2.3248(0.4);2.0816(0.4);2.0765(0.4 );2.0690(0.7);2.0560(0.4); 1.5357(16.0); 1.2351(2.0);0.8388(3.3);0.8201(1.8);0.0 078(1.8);-0.0002(46.8) |
| **I-17** | 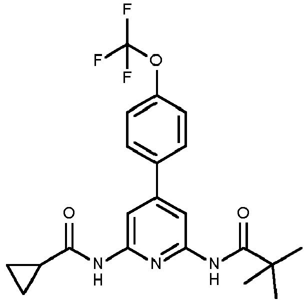 | I-17: ¹H-NMR(400.0 MHz, d₆-DMSO): δ= 10.6784(1.0);9.3273(1.0);8.0894(1.2 );7.9210(1.3);7.9190(1.3);7.7915(1.3);7. 7697(1.5);7.5242(1.1);7.5034(1.0);3.316 1(47.6);2.6703(0.6);2.5013(100.8);2.497 2(79.2);2.3281(0.6);2.0515(0.4);1.3980( 1.1);1.2573(16.0);0.8309(1.8);0.8126(0. 9);-0.0002(56.0) |
| **I-18** | 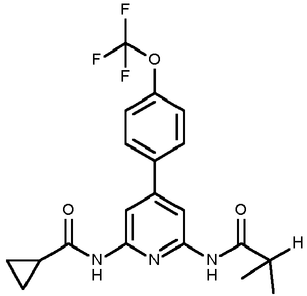 | I-18: ¹H-NMR(400.0 MHz, d₆-DMSO): δ= 10.5256(2.8);10.1242(2.8);8.0493(10 .3);7.7731(4.1);7.7512(5.0);7.5248(3.2); 7.5043(2.8);3.3209(18.1);2.8536(0.4);2. 8366(0.9);2.8197(1.3);2.8026(1.0);2.785 7(0.4);2.6756(0.4);2.6714(0.5);2.5066(6 4.8);2.5023(84.5);2.4980(61.1);2.3332(0 .4);2.3289(0.5);2.0991(0.6);2.0860(1.0); 2.0730(0.7);1.1166(16.0);1.0996(15.7);0 .8395(5.0);0.8205(2.8);0.0078(1.9);-0.0002(46.6);-0.0083(2.0) |
| **I-19** | 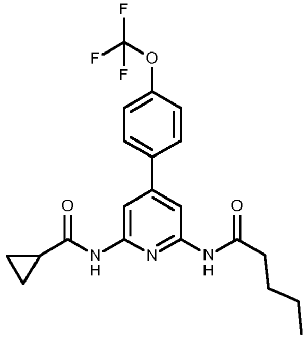 | I-19: ¹H-NMR(400.0 MHz, d₆-DMSO): δ= 10.s107(1.4);10.1467(1.4);8.0463(3. 4);7.7656(1.8);7.7438(2.2);7.5246(1.6); 7.5039(1.4);5.7535(16.0);3.3196(12.6);2 .5028(30.7);2.4577(1.0);2.4395(1.9);2.4 208(1.1);2.1033(0.4);2.0910(0.5);2.0774 (0.4);1.6007(0.8);1.5823(1.2);1.5635(0. 9);1.5446(0.3);1.3586(0.7);1.3398(1.2); 1.3211(1.1);1.3029(0.7);0.9187(2.1);0.9 004(4.1);0.8821(2.0);0.8646(0.5);0.8382 (2.8);0.8197(1.4);-0.0002(18.6) |
| **I-20** | 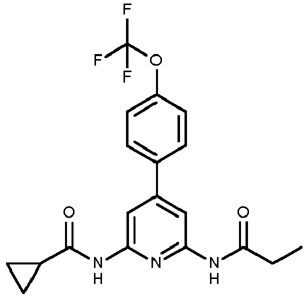 | I-20: ¹H-NMR(400.0 MHz, d₆-DMSO): δ= 10.5075(4.4);10.1367(4.4);8.0475(5. 4);8.0432(7.6);7.7749(0.7);7.7674(6.9); 7.7624(2.5);7.7506(2.5);7.7454(8.6);7.7 383(1.1);7.5279(5.0);7.5079(4.2);3.3246 (328.8);3.2878(0.5);2.9948(0.7);2.7110( 0.5);2.6799(0.4);2.6756(0.8);2.6709(1.2 );2.6665(0.9);2.6622(0.4);2.5413(119.8) ;2.5244(2.8);2.5195(4.2);2.5109(64.1);2 .5065(132.9);2.5019(177.6);2.4973(132. 1);2.4929(65.8);2.4778(2.4);2.4589(5.9) ;2.4400(5.8);2.4213(1.9);2.3678(0.4);2. 3333(0.8);2.3287(1.1);2.3242(0.8);2.119 7(0.4);2.1028(1.0);2.0896(1.5);2.0761(1 .0);2.0724(1.0);2.0584(0.4); 1.1049(7.4); 1.0862(16.0);1.0673(7.2);0.8402(7.8);0. 8350(6.3);0.8202(4.4);0.7985(0.4);-0.0001(2.9) |
| **I-21** | 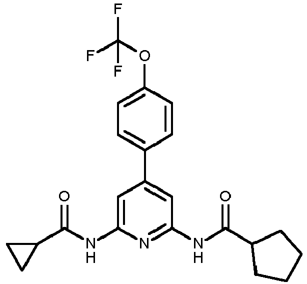 | I-21: ¹H-NMR(400.0 MHz, d₆-DMSO): δ= 10.5251(7.7);10.1263(7.8);8.0492(16 .0);7.7732(11.3);7.7686(4.6);7.7513(13. 7);7.5236(8.8);7.5032(7.6);3.3308(212.5 );3.2985(0.5);3.2908(0.4);3.0401(0.5);3. 0208(1.7);3.0019(2.9);2.9822(2.2);2.962 4(0.8);2.7133(0.4);2.6729(0.6);2.6685(0 .5);2.5432(101.6);2.5083(70.6);2.5039(9 2.7);2.4994(70.0);2.3696(0.4);2.3350(0. 4);2.3305(0.6);2.3263(0.4);2.1148(0.6); 2.0972(1.8);2.0844(2.7);2.0714(1.8);2.0 532(0.7);1.9027(0.9);1.8948(1.1);1.8817 (2.2);1.8735(3.4);1.8534(4.2);1.8388(2. 5); 1.7706(1.0); 1.7525(3.0); 1.7340(3.9); 1.7160(3.4); 1.7024(3.7); 1.6896(5.6); 1.6 804(3.6); 1.6738(3.6); 1.6569(2.6); 1.6305 (1.1);1.6065(0.8);1.5980(1.0);1.5900(0. 8);1.5713(3.5);1.5610(3.7);1.5549(4.1); 1.5423(3.2);1.5361(2.3);1.5141(0.6);0.8 708(0.5);0.8406(13.6);0.8213(7.6);0.799 9(0.7);-0.0002(1.2) |
| **I-22** | 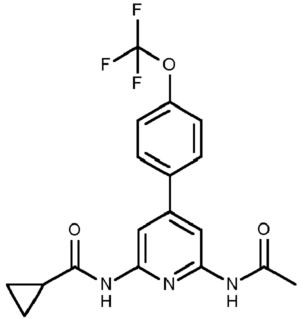 | I-22: ¹H-NMR(400.0 MHz, d₆-DMSO): δ= 10.5061(2.5);10.2091(2.5);8.3135(0. 6);8.0458(2.8);8.0430(3.3);8.0204(1.3); 7.7634(0.4);7.7562(4.1);7.7511(1.5);7.7 394(1.5);7.7341(5.1);7.7271(0.6);7.5275 (3.0);7.5075(2.5);5.7536(16.0);3.3165(8 9.9);2.6751(0.9);2.6706(1.2);2.6660(0.9 );2.6614(0.4);2.5414(0.3);2.5240(3.0);2. 5192(4.6);2.5105(68.8);2.5061(142.6);2. 5015(190.4);2.4969(140.4);2.4925(69.1) ;2.3328(0.9);2.3283(1.3);2.3237(0.9);2. 1429(12.3);2.1084(0.6);2.1022(0.5);2.09 53(0.9);2.0855(0.6);2.0831(0.6);2.0733( 1.5);0.8456(3.6);0.8396(4.4);0.8199(2.6 );0.1461(0.4);0.0081(3.0);-0.0002(101.9);-0.0084(3.9);-0.1498(0.4) |
| **I-23** | 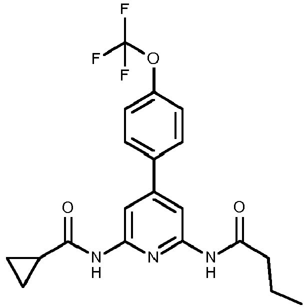 | I-23: ¹H-NMR(400.0 MHz, d₆-DMSO): δ= 10.5121(4.5);10.1439(4.6);8.0514(6. 6);8.0481(8.1);7.7750(0.8);7.7678(6.9); 7.7627(2.5);7.7508(2.7);7.7457(8.4);7.7 386(1.1);7.5248(5.1);7.5047(4.4);5.7543 (1.8);3.3218(46.2);2.6728(0.4);2.5260(1 .3);2.5125(24.4);2.5082(48.3);2.5036(62 .8);2.4991(46.3);2.4947(23.0);2.4361(3. 4);2.4179(6.8);2.3995(3.7);2.3303(0.4); 2.1226(0.4);2.1093(0.9);2.1041(1.0);2.0 917(1.6);2.0789(1.0);2.0742(1.0);2.0607 (0.4); 1.6672(0.5); 1.6487(2.5); 1.6302(4. 8);1.6118(4.8);1.5935(2.5);1.5750(0.6); 1.3977(0.4); 1.2329(0.8);0.9422(7.9);0.9 238(16.0);0.9053(7.2);0.8480(6.0);0.838 8(8.3);0.8201(4.4);0.7989(0.4);0.0079(1 .8);-0.0002(49.3);-0.0084(2.0) |
| **I-24** | 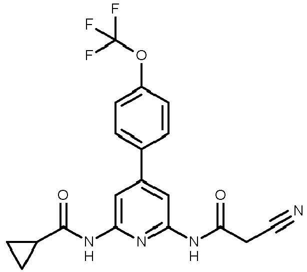 | I-24: ¹H-NMR(400.0 MHz, d₆-DMSO): δ= 10.5944(7.2);10.5694(8.0);8.1118(9. 6);8.1091(9.6);7.9627(1.0);7.7801(11.6) ;7.7582(14.0);7.6874(0.5);7.6655(0.6);7 .5523(0.7);7.5363(9.6);7.5161(8.2);7.47 52(0.4);6.4220(0.4);6.4186(0.4);5.8951( 0.4);5.7515(8.8);4.0570(3.0);4.0222(1.0 );4.0042(0.5);3.3125(61.3);2.6767(0.4); 2.6722(0.6);2.6680(0.4);2.5387(0.3);2.5 343(0.3);2.5255(1.4);2.5119(36.5);2.507 7(72.1);2.5033(93.8);2.4987(67.9);2.494 5(33.5);2.3343(0.4);2.3301(0.6);2.3254( 0.4);2.1170(0.6);2.1010(1.7);2.0877(2.4 );2.0737(1.9);2.0574(0.7);1.9888(1.0);1. 3978(0.6); 1.3536(0.5); 1.3362(0.4); 1.259 9(0.4);1.2504(0.6);1.2314(0.8);1.1764(0 .6);0.8840(0.4);0.8557(16.0);0.8483(11. 7);0.8357(8.5);0.8133(0.7);0.7902(0.5); 0.7788(0.6);0.7747(0.6);0.7546(0.4);0.0 079(0.6);-0.0002(19.1);-0.0084(0.8) |
| **I-25** | 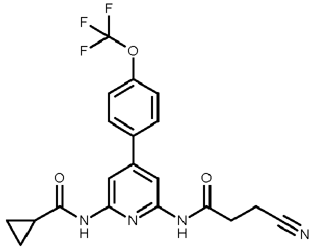 | I-25: ¹H-NMR(400.0 MHz, d₆-DMSO): δ= 10.5293(8.1);10.3921(8.6);8.0793(10 .5);8.0352(2.9);7.7781(9.9);7.7566(12.1 );7.5278(9.4);7.5067(8.2);5.7513(8.0);4. 0402(0.4);4.0223(0.4);3.3203(51.4);2.86 42(2.8);2.8482(6.6);2.8318(5.0);2.7605( 7.4);2.7440(10.0);2.7263(3.7);2.6740(0. 4);2.6181(0.5);2.6054(0.4);2.5050(54.6) ;2.3311(0.3);2.1295(0.6);2.1121(2.0);2. 0991(2.9);2.0868(2.1);2.0684(0.8);1.989 4(1.6);1.3975(0.4);1.2509(0.4);1.2321(1 .1);1.1948(0.4);1.1769(0.);1.1594(0.4); 0.8477(16.0);0.8294(8.2);0.8087(0.8);-0.0002(5.3) |
| **I-26** | 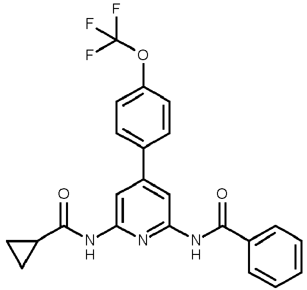 | I-26: ¹H-NMR(400.0 MHz, d₆-DMSO): δ= 10.6829(8.1);10.5076(9.1);8.3143(1. 6);8.1507(9.1);8.0826(11.1);8.0798(11.3 );8.0162(10.2);7.9983(10.7);7.9951(8.9) ;7.8341(12.0);7.8123(14.5);7.6410(1.8); 7.6226(5.8);7.6045(4.5);7.5627(8.7);7.5 473(11.9);7.5438(14.1);7.5297(10.0);5.7 544(9.5);3.3193(61.7);3.2955(0.7);2.675 1(1.0);2.6709(1.4);2.6668(1.1);2.5237(3 .5);2.5062(159.4);2.5019(213.6);2.4976( 164.8);2.3332(1.0);2.3285(1.4);2.3243(1 .1);2.1414(0.6);2.1232(1.7);2.1113(2.6); 2.0994(1.8);2.0805(0.7);1.3976(0.4);1.2 318(0.6);0.8817(0.7);0.8591(9.6);0.8476 (16.0);0.8276(7.9);0.8070(0.9);-0.0003(7.6) |
| **I-27** | 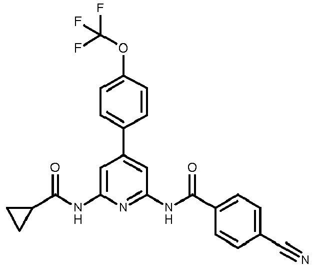 | I-27: ¹H-NMR(400.0 MHz, d₆-DMSO): δ= 10.8603(3.4);10.6742(3.1);8.1722(3. 7);8.1445(3.8);8.1236(5.7);8.0629(4.3); 8.0388(5.6);8.0179(3.9);7.8313(4.2);7.8 095(5.1);7.5521(3.6);7.5316(3.2);3.3197 (14.2);2.6716(0.4);2.5063(52.5);2.5026( 67.9);2.3292(0.4);2.1243(0.7);2.1139(1. 0);2.1011(0.8);1.3977(16.0);0.8610(3.9) ;0.8512(6.3);0.8319(3.1);0.8113(0.4);-0.0002(2.3) |
| **I-28** | 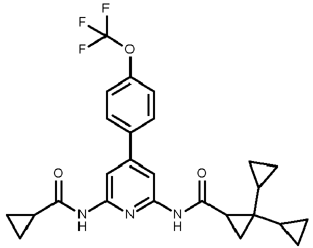 | I-28: ¹H-NMR(400.0 MHz, d₆-DMSO): δ= 10.5071(8.5);10.3650(8.5);8.0644(0. 4);8.0377(8.7);8.0289(11.2);7.7537(10.5 );7.7319(12.5);7.5202(9.3);7.4996(8.0); 3.3251(41.6);2.6740(0.5);2.5049(73.4);2 .3318(0.5);2.1264(0.8);2.1095(1.9);2.09 61(2.8);2.0826(2.0);2.0649(0.8);1.9364( 2.2);1.9203(3.3);1.9020(2.3);1.3975(2.4 );1.2314(0.5);1.0708(0.8);1.0566(1.7);1. 0506(1.9);1.0367(3.3);1.0284(1.6);1.022 5(2.2);1.0157(2.1);0.9902(4.6);0.9769(7 .6);0.9646(4.7);0.9419(0.9);0.9189(0.4); 0.9042(0.5);0.8858(0.9);0.8522(10.9);0. 8435(16.0);0.8249(8.1);0.8042(0.8);0.62 61(2.9);0.6146(3.2);0.6058(3.6);0.5946( 2.8);0.5816(1.8);0.5716(3.0);0.5591(3.0 );0.5429(1.6);0.3871(1.2);0.3768(2.6);0. 3646(4.4);0.3529(5.6);0.3429(7.6);0.331 0(7.2);0.3101(6.1);0.2957(3.0);0.2866(3 .4);0.2769(2.8);0.2670(1.9);0.2539(1.0); 0.1066(0.6);0.0950(1.0);0.0847(1.8);0.0 674(4.7);0.0623(4.7);0.0548(4.7);0.0396 (1.7);0.0287(0.9);-0.0002(2.0) |
| **I-29** | 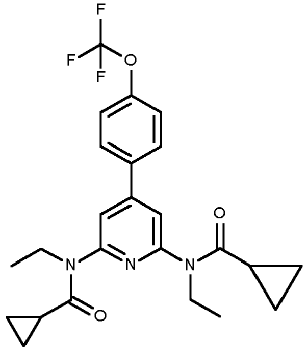 | I-29: ¹H-NMR(400.0 MHz, d₆-DMSO): δ= 7.9952(4.5);7.9734(5.1);7.7213(12.4 );7.5474(3.7);7.5265(3.3);3.9466(2.0);3. 9291(6.2);3.9114(6.2);3.8938(2.1);3.321 0(21.1);2.5080(24.7);2.5038(32.1);2.499 6(24.9);1.7192(0.6);1.7078(1.2);1.6991( 1.6);1.6888(2.3);1.6781(1.7);1.6697(1.3 );1.6581(0.7);1.2327(0.9);1.1522(7.6);1. 1347(16.0);1.1170(7.4);0.9173(1.8);0.90 73(4.8);0.8994(6.6);0.8894(5.3);0.8817( 2.3);0.8529(0.5);0.8444(0.4);0.7651(2.0 );0.7574(4.6);0.7487(4.5);0.7382(5.0);0. 7298(3.9);0.7210(1.6);-0.0002(4.7) |
| **I-30** | 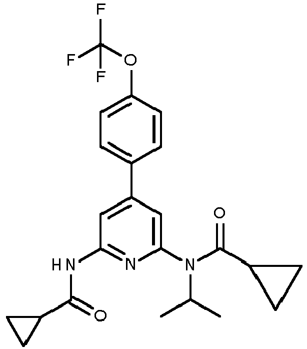 | I-30: ¹H-NMR(400.0 MHz, d₆-DMSO): δ= 11.0460(2.3);8.4697(3.0);8.0917(0.8 );8.0697(0.8);7.9079(3.1);7.8862(3.5);7. 8469(2.1);7.5566(0.8);7.5365(3.3);7.515 9(2.4);7.4128(3.1);4.8086(0.4);4.7917(0 .6);4.7747(0.4);4.7619(0.4);4.7460(0.8); 4.7293(1.1);4.7121(0.8);3.3182(26.1);2. 6716(0.4);2.5027(62.2);2.3293(0.4);2.09 79(0.6);2.0844(0.9);2.0698(0.6);1.3977( 12.8);1.1891(0.6);1.1809(0.8);1.1705(1. 3);1.1495(11.7);1.1328(16.0);1.1163(5.7 );0.8549(5.0);0.8352(2.9);0.8279(2.0);0. 8181(1.6);0.8078(1.6);0.7971(2.1);0.788 8(2.6);0.7801(1.9);0.6205(1.7);0.6112(1 .8);0.6016(2.1);0.5934(2.2);0.5852(1.4); 0.5746(1.0);0.5662(0.8);-0.0003(40.8) |
| **I-31** | 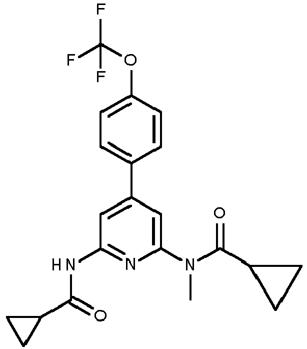 | I-31: ¹H-NMR(400.0 MHz, d₆-DMSO): δ= 11.0348(4.8);8.3441(6.5);8.3412(6.3 );7.8841(7.5);7.8621(8.5);7.5354(5.6);7. 5151(5.2);7.5036(7.6);7.5004(7.0);3.319 6(66.5);3.3085(28.4);2.6763(0.5);2.6718 (0.6);2.6671(0.4);2.5071(76.3);2.5026(9 7.8);2.4982(72.7);2.3340(0.5);2.3294(0. 6);2.3250(0.5);2.0813(0.5);2.0743(2.5); 2.0684(1.2);2.0650(1.3);2.0580(1.0);2.0 511(2.0);2.0371(1.3);2.0202(0.5);1.7168 (0.4); 1.7056(1.0); 1.6972(1.2); 1.6863(1. 9);1.6753(1.3);1.6669(1.0);1.6551(0.5); 0.8735(1.6);0.8543(16.0);0.8447(10.4);0 .8366(6.6);0.8115(0.5);0.7529(1.6);0.74 54(3.7);0.7369(3.2);0.7260(3.9);0.7176( 2.8);0.7084(1.1);0.1461(0.4);0.0078(3.3 );-0.0002(74.8);-0.0084(3.4);-0.1496(0.4) |
| **I-32** | 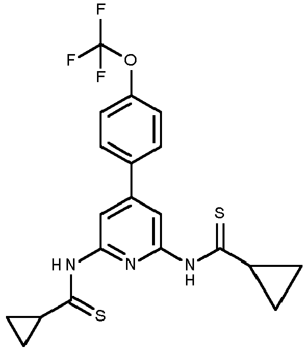 | I-32: ¹H-NMR(400.0 MHz, d₆-DMSO): δ= 12.1966(2.8);8.6524(1.2);7.8535(4.8 );7.8316(5.7);7.5708(3.8);7.5502(3.3);3. 7904(0.4);3.3209(42.9);2.6710(0.8);2.66 68(0.6);2.5711(1.2);2.5649(1.2);2.5061( 80.3);2.5017(109.2);2.4974(86.4);2.328 6(0.8);1.3980(16.0);1.2340(0.4);1.1708( 4.6);1.0382(3.7);1.0285(4.2);1.0195(4.4 );1.0106(3.2);-0.0002(0.5) |
| **I-33** | 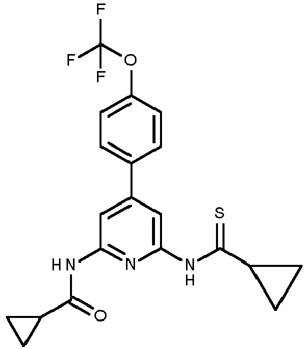 | I-33: ¹H-NMR(400.0 MHz, d₆-DMSO): δ=11.9941(1.8);10.7700(2.4);8.3789(0. 7);8.2338(3.1);7.8081(3.4);7.7863(4.1); 7.5415(2.9);7.5207(2.5);3.3186(16.5);2. 5557(0.5);2.5070(27.4);2.5027(36.7);2.4 986(29.8);2.0921(0.6);2.0797(0.8);2.066 6(0.6);1.3977(16.0);1.1603(1.6);1.0196( 1.2); 1.0108(1.4); 1.0027(1.5);0.8607(4.5 );0.8411(2.6);-0.0002(22.3) |
| **I-34** | 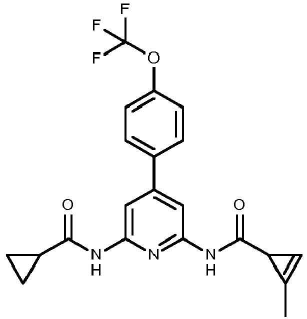 | I-34: ¹H-NMR(400.0 MHz, d₆-DMSO): δ= 10.5222(4.2);9.9752(3.9);8.3131(0.4 );8.1420(1.6);8.0625(4.8);8.0595(5.9);8. 0305(4.6);7.7518(5.6);7.7300(7.0);7.516 8(4.6);7.4961(3.9);6.8290(4.4);5.7535(3 .0);3.3186(91.7);2.6749(0.9);2.6707(1.2 );2.6665(0.9);2.5060(145.5);2.5017(194. 6);2.4974(146.0);2.3663(4.8);2.3630(5.1 );2.3283(1.2);2.3239(0.9);2.1372(16.0); 2.1016(0.9);2.0888(1.3);2.0733(1.1);2.0 574(0.4);0.8367(6.9);0.8181(3.9);0.7973 (0.4);0.1458(0.4);0.0076(3.0);-0.0003(86.0);-0.0082(3.8);-0.1498(0.4) |
| **I-35** | 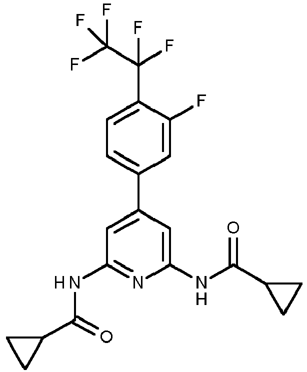 | I-35: ¹H-NMR(400.0 MHz, d₆-DMSO): δ= 10.5884(7.0);8.0744(11.4);7.9061(1. 2);7.8864(2.3);7.8674(1.5);7.7585(1.8); 7.7293(1.8);7.7059(2.2);7.6861(1.9);3.3 222(12.0);2.6721(0.4);2.5035(70.3);2.33 02(0.4);2.1395(0.6);2.1233(1.8);2.1091( 2.7);2.0946(2.0);2.0776(1.0);1.3980(0.9 );0.8555(16.0);0.8459(11.0);0.8364(8.8) ;-0.0002(28.4) |
| **I-36** | 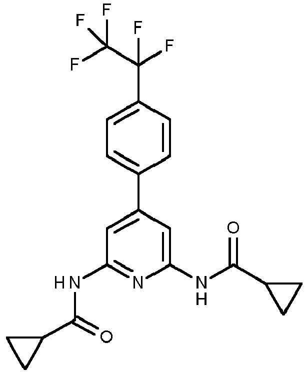 | I-36: ¹H-NMR(400.0 MHz, d₆-DMSO): δ= 10.5485(7.4);8.3083(2.0);8.0926(16. 0);8.0703(0.5);7.8816(1.8);7.8597(11.4) ;7.8527(10.5);7.8308(1.8);3.5737(0.4);3 .5579(0.4);3.5434(0.5);3.5252(0.5);3.51 49(0.5);3.4783(0.6);3.4614(0.7);3.4238( 1.5);3.4056(1.9);3.3480(883.9);3.3420(6 36.4);3.3357(449.1);3.3329(399.5);3.33 01(406.3);3.2571(0.7);3.2442(0.7);3.231 8(0.5);3.2140(0.3);2.6767(1.9);2.6723(2 .5);2.6683(1.8);2.5785(0.5);2.5252(7.5); 2.5119(155.0);2.5076(311.5);2.5031(41 1.2);2.4987(302.7);2.3340(1.8);2.3300(2 .4);2.1341(0.6);2.1183(2.0);2.1045(3.0); 2.0873(2.1);2.0741(0.8);1.2348(1.1);0.8 510(15.4);0.8308(8.6);0.8090(0.7);0.008 0(1.7);-0.0002(51.3);-0.0084(1.8) |
| **I-37** | 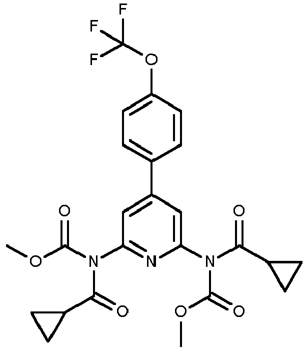 | I-37: ¹H-NMR(400.0 MHz, d₆-DMSO): δ= 10.5058(0.7);8.1713(0.8);8.1679(0.8 );8.0111(1.8);7.9890(2.0);7.9155(5.9);7. 8906(0.9);7.8687(1.0);7.5429(1.8);7.521 3(1.7);7.4810(0.8);7.4779(0.8);5.7536(2 .1);3.7166(16.0);3.6967(3.6);3.3162(21. 2);2.6107(0.5);2.6054(0.6);2.5927(1.1); 2.5859(0.5);2.5798(0.6);2.5741(0.6);2.5 235(0.7);2.5100(15.7);2.5058(31.8);2.50 13(43.7);2.4968(32.9);2.4926(16.0);1.01 60(1.2);1.0085(2.9);0.9895(5.1);0.9788( 3.2);0.9618(0.7);0.9544(0.4);0.0079(0.9 );-0.0002(24.0);-0.0084(0.8) |
| **I-38** | 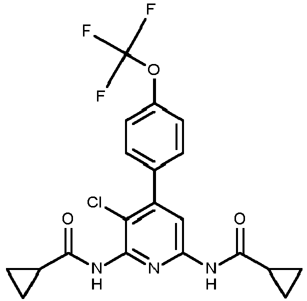 | I-38: ¹H-NMR(400.0 MHz, d₆-DMSO): δ= 12.0384(1.5);11.0610(4.1);10.3652(4 .9);8.3153(0.6);8.0253(7.8);7.6134(4.9); 7.5916(7.8);7.5229(5.3);7.5017(3.4);3.3 189(144.1);2.6703(3.2);2.5838(0.4);2.50 55(419.4);2.5013(540.4);2.4970(400.5); 2.3277(3.0);2.0629(0.4);2.0452(1.0);2.0 322(1.5);2.0172(1.1);2.0007(0.5);1.9527 (0.9);1.9356(0.4);1.9195(1.0);1.9055(1. 7);1.8987(1.0);1.8933(1.1);1.8759(0.5); 1.5084(0.4); 1.5003(0.4);1.4879(0.7);1.4 768(0.4); 1.4691(0.5); 1.2979(0.5); 1.2711 (0.6); 1.2584(1.0);1.2357(1.9);1.2050(0. 4);1.1885(0.5);1.1735(0.3);1.1347(0.6); 1.1174(0.6);1.0485(1.0);1.0320(1.1);0.8 808(0.6);0.8474(1.0);0.8267(8.5);0.8077 (16.0);0.7787(2.1);0.7714(1.8);0.7673(1 .9);0.7594(1.0);0.1459(1.1);-0.0001(241.9);-0.1495(1.2) |
| **I-39** | 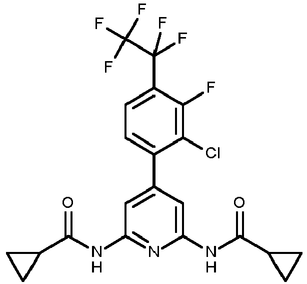 | I-39: ¹H-NMR(400.0 MHz, d₆-DMSO): δ= 10.6324(6.4);7.8609(7.1);7.8554(4.9 );7.8368(1.6);7.8201(1.2);7.5487(2.3);7. 5281(2.1);3.4692(32.6);3.4613(33.7);3.4 541(21.7);3.4448(33.6);3.4227(32.5);2.5 583(47.0);2.5419(0.5);2.5236(11.9);2.51 92(15.6);2.5148(11.8);2.1085(1.1);2.095 6(1.7);2.0811(1.7);2.0670(0.9);0.8447(1 0.5);0.8303(16.0) |
| **I-40** | 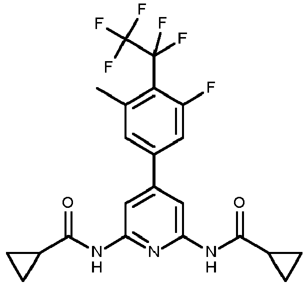 | I-40: ¹H-NMR(400.0 MHz, d₆-DMSO): δ= 10.5752(7.3);8.0365(13.1);7.5397(2. 0);7.5224(3.8);7.5103(2.0);3.3401(79.1) ;2.5615(3.8);2.5509(7.7);2.5445(13.0);2 .5095(38.4);2.5051(50.5);2.5007(38.9);2 .1328(0.6);2.1163(1.7);2.1022(2.6);2.08 60(1.8);2.0762(0.6);2.0712(0.7);0.8550( 16.0);0.8448(9.6);0.8353(8.0);0.8124(0. 6);-0.0002(1.0) |
| **I-41** | 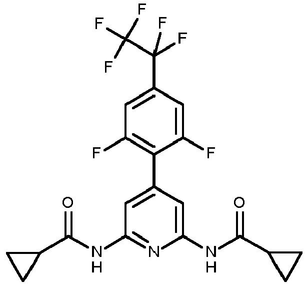 | I-41: ¹H-NMR(400.0 MHz, d₆-DMSO): δ= 10.6228(6.8);7.8855(8.3);7.7688(3.7 );7.7504(3.6);3.3397(38.5);2.5471(5.4); 2.5123(20.8);2.5079(27.4);2.5035(21.1); 2.1334(0.6);2.1179(1.9);2.1030(2.4);2.0 873(2.0);2.0718(0.6);0.8390(12.6);0.823 8(16.0);-0.0002(0.8) |
| **I-42** | 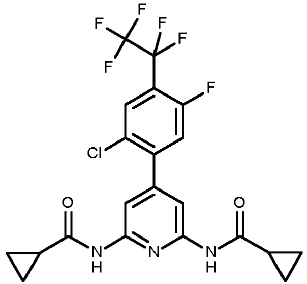 | I-42: ¹H-NMR(400.0 MHz, d₆-DMSO): δ= 10.6118(6.8);8.0209(2.4);8.0045(2.4 );7.8434(12.0);7.7346(2.2);7.7069(2.3); 3.3551(24.1);2.5494(46.7);2.5328(0.7);2 .5145(13.4);2.5101(17.6);2.5057(13.5);2 .1353(0.6);2.1198(1.9);2.1050(2.4);2.08 94(1.9);2.0739(0.6);0.8380(11.0);0.8234 (16.0);-0.0002(0.5) |
| **I-43** | 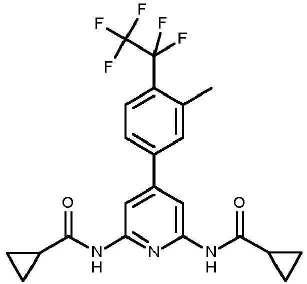 | I-43: ¹H-NMR(400.0 MHz, d₆-DMSO): δ= 10.5457(9.1);8.0696(16.0);7.7394(2. 2);7.7175(4.0);7.6632(6.5);7.6480(2.0); 3.3546(47.2);2.5475(41.7);2.5350(10.1); 2.5276(6.5);2.5167(12.1);2.5126(23.2);2 .5082(30.6);2.5037(23.4);2.1404(0.7);2. 1226(2.0);2.1172(1.8);2.1100(3.2);2.097 0(2.1);2.0926(2.0);2.0789(0.9);0.8836(0 .7);0.8527(15.2);0.8336(8.6);0.8122(0.8 );-0.0002(0.8) |
| **I-44** | 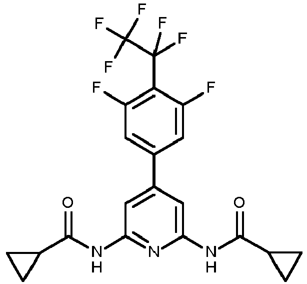 | I-44: ¹H-NMR(400.0 MHz, d₆-DMSO): δ= 10.6147(6.5);8.0447(11.6);7.6654(3. 4);7.6372(3.6);3.3497(20.4);2.5484(27.1 );2.5133(14.3);2.5090(18.6);2.5047(14.4 );2.1374(0.6);2.1217(1.7);2.1068(2.3);2. 0914(1.9);2.0761(0.6);0.8605(16.0);0.84 79(9.0);0.8433(8.6);-0.0002(0.4) |
| **I-45** | 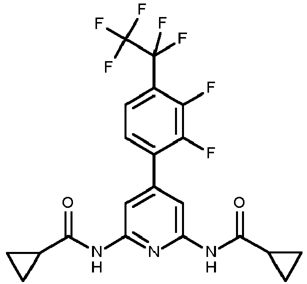 | I-45: ¹H-NMR(400.0 MHz, d₆-DMSO): δ=10.6210(6.5);8.0118(7.9);7.7114(0.7 );7.6937(1.4);7.6746(1.1);7.6243(1.1);7. 6071(1.4);7.5877(0.7);3.3498(31.9);2.54 94(45.9);2.5141(14.8);2.5098(20.2);2.50 55(16.3);2.1394(0.5);2.1244(1.7);2.1092 (2.3);2.0940(1.9);2.0789(0.6);0.8511(16 .0);0.8353(10.8);-0.0002(0.5) |
| **I-46** | 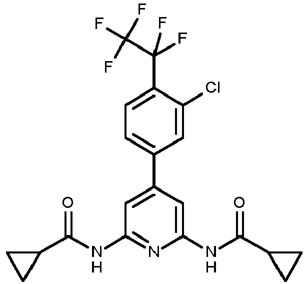 | I-46: ¹H-NMR(400.0 MHz, d₆-DMSO): δ= 10.6063(8.0);8.0797(11.2);7.9539(2. 8);7.9332(3.9);7.8967(4.5);7.8418(2.7); 7.8214(1.9);3.4547(54.6);3.4325(44.2);3 .4263(41.6);3.4165(47.6);2.5658(11.2);2 .5582(41.9);2.5236(16.5);2.5192(18.8);2 .5148(13.2);2.1058(2.6);2.0897(2.1);0.8 737(14.9);0.8686(16.0);0.8486(8.7);0.82 69(1.3) |
| **I-47** | 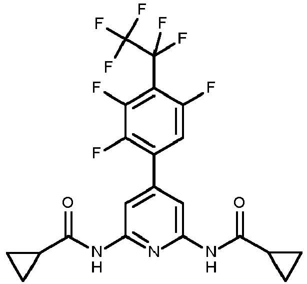 | I-47: ¹H-NMR(400.0 MHz, d₆-DMSO): δ= 10.6407(6.6);7.9950(8.4);7.6778(0.8 );7.6653(0.8);7.6495(0.9);7.6360(1.0);3. 3631(17.5);2.5542(36.9);2.5378(0.5);2.5 192(8.2);2.5148(11.0);2.5104(8.7);2.141 7(0.6);2.1265(2.0);2.1111(2.6);2.0960(2 .2);2.0807(0.7);0.8556(16.0);0.8401(13. 6) |
| **I-48** | 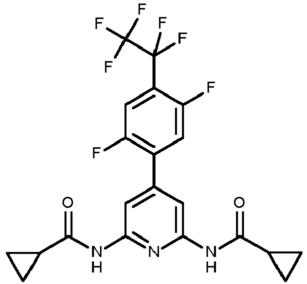 | I-48: ¹H-NMR(400.0 MHz, d₆-DMSO): δ= 10.6147(6.4);7.9851(7.6);7.9183(1.0 );7.9035(1.1);7.8936(1.1);7.8788(1.0);7. 7916(1.0);7.7769(1.1);7.7641(1.1);7.749 4(1.0);3.3655(25.1);2.5540(41.7);2.5191 (9.7);2.5147(12.7);2.5103(9.6);2.1399(0 .6);2.1245(1.8);2.1093(2.3);2.0941(2.0); 2.0834(0.5);2.0786(0.6);0.8538(16.0);0. 8391(10.6) |
| **I-49** | 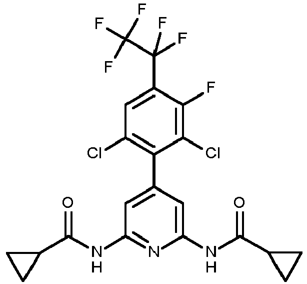 | I-49: ¹H-NMR(400.0 MHz, d₆-DMSO): δ= 10.6342(7.2);8.0674(2.5);8.0520(2.4 );7.6981(9.1);7.6606(0.3);3.3555(56.2); 3.3484(127.4);2.7153(0.4);2.5461(67.8); 2.5450(66.5);2.5424(52.6);2.5069(35.9); 2.3721(0.4);2.1110(2.3);2.0975(2.8);2.0 844(2.2);0.8163(16.0) |
| **I-50** | 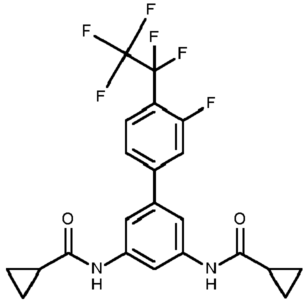 | I-50: ¹H-NMR(400.0 MHz, d₆-DMSO): δ= 10.3681(5.0);7.9669(3.0);7.9629(2.0 );7.8640(1.1);7.8441(2.1);7.8233(1.3);7. 6915(6.5);7.6870(6.6);7.6542(1.6);7.633 0(2.0);7.6227(1.7);7.6152(1.7);3.3236(5 8.4);2.6765(0.3);2.6716(0.5);2.6671(0.4 );2.5249(1.5);2.5115(29.0);2.5071(59.5) ;2.5026(79.2);2.4980(57.7);2.4936(28.2) ;2.3339(0.3);2.3294(0.5);2.3248(0.3);1. 8445(0.6);1.8280(1.6);1.8209(1.1);1.813 8(2.5); 1.7975(1.7); 1.7829(0.7); 1.2334(0 .5);0.8209(16.0);0.8093(8.0);0.8018(7.0 );0.7785(0.4);0.0080(0.7);-0.0002(19.2);-0.0084(0.7) |
| **I-51** | 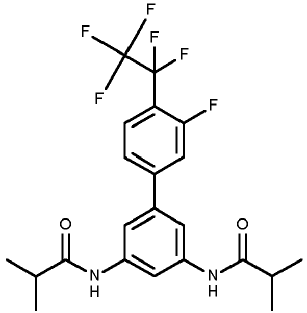 | I-51: ¹H-NMR(400.0 MHz, d₆-DMSO): δ= 9.9897(2.4);8.0256(1.4);8.0218(0.9); 7.8690(0.5);7.8491(1.0);7.8282(0.6);7.7 009(3.2);7.6963(3.1);7.6661(0.7);7.6462 (0.9);7.6350(0.8);7.6284(0.8);3.3214(21 .1);2.6714(0.4);2.6534(0.4);2.6365(1.1); 2.6194(1.5);2.6024(1.1);2.5854(0.4);2.5 246(1.0);2.5111(18.3);2.5067(36.5);2.50 22(47.9);2.4977(34.7);2.4933(17.1);1.39 77(8.8);1.1214(16.0);1.1044(15.7);0.007 9(0.7);-0.0002(16.7);-0.0085(0.7) |
| **I-52** | 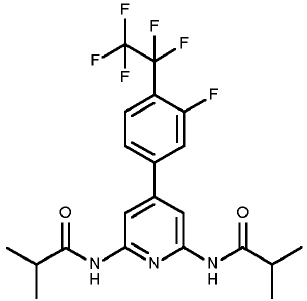 | I-52: ¹H-NMR(400.0 MHz, d₆-DMSO): δ=10.2171(2.8);8.0851(6.1);7.9208(0.5 );7.9010(1.0);7.8819(0.6);7.8027(0.7);7. 7730(0.7);7.7427(0.8);7.7222(0.6);3.322 4(22.1);2.8429(0.4);2.8259(1.0);2.8088( 1.3);2.7919(1.0);2.7750(0.4);2.6714(0.3 );2.5110(22.8);2.5068(45.5);2.5024(60.0 );2.4980(44.0);2.4506(0.4);2.4332(0.8); 2.4157(1.1);2.3983(0.8);2.3808(0.4);2.3 293(0.3);1.1157(16.0);1.0986(15.7);1.06 59(11.8); 1.0484(11.5);-0.0002(2.0) |
| **I-53** | 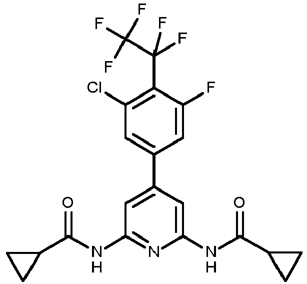 | I-53: ¹H-NMR(400.0 MHz, d₆-DMSO): δ= 10.6150(6.8);8.0420(12.7);7.7847(1. 7);7.7669(3.8);7.7568(1.8);3.3227(145.9 );2.6715(0.8);2.6668(0.6);2.5068(99.6); 2.5025(127.8);2.4981(92.2);2.3292(0.8); 2.3247(0.6);2.1344(0.5);2.1174(1.6);2.1 033(2.4);2.0881(1.8);2.0729(0.7);1.3979 (5.4);0.8578(16.0);0.8465(8.6);0.8389(7 .5);0.8101(0.7);0.1462(0.5);0.0077(4.1); -0.0002(98.7);-0.0082(4.1);-0.1498(0.5) |
| **I-54** | 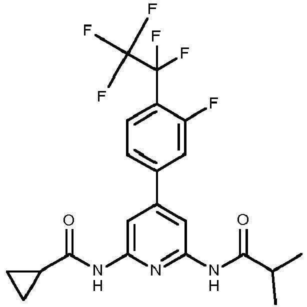 | I-54: ¹H-NMR(400.0 MHz, d₆-DMSO): δ= 10.6082(2.6);10.2128(2.6);8.0845(10 .4);7.9206(1.0);7.9010(1.9);7.8814(1.2); 7.7875(1.4);7.7581(1.4);7.7305(1.7);7.7 093(1.4);3.3390(121.3);3.2911(0.4);2.86 48(0.4);2.8481(1.0);2.8309(1.4);2.8138( 1.0);2.7968(0.4);2.6784(0.9);2.5483(59. 9);2.5135(108.6);2.5092(139.0);2.5048( 103.3);2.3361(0.9);2.1096(0.7);2.0951(1 1);2.0798(0.7);1.2415(0.5);1.1267(16.0 );1.1097(15.7);0.8582(6.7);0.8478(3.9); 0.8394(3.2) |
| **I-55** | 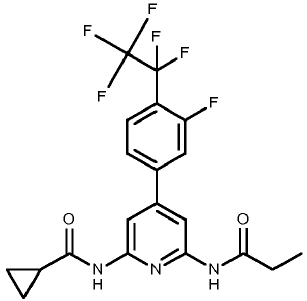 | I-55: ¹H-NMR(400.0 MHz, d₆-DMSO): δ= 10.5913(4.5);10.2244(4.6);8.0839(6. 2);8.0788(8.2);7.9213(1.7);7.9016(3.0); 7.8826(2.0);7.7758(2.3);7.7471(2.2);7.7 243(2.8);7.7033(2.3);3.3424(82.7);3.338 0(61.7);2.7184(0.6);2.6782(0.4);2.5483( 137.7);2.5313(1.4);2.5135(49.2);2.5091( 63.5);2.5046(47.0);2.4726(6.2);2.4539(6 .3);2.4351(2.0);2.3747(0.7);2.3358(0.4); 2.1307(0.4);2.1136(1.1);2.1000(1.7);2.0 866(1.2);2.0694(0.5);1.1140(7.5);1.0952 (16.0);1.0764(7.2);0.8571(10.5);0.8469( 6.2);0.8372(5.0);0.8141(0.4) |
| **I-56** | 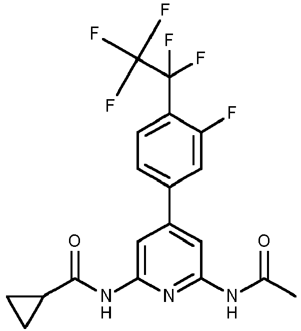 | I-56: ¹H-NMR(400.0 MHz, d₆-DMSO): δ= 10.5949(3.3);10.2976(3.3);8.0801(3. 8);8.0771(4.4);8.0530(2.0);7.9258(1.2); 7.9061(2.1);7.8873(1.4);7.7682(1.6);7.7 399(1.5);7.7144(1.9);7.6921(1.6);3.3383 (110.1);3.3336(83.3);2.6825(0.4);2.6783 (0.6);2.6736(0.5);2.5485(52.1);2.5315(1 .9);2.5181(36.0);2.5137(71.6);2.5092(94 .2);2.5046(69.7);2.5002(34.9);2.3405(0. 4);2.3359(0.6);2.3315(0.4);2.1571(16.0) ;2.1369(0.4);2.1190(0.8);2.1054(1.2);2. 0900(0.8);2.0739(0.3);0.8582(8.2);0.846 9(4.3);0.8387(3.8) |
| **I-57** | 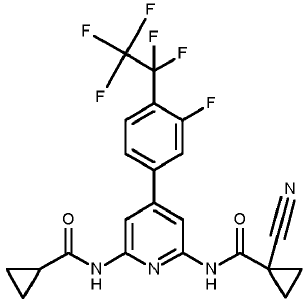 | I-57: ¹H-NMR(400.0 MHz, d₆-DMSO): δ= 10.9149(6.3);9.9315(5.9);8.2095(8.0 );8.2062(8.1);7.9261(1.4);7.9073(2.5);7. 8831(9.3);7.8798(8.8);7.8128(1.8);7.804 3(1.5);7.7840(1.8);7.7404(3.7);7.7200(3 .0);3.3382(160.9);2.6830(0.8);2.6783(1. 0);2.6741(0.8);2.5486(36.2);2.5316(4.0) ;2.5182(69.4);2.5138(135.5);2.5093(177 .1);2.5048(130.9);2.5005(65.8);2.3406(0 .8);2.3361(1.0);2.3315(0.8);2.0955(0.6); 2.0794(1.7);2.0645(2.3);2.0490(1.9);2.0 333(0.6); 1.7739(1.9); 1.7576(4.8); 1.7488 (8.4);1.7420(6.8);1.7161(7.0); 1.7089(8. 4); 1.7001(4.8); 1.6838(1.8); 1.2407(0.5); 0.8643(16.0);0.8505(9.5) |
| **I-58** | 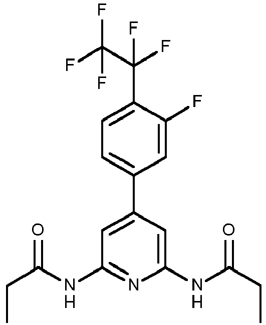 | I-58: ¹H-NMR(400.0 MHz, d₆-DMSO): δ= 10.2233(4.s);8.08s2(6.7);7.9340(0.8 );7.9142(1.5);7.8955(1.0);7.7911(1.1);7. 7611(1.1);7.7377(1.4);7.7165(1.2);3.339 5(78.6);2.7186(0.4);2.6784(0.4);2.5488( 74.1);2.5315(1.5);2.5179(25.9);2.5138(5 0.1);2.5094(65.8);2.5050(49.7);2.4834(2 .2);2.4647(6.2);2.4458(6.3);2.4270(2.1); 2.3750(0.4);2.3360(0.4);1.1100(7.6);1.0 912(16.0);1.0724(7.4);1.0582(0.3) |
| **I-59** | 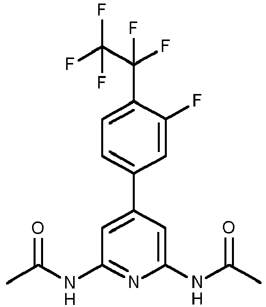 | I-59: ¹H-NMR(400.0 MHz, d₆-DMSO): δ= 10.2969(3.2);8.0524(2.4);7.9358(0.6 );7.9167(1.0);7.8981(0.6);7.7674(0.7);7. 7396(0.7);7.7142(1.1);7.6938(0.9);3.341 1(22.3);3.3376(20.8);2.5480(27.2);2.531 2(0.5);2.5133(15.8);2.5089(20.1);2.5045 (14.7);2.1514(16.0);2.0719(0.4) |
| **I-60** | 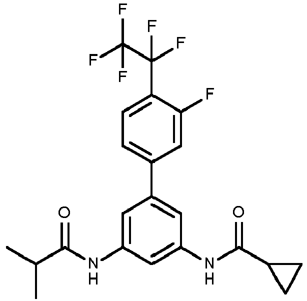 | I-60: ¹H-NMR(400.0 MHz, d₆-DMSO): δ= 10.3715(2.3);10.0028(2.5);8.0049(2. 8);7.8726(1.0);7.8526(1.9);7.8318(1.1); 7.7017(6.3);7.6972(6.2);7.6669(1.4);7.6 461(1.8);7.6354(1.6);7.6284(1.6);3.3426 (77.4);2.7186(0.5);2.6783(0.4);2.6584(0 .4);2.6413(1.1);2.6243(1.5);2.6072(1.2); 2.5902(0.6);2.5485(102.4);2.5316(1.4);2 .5180(22.4);2.5137(43.9);2.5092(57.4);2 .5047(42.7);2.5005(21.8);2.3747(0.5);2. 3358(0.4); 1.8368(0.7); 1.8293(0.5); 1.822 4(1,1);1.8067(0.8);1.1270(16.0);1.1100( 15.6);0.8282(7.7);0.8159(3.9);0.8104(3. 5) |
| **I-61** | 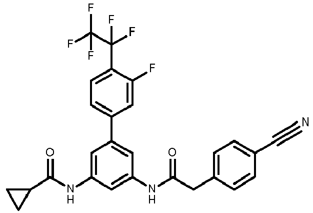 | I-61: ¹H-NMR(400.0 MHz, d₆-DMSO): δ= 10.4346(5.3);10.3898(4.9);7.9962(5. 5);7.8672(2.0);7.8472(4.0);7.8276(10.2) ;7.8073(10.1);7.6914(11.4);7.6871(11.3) ;7.6639(2.9);7.6355(6.5);7.6176(2.9);7. 5650(9.0);7.5445(7.6);3.8083(14.8);3.40 85(0.3);3.4008(0.4);3.3392(361.9);3.001 9(0.4);2.7186(0.9);2.6830(1.0);2.6784(1 .4);2.6741(1.0);2.5487(212.0);2.5317(5. 2);2.5181(86.4);2.5138(167.8);2.5094(2 19.2);2.5049(163.4);2.5006(84.0);2.375 0(0.9);2.3406(1.0);2.3358(1.3);2.3318(1 .0); 1.8427(0.5); 1.8276(1.7); 1.8123(2.3); 1.7976(1.9); 1.7813(0.6); 1.2402(0.8);0.8 279(16.0);0.8141(9.6) |
| **I-62** | 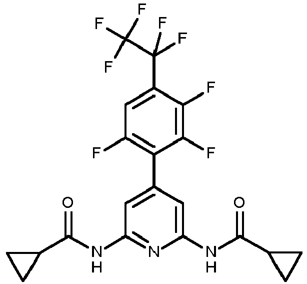 | I-62: ¹H-NMR(400.0 MHz, d₆-DMSO): δ= 10.6425(6.3);8.3147(0.6);7.9694(0.6 );7.9103(7.8);7.8338(0.7);7.8199(0.8);7. 8103(0.8);7.8014(0.7);3.3191(113.3);2.6 752(1.4);2.6708(1.8);2.6663(1.3);2.5238 (6.3);2.5103(114.7);2.5062(222.6);2.501 7(289.2);2.4973(211.8);2.3329(1.3);2.32 85(1.7);2.3240(1.3);2.1305(0.5);2.1157( 1.7);2.1007(2.2);2.0857(2.3);2.0698(0.7 );1.3981(3.2);1.0687(0.6);0.8362(9.4);0. 8223(16.0);0.0078(1.0);-0.0002(27.6);-0.0084(1.0) |

Die Messung der logP Werte erfolgt gemäß EEC Directive 79/831 Annex V.A8 durch HPLC (High Performance Liquid Chromatography) an einer Phasenumkehrsäule (C 18). Temperatur: 55°C. Die Bestimmung mit der LC-MS im sauren Bereich erfolgt bei pH 2,7 mit 0,1 % wässriger Ameisensäure und Acetonitril (enthält 0,1% Ameisensäure) als Eluenten; linearer Gradient von 10% Acetonitril bis 95% Acetonitril. In der Tabelle logP (HCOOH) genannt.

Die Bestimmung mit der LC-MS im neutralen Bereich erfolgt bei pH 7.8 mit 0,001 molarer wässriger Ammoniumhydrogencarbonat-Lösung und Acetonitril als Eluenten; linearer Gradient von 10 % Acetonitril bis 95 % Acetonitril. In der Tabelle logP (neutral) genannt.

Die Eichung erfolgt mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinander folgenden Alkanonen).

Die NMR-Daten ausgewählter Beispiele werden entweder in klassischer Form (δ-Werte, Multiplettaufspaltung, Anzahl der H-Atome) oder als NMR-Peak-Listen aufgeführt.

Das Lösungsmittel, in welchem das NMR-Spektrum aufgenommen wurde ist jeweils angegeben.

### NMR-Peak-Listenverfahren

Die 1H-NMR-Daten ausgewählter Beispiele werden in Form von 1H-NMR-Peaklisten notiert. Zu jedem Signalpeak wird erst der δ-Wert in ppm und dann die Signalintensität in runden Klammern aufgeführt. Die δ-Wert- Signalintensitäts- Zahlenpaare von verschiedenen Signalpeaks werden durch Semikolons voneinander getrennt aufgelistet.

Die Peakliste eines Beispieles hat daher die Form:
δ₁ (Intensität₁⁾; δ₂ (Intensität₂);........; δᵢ (Intensitätᵢ⁾;......; δₙ (Intensitätₙ)

Die Intensität scharfer Signale korreliert mit der Höhe der Signale in einem gedruckten Beispiel eines NMR-Spektrums in cm und zeigt die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden.

Zur Kalibrierung der chemischen Verschiebung von 1H-NMR-Spektren benutzen wir Tetramethylsilan und/oder die chemische Verschiebung des Lösungsmittels, besondern im Falle von Spektren, die in DMSO gemessen werden. Daher kann in NMR-Peaklisten der Tetramethylsilan-Peak vorkommen, muss es aber nicht.

Die Listen der 1H-NMR-Peaks sind ähnlich den klassischen 1H-NMR-Ausdrucken und enthalten somit gewöhnlich alle Peaks, die bei einer klassischen NMR-Interpretation aufgeführt werden.

Darüber hinaus können sie wie klassische 1H-NMR-Ausdrucke Lösungsmittelsignale, Signale von Stereoisomeren der Zielverbindungen, die ebenfalls Gegenstand der Erfindung sind, und/oder Peaks von Verunreinigungen zeigen.

Bei der Angabe von Verbindungssignalen im Delta-Bereich von Lösungsmitteln und/oder Wasser sind in unseren Listen von 1H-NMR-Peaks die gewöhnlichen Lösungsmittelpeaks, zum Beispiel Peaks von DMSO in DMSO-D₆ und der Peak von Wasser, gezeigt, die gewöhnlich im Durchschnitt eine hohe Intensität aufweisen.

Die Peaks von Stereoisomeren der Targetverbindungen und/oder Peaks von Verunreinigungen haben gewöhnlich im Durchschnitt eine geringere Intensität als die Peaks der Zielverbindungen (zum Beispiel mit einer Reinheit von >90%).

Solche Stereoisomere und/oder Verunreinigungen können typisch für das jeweilige Herstellungsverfahren sein. Ihre Peaks können somit dabei helfen, die Reproduktion unseres Herstellungsverfahrens anhand von "Nebenprodukt-Fingerabdrücken" zu erkennen.

Einem Experten, der die Peaks der Zielverbindungen mit bekannten Verfahren (MestreC, ACD-Simulation, aber auch mit empirisch ausgewerteten Erwartungswerten) berechnet, kann je nach Bedarf die Peaks der Zielverbindungen isolieren, wobei gegebenenfalls zusätzliche Intensitätsfilter eingesetzt werden. Diese Isolierung wäre ähnlich dem betreffenden Peak-Picking bei der klassischen 1H-NMR-Interpretation.

Weitere Details zu 1H-NMR-Peaklisten können der Research Disclosure Database Number 564025 entnommen werden.

### Anwendungsbeislpiele

### Ctenocephalides felis - in-vitro Kontakttests mit adulten Katzenflöhen

Für die Beschichtung der Teströhrchen werden zunächst 9 mg Wirkstoff in 1 ml Aceton p.a. gelöst und anschließend mit Aceton p.a. auf die gewünschte Konzentration verdünnt. 250 µl der Lösung werden durch Drehen und Kippen auf einem Rotationsschüttler (2 h Schaukelrotation bei 30 rpm) homogen auf den Innenwänden und dem Boden eines 25ml Glasröhrchens verteilt. Bei 900 ppm Wirkstofflösung und 44,7 cm² Innenoberfläche wird bei homogener Verteilung eine Flächendosis von 5 µg/cm² erreicht.

Nach Abdampfen des Lösungsmittels werden die Gläschen mit 5-10 adulten Katzenflöhen (*Ctenocephalides felis*) besetzt, mit einem gelochten Kunststoffdeckel verschlossen und liegend bei Raumtemperatur und Umgebungsfeuchte inkubiert. Nach 48 h wird die Wirksamkeit bestimmt. Hierzu werden die Gläschen aufrecht gestellt und die Flöhe auf den Boden des Gläschens geklopft. Flöhe, die unbeweglich auf dem Boden verbleiben oder sich unkoordiniert bewegen, gelten als tot bzw. angeschlagen.

Eine Substanz zeigt gute Wirkung gegen *Ctenocephalides felis,* wenn in diesem Test bei einer Aufwandmenge von 5 µg/cm² mindestens 80% Wirkung erzielt wurde. Dabei bedeutet 100% Wirkung, dass alle Flöhe angeschlagen oder tot waren. 0% Wirkung bedeutet, dass keine Flöhe geschädigt wurden.

Bei diesem Test zeigen z.B. die folgenden Verbindungen der Herstellungsbeispiele eine Wirkung von 100% bei einer Aufwandmenge von 5 µg/cm² (= 500g/ha): I-017

### Ctenocephalides felis - Oraltest

| | |
|---|---|
| Lösungsmittel: | Dimethylsulfoxid |

Zwecks Herstellung einer zweckmäßigen Wirkstoffzubereitung vermischt man 10 mg Wirkstoff mit 0,5 ml Dimethylsulfoxid. Durch Verdünnen mit citriertem Rinderblut erhält man die gewünschte Konzentration.

Ca. 20 nüchterne adulte Katzenflöhe (*Ctenocephalides felis*) werden in eine Kammer eingesetzt, die oben und unten mit Gaze verschlossen ist. Auf die Kammer wird ein Metallzylinder gestellt, dessen Unterseite mit Parafilm verschlossen ist. Der Zylinder enthält die Blut-Wirkstoffzubereitung, die von den Flöhen durch die Parafilmmembran aufgenommen werden kann.

Nach 2 Tagen wird die Abtötung in % bestimmt. Dabei bedeutet 100 %, dass alle Flöhe abgetötet wurden; 0 % bedeutet, dass keiner der Flöhe abgetötet wurde.

Bei diesem Test zeigen z.B. die folgenden Verbindungen der Herstellungsbeispiele eine Wirkung von 100% bei einer Aufwandmenge von 100ppm: I-001, I-003, I-004, I-005, I-006, I-007, I-014, I-015, I-017, I-018, I-021, I-022, I-023, I-026, I-027, I-028, I-036, I-043, I-046, I-050, I-051, I-052, I-055

Bei diesem Test zeigen z.B. die folgenden Verbindungen der Herstellungsbeispiele eine Wirkung von 95% bei einer Aufwandmenge von 100ppm: I-012

### Lucilia cuprina - Test

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration.

Ca. 20 L1-Larven der Australischen Schafgoldfliege (*Lucilia cuprina*) werden in ein Testgefäß überführt, welches gehacktes Pferdefleisch und die Wirkstoffzubereitung der gewünschten Konzentration enthält.

Nach 2 Tagen wird die Abtötung in % bestimmt. Dabei bedeutet 100 %, dass alle Larven abgetötet wurden; 0 % bedeutet, dass keine Larven abgetötet wurden.

Bei diesem Test zeigen z.B. die folgenden Verbindungen der Herstellungsbeispiele eine Wirkung von 100% bei einer Aufwandmenge von 100ppm: I-014

Bei diesem Test zeigen z.B. die folgenden Verbindungen der Herstellungsbeispiele eine Wirkung von 95% bei einer Aufwandmenge von 100ppm: I-001, I-036

### Musca domestica-Test

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration.

Gefäße, die einen Schwamm enthalten, der mit Zuckerlösung und der Wirkstoffzubereitung der gewünschten Konzentration behandelt wurde, werden mit 10 adulten Stubenfliegen *(Musca domestica)* besetzt.

Nach 2 Tagen wird die Abtötung in % bestimmt. Dabei bedeutet 100 %, dass alle Fliegen abgetötet wurden; 0 % bedeutet, dass keine der Fliegen abgetötet wurde.

Bei diesem Test zeigen z.B. die folgenden Verbindungen der Herstellungsbeispiele eine Wirkung von 90% bei einer Aufwandmenge von 100ppm: I-006

Bei diesem Test zeigen z.B. die folgenden Verbindungen der Herstellungsbeispiele eine Wirkung von 80% bei einer Aufwandmenge von 100ppm: I-014

### Meloidogyne incognita- Test

| | |
|---|---|
| Lösungsmittel: | 125,0 Gewichtsteile Aceton |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser auf die gewünschte Konzentration.

Gefäße werden mit Sand, Wirkstofflösung, einer Ei-Larven-Suspension des südlichen Wurzelgallenälchens (*Meloidogyne incognita*) und Salatsamen gefüllt. Die Salatsamen keimen und die Pflänzchen entwickeln sich. An den Wurzeln entwickeln sich die Gallen.

Nach 14 Tagen wird die nematizide Wirkung anhand der Gallenbildung in % bestimmt. Dabei bedeutet 100 %, dass keine Gallen gefunden wurden; 0 % bedeutet, dass die Zahl der Gallen an den behandelten Pflanzen der unbehandelten Kontrolle entspricht.

Bei diesem Test zeigen z. B. die folgenden Verbindungen der Herstellungsbeispiele Wirkung von 90% bei einer Aufwandmenge von 20ppm: I-001

### Myzus persicae - Sprühtest

| | |
|---|---|
| Lösungsmittel: | 78 Gewichtsteile Aceton |
| | 1,5 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Chinakohlblattscheiben (*Brassica pekinensis*), die von allen Stadien der Grünen Pfirsichblattlaus (*Myzus persicae*) befallen sind, werden mit einer Wirkstoffzubereitung der gewünschten Konzentration gespritzt.

Nach 6 Tagen wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele Wirkung von 90% bei einer Aufwandmenge von 500g/ha: I-008

### Phaedon cochleariae - Sprühtest

| | |
|---|---|
| Lösungsmittel: | 78,0 Gewichtsteile Aceton |
| | 1,5 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Chinakohlblattscheiben (*Brassica pekinensis*) werden mit einer Wirkstoffzubereitung der gewünschten Konzentration gespritzt und nach dem Abtrocknen mit Larven des Meerrettichblattkäfers *(Phaedon cochleariae)* besetzt.

Nach 7 Tagen wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele Wirkung von 100% bei einer Aufwandmenge von 500g/ha: I-001, I-004, I-010, I-012, I-014, I-015, I-016, I-017, I-018, I-019, I-020, I-022, I-023, I-027, I-028, I-035, I-037, I-040, I-043, I-045, I-046, I-050, I-051, I-052, I-053, I-054, I-055, I-056, I-058, I-059, I-060, I-062

Bei diesem Test zeigen z. B. die folgenden Verbindungen der Herstellungsbeispiele Wirkung von 83% bei einer Aufwandmenge von 500g/ha: I-005, I-006, I-025, I-026, I-036, I-048

### Spodoptera frugiperda - Sprühtest

| | |
|---|---|
| Lösungsmittel: | 78,0 Gewichtsteile Aceton |
| | 1,5 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Maisblattscheiben (*Zea mays*) werden mit einer Wirkstoffzubereitung der gewünschten Konzentration gespritzt und nach dem Abtrocknen mit Raupen des Heerwurms *(Spodoptera frugiperda)* besetzt.

Nach 7 Tagen wird die Wirkung in % bestimmt. Dabei bedeutet 100 %, dass alle Raupen abgetötet wurden; 0 % bedeutet, dass keine Raupe abgetötet wurde.

Bei diesem Test zeigen z. B. die folgenden Verbindungen der Herstellungsbeispiele Wirkung von 100% bei einer Aufwandmenge von 500g/ha: I-001, I-002, I-003, I-004, I-005, I-006, I-009, I-011, I-012, I-013, I-014, I-015, I-016, I-017, I-018, I-019, I-020, I-021, I-022, I-023, I-025, I-026, I-027, I-028, I-032, I-033, I-035, I-036, I-040, I-042, I-043, I-044, I-045, I-046, I-048, I-050, I-051, I-052, I-053, I-054, I-055, I-056, I-057, I-058, I-059, I-060, I-062

Bei diesem Test zeigen z. B. die folgenden Verbindungen der Herstellungsbeispiele Wirkung von 83% bei einer Aufwandmenge von 500g/ha: I-007, I-038, I-041, I-047, I-061

### Tetranychus urticae - Sprühtest, OP-resistent

| | |
|---|---|
| Lösungsmittel: | 78,0 GewichtsteileAceton |
| | 1,5 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Bohnenblattscheiben *(Phaseolus vulgaris),* die von allen Stadien der Gemeinen Spinnmilbe *(Tetranychus urticae)* befallen sind, werden mit einer Wirkstoffzubereitung der gewünschten Konzentration gespritzt.

Nach 6 Tagen wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele eine Wirkung von 90% bei einer Aufwandmenge von 500g/ha: I-015, I-034, I-036

## Patentansprüche

1. Verbindungen der allgemeinen Formel (I) 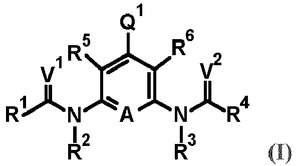 in denen
Q¹ für eine Substruktur der Formel (Q¹-A) steht: 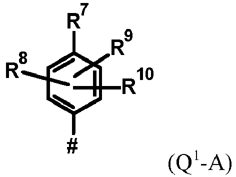
wobei Q¹-A an der mit # gekennzeichneten Stelle mit dem Sechsring gemäß Formel (I) verbunden ist,
R⁷ für Fluormethyl, Difluormethyl, Trifluormethyl, Fluorethyl, Difluorethyl, Trifluorethyl, Tetrafluorethyl, Pentafluorethyl, Trifluormethoxy, Difluormethoxy, Difluorchlormethoxy, Dichlorfluormethoxy, Pentafluorethoxy, Tetrafluorethoxy, Trifluorethoxy, Chlortetrafluorethoxy, Dichlortrifluorethoxy, Trichlordifluorethoxy, Chlortrifluorethoxy, Chlordifluorethoxy, Trifluormethylsulfonyl, Trifluormethylsulfinyl, Trifluormethylsulfanyl, Difluormethylsulfonyl, Difluormethylsulfinyl, Difluormethylsulfanyl, Pentafluorethylsulfonyl, Pentafluorethylsulfinyl, Pentafluorethylsulfanyl, Trifluorethylsulfonyl, Trifluorethylsulfinyl, Trifluorethylsulfanyl, Difluorethylsulfonyl, Difluorethylsulfinyl, Difluorethylsulfanyl, Tetrafluorethylsulfonyl, Tetrafluorethylsulfinyl, Tetrafluorethylsulfanyl, Trifluormethylcyclopropyl oder SF₅ steht,
R⁸, R⁹, R¹⁰ unabhängig voneinander für Wasserstoff, Cyano, Fluor, Chlor, Brom, Iod, Methyl, Ethyl, n-Propyl, i-Propyl, Cyclopropyl, Trifluormethyl, Methoxy oder Ethoxy stehen.
R¹ für (C₁-C₄)Alkyl, (C₁-C₄)Halogenalkyl, (C₁-C₄)Alkoxy-(C₁-C₄)alkyl, (C₂-C₄)Alkenyl, (C₂-C₄)Alkinyl, (C₃-C₆)Cycloalkyl, (C₃-C₆)Cycloalkyl-(C₁-C₄)alkyl, (C₃-C₆)Cycloalkyl-(C₃-C₆)Cycloalkyl, (C₁-C₄)Alkyl-(C₃-C₆)Cycloalkyl, (C₁-C₄)Halogenalkyl-(C₃-C₆)cycloalkyl, Halogen(C₃-C₆)cycloalkyl, Cyano(C₃-C₆)cycloalkyl oder (C₁-C₄)Alkoxy-(C₃-C₆)cycloalkyl steht,
R², R³ unabhängig voneinander für Wasserstoff, Methyl, Ethyl, n-Propyl, i-Propyl, cyclo-Propyl, n-Butyl, i-Butyl, tert.-Butyl, cyclo-Butyl, Methoxy, Ethoxy, Methoxymethyl, Ethoxymethyl, Methoxycarbonyl, Ethoxycarbonyl, n-Propoxycarbonyl oder i-Propoxycarbonyl stehen,
R⁴ für (C₁-C₆)Alkyl, (C₁-C₆)Halogenalkyl, (C₁-C₆)Cyanoalkyl, (C₁-C₄)Alkoxy-(C₁-C₄)alkyl, (C₁-C₄)Halogenalkoxy-(C₁-C₄)alkyl, (C₂-C₄)Alkenyl, (C₂-C₄)Halogenalkenyl, (C₂-C₄)Cyanoalkenyl, (C₂-C₄)Alkinyl, (C₂-C₆)Halogenalkinyl, (C₂-C₆)Cyanoalkinyl, (C₃-C₆)Cycloalkyl, (C₃-C₆)Cycloalkyl-(C₁-C₄)alkyl, Cyano(C₃-C₆)cycloalkyl-(C₁-C₄)alkyl, Halogen(C₃-C₆)cycloalkyl-(C₁-C₄)alkyl, (C₁-C₄)Halogenalkyl-(C₃-C₆)cycloalkyl-(C₁-C₄)alkyl, (C₃-C₆)Cycloalkyl-(C₃-C₆)Cycloalkyl, (C₁-C₄)Alkyl-(C₃-C₆)Cycloalkyl, (C₁-C₄)Halogenalkyl-(C₃-C₆)cycloalkyl, Halogen(C₃-C₆)cycloalkyl, Cyano(C₃-C₆)cycloalkyl, (C₂-C₄)Alkinyl-(C₃-C₆)cycloalkyl, (C₁-C₄)Alkoxy-(C₃-C₆)cycloalkyl, Carbamoyl-(C₃-C₈)cycloalkyl, Thiocarbamoyl-(C₃-C₈)cycloalkyl, (C₃-C₆)Cycloalkenyl, Halogen(C₃-C₆)Cycloalkenyl, (C₁-C₄)Alkyl-(C₃-C₆)Cycloalkenyl oder (C₁-C₄)Halogenalkyl-(C₃-C₆)Cycloalkenyl steht,
oder für durch Phenyl oder Pyridyl substituiertes (C₁-C₄)Alkyl, (C₁-C₄)Halogenalkyl oder (C₃-C₆)Cycloalkyl steht, wobei Phenyl oder Pyridyl jeweils gegebenenfalls einfach oder mehrfach, gleich oder verschieden durch Halogen, Cyano, (C₁-C₄)Alkyl, (C₃-C₆)Cycloalkyl, (C₁-C₄)Alkoxy, (C₁-C₄)Halogenalkyl, (C₁-C₄)Halogenalkoxy, (C₁-C₄)Alkylthio, (C₁-C₄)Halogenalkylthio, (C₁-C₄)Alkylsulfinyl, (C₁-C₄)Alkylsulfonyl, (C₁-C₄)Halogenalkylsulfinyl oder (C₁-C₄)Halogenalkylsulfonyl substituiert sein können,
oder für jeweils gegebenenfalls einfach oder mehrfach, gleich oder verschieden substituiertes Phenyl oder Pyridyl steht, wobei die Substituenten ausgewählt sind aus Halogen, Cyano, (C₁-C₄)Alkyl, (C₃-C₆)Cycloalkyl, (C₁-C₄)-Alkoxy, (C₁-C₄)Halogenalkyl, (C₁-C₄)Halogenalkoxy, (C₁-C₄)Alkylthio, (C₁-C₄)Halogenalkylthio, (C₁-C₄)Alkylsulfinyl, (C₁-C₄)Alkylsulfonyl, (C₁-C₄)Halogenalkylsulfinyl, (C₁-C₄)Halogenalkylsulfonyl,
R⁵, R⁶ unabhängig voneinander für Wasserstoff, Cyano, Fluor, Chlor, Brom, oder Methyl stehen,
V¹, V² unabhängig voneinander für Sauerstoff oder Schwefel stehen,
A für Stickstoff, =N⁺(O⁻)- oder =C(H)- steht.

2. Verbindungen der allgemeinen Formel (I) gemäß Anspruch 1, in denen
Q¹ für eine Substruktur der Formel (Q¹-A) steht: 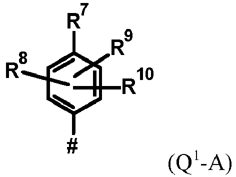
wobei Q¹-A an der mit # gekennzeichneten Stelle mit dem Sechsring gemäß Formel (I) verbunden ist,
R⁷ für Trifluormethyl, Difluorethyl, Trifluorethyl, Pentafluorethyl, Trifluormethoxy, Difluormethoxy, Pentafluorethoxy, Trifluorethoxy, Trifluormethylsulfonyl, Trifluormethylsulfinyl, Trifluormethylsulfanyl, Difluormethylsulfonyl, Difluormethylsulfinyl, Difluormethylsulfanyl, Trifluormethylcyclopropyl oder SF₅ steht,
R⁸, R⁹, R¹⁰ unabhängig voneinander für Wasserstoff, Fluor, Chlor oder Methyl stehen.
R¹ für Methyl, Ethyl, Isopropyl oder Cyclopropyl steht,
R² für Wasserstoff, Ethyl oder Methoxycarbonyl steht,
R³ für Wasserstoff, Methyl, Ethyl, Isopropyl oder Methoxycarbonyl steht,
R⁴ für Methyl, Ethyl, Propyl, Butyl, Isopropyl, Isobutyl, tert-Butyl, Trifluormethyl, Cyanomethyl, Cyanoethyl, Cyanopropyl, Cyanobutyl, Cyanopentyl, (Trifluormethyl)propyl, Fluorpropyl, Chlorpropyl, Phenylpropyl, Cyclopropyl, Cyanocyclopropyl, Fluorcyclopropyl, Chlorcyclopropyl, Methylcyclopropyl, (Trifluormethyl)cyclopropyl, Cyclopropylcyclopropyl, Dicyclopropylcyclopropyl, Phenylcyclopropyl, Cyclobutyl, Cyclopentyl, Fluorcyclopentyl, Chlorcyclopentyl, Methylcyclopentyl, (Trifluormethyl)cyclopentyl, [(Trifluormethyl)phenyl]methyl, (Cyanophenyl)methyl, (Fluorphenyl)methyl, (Chlorphenyl)methyl, Phenyl, Cyanophenyl, Fluorphenyl, Chlorphenyl oder Methylcycloprop-2-en-1-yl steht,
R⁵ für Wasserstoff oder Chlor steht,
R⁶ für Wasserstoff steht,
V¹, V² unabhängig voneinander für Sauerstoff oder Schwefel stehen,
A für Stickstoff, oder =C(H)- steht.

3. Verbindungen der allgemeinen Formel (I) gemäß Anspruch 1 oder 2, in denen
Q¹ für 4-(Trifluormethoxy)phenyl 4-(Pentafluorethyl)phenyl, 3-Fluor-4-(pentafluorethyl)phenyl, 2-Chlor-3-fluor-4-(pentafluorethyl)phenyl, 3-Fluor-5-methyl-4-(pentafluorethyl)phenyl, 2,6-Difluor-4-(pentafluorethyl)phenyl, 2-Chlor-5-fluor-4-(pentafluorethyl)phenyl, 3-Methyl-4-(pentafluorethyl)phenyl, 3,5-Difluor-4-(pentafluorethyl)phenyl, 2,3-Difluor-4-(pentafluorethyl)phenyl, 3-Chlor-4-(pentafluorethyl)phenyl, 2,3,5-Trifluor-4-(pentafluorethyl)phenyl, 2,5-Difluor-4-(pentafluorethyl)phenyl, 2,6-Dichlor-3-fluor-4-(pentafluorethyl)phenyl, 3-Chlor-5-fluor-4-(pentafluorethyl)phenyl oder 2,3,6-Trifluor-4-(pentafluorethyl)phenyl steht,
R¹ für Methyl, Ethyl, Isopropyl oder Cyclopropyl steht,
R² für Wasserstoff, Ethyl oder Methoxycarbonyl steht,
R³ für Wasserstoff, Methyl, Ethyl, Isopropyl oder Methoxycarbonyl steht,
R⁴ für Cyclopropyl, [4-(Trifluormethyl)phenyl]methyl, (3-Cyanophenyl)methyl, 1-methylcyclopropyl, 1-Cyanocyclopropyl, 1-(Trifluormethyl)cyclopropyl, (4-Cyanophenyl)methyl, 1-Phenylcyclopropyl, 2-Cyanopropan-2-yl, 2-Phenylpropan-2-yl, 1-Methylcyclopentyl, 1-Fluorcyclopropyl, 1-Chlorcyclopropyl, 1-Cyclopropylcyclopropyl, 2-Fluorpropan-2-yl, 1,1,1-Trifluor-2-methylpropan-2-yl, tert-Butyl, Isopropyl, Butyl, Ethyl, Cyclopentyl, Methyl, Propyl, Cyanomethyl, 1-Cyanoethan-2-yl, Phenyl, 4-Cyanophenyl, 2,2-Dicyclopropylcyclopropyl oder 2-Methylcycloprop-2-en-1-yl steht,
R⁵ für Wasserstoff oder Chlor steht,
R⁶ für Wasserstoff steht,
V¹, V² unabhängig voneinander für Sauerstoff oder Schwefel stehen,
A für Stickstoff, oder =C(H)- steht.

4. Agrochemische Formulierung enthaltend Verbindungen der allgemeinen Formel (I) und / oder deren Salze gemäß einem der Ansprüche 1 bis 3 in biologisch wirksamen Gehalten von zwischen 0,00000001 und 98 Gew.-%, bezogen auf das Gewicht der agrochemischen Formulierung, sowie Streckmittel und/oder oberflächenaktive Stoffe.

5. Agrochemische Formulierung gemäß Anspruch 4 zusätzlich enthaltend einen weiteren agrochemischen Wirkstoff.

6. Verfahren zur Bekämpfung von tierischen Schädlingen, bei dem man Verbindungen der allgemeinen Formel (I) und / oder deren Salze gemäß einem der Ansprüche 1 bis 3 auf tierische Schädlinge und/oder ihren Lebensraum einwirken lässt, wobei Verfahren zur Behandlung des menschlichen oder tierischen Körpers ausgeschlossen sind.

7. Verwendung von Verbindungen der allgemeinen Formel (I) gemäß einem der Ansprüche 1 bis 3 zur Bekämpfung von tierischen Schädlingen, wobei die Behandlung des menschlichen oder tierischen Körpers ausgeschlossen ist.

8. Verwendung von Verbindungen der allgemeinen Formel (I) gemäß einem der Ansprüche 1 bis 3 zum Schutz des Vermehrungsmaterials von Pflanzen.
